# EUROPEAN PATENT APPLICATION

(11) **EP 4 122 952 A1**
(43) Date of publication of application: **25.01.2023**
(21) Application number: 21771688.5
(22) Date of filing: 19.03.2021
(51) Int. Cl.: C07K 14/55, C07K 19/00, C12N 15/26, C12N 15/62, C12N 15/63, C12N 5/10, A61K 38/20, A61P 35/00, A61P 37/00

(54) **INTERLEUKIN-2 MUTANT AND USE THEREOF**

(30) Priority: 19.03.2020 CN 202010197740; 11.03.2021 CN 202110266549
(71) Applicant: Innovent Biologics (Suzhou) Co., Ltd., Suzhou, Jiangsu 215123 (CN)
(72) Inventor: HE, Kaijie, Suzhou, Jiangsu 215123 (CN); FU, Fenggen, Suzhou, Jiangsu 215123 (CN); ZHOU, Shuaixiang, Suzhou, Jiangsu 215123 (CN); WU, Weiwei, Suzhou, Jiangsu 215123 (CN)
(74) Representative: Ruscoe, Peter James
(86) International application number: PCT/CN2021/081840
(87) International publication number: WO 2021/185361

(57) **Abstract**

The present invention relates to a novel interleukin-2 (IL-2) mutant protein. The present invention also provides a fusion protein, a dimeric molecule and an immunoconjugate comprising the IL-2 mutant protein, as well as a nucleic acid encoding same, a vector and a host cell comprising the nucleic acid, a pharmaceutical composition comprising same and therapeutic use. The present invention further provides a method for preparing the IL-2 mutant protein, the fusion protein, the dimeric molecule and the immunoconjugate.

## Description

### TECHNICAL FIELD

The present invention relates to a novel interleukin-2 (IL-2) mutant protein and use thereof. In particular, the present invention relates to an IL-2 mutant protein that has improved properties, such as an improved binding property to an IL-2 receptor and improved druggability, compared to a wild-type IL-2. The present invention further provides a fusion protein, a dimer and an immunoconjugate comprising the IL-2 mutant protein, a nucleic acid encoding the IL-2 mutant protein, the dimer and the immunoconjugate, and a vector and a host cell comprising the nucleic acid. The present invention further provides a method for preparing the IL-2 mutant protein, the fusion protein, the dimer and the immunoconjugate, a pharmaceutical composition comprising same, and therapeutic use.

### BACKGROUND

Interleukin-2 (IL-2), also known as T-cell growth factor (TCGF), is a multifunctional cytokine produced mainly by activated T cells, particularly by CD4⁺T helper cells. In eukaryotic cells, human IL-2 (UniProt: P60568) is synthesized as a precursor polypeptide of 153 amino acids, and mature secretory IL-2 is produced after removal of 20 N-terminus amino acids. The sequences of IL-2 from other species have also been disclosed. See NCBI Ref Seq No. NP032392 (mice), NP446288 (rats) or NP517425 (chimpanzees).

Interleukin-2 has 4 antiparallel and amphipathic α helices, which form a quaternary structure essential for its function (Smith, Science 240,1169-76 (1988); Bazan, Science 257,410-413 (1992)). In most cases, IL-2 acts through three different receptors: interleukin-2 receptor α (IL-2Rα; CD25), interleukin-2 receptor β (IL-2RP; CD122), and interleukin-2 receptor y (IL-2Ry; CD132). IL-2RP and IL-2Ry are critical for IL-2 signaling, while IL-2Rα (CD25) is not essential for signaling but can enable IL-2 to bind to a receptor with high affinity (Krieg et al., Proc Natl Acad Sci 107,11906-11 (2010)). The trimeric receptor (IL-2αβγ) formed by the combination of IL-2Rα, IL-2RP, and IL-2Rγ is an IL-2 high-affinity receptor (with a K_{D} of about 10 pM), the dimeric receptor (IL-2βγ) consisting of IL-2RP and IL-2Rγ is an intermediate affinity receptor (with a K_{D} of about 1 nM), and the IL-2 receptor formed solely by subunit α is a low affinity receptor.

Immune cells express dimeric or trimeric IL-2 receptors. The dimeric receptor is expressed on cytotoxic CD8⁺ T cells and natural killer (NK) cells, whereas the trimeric receptor is expressed predominantly on activated lymphocytes and CD4⁺ CD25⁺ FoxP3⁺ suppressive regulatory T cells (Treg) (Byman, O. and Sprent. J. Nat. Rev. Immunol. 12, 180-190 (2012)). Effector T cells and NK cells in a resting state are relatively insensitive to IL-2 because they do not have CD25 on the cell surface. However, Treg cells consistently express the highest level of CD25 *in vivo,* and therefore normally IL-2 would preferentially stimulate Treg cell proliferation.

IL-2 mediates multiple actions in an immune response by binding to IL-2 receptors on different cells. In one aspect, IL-2 has an stimulatory effect on the immune system, stimulating the proliferation and differentiation of T cells and natural killer (NK) cells. Therefore, IL-2 has been approved as an immunotherapeutic agent for the treatment of cancer and chronic viral infections. In another aspect, IL-2 also contributes to the maintenance of immunosuppressive CD4⁺ CD25⁺ regulatory T cells (i.e., Treg cells) (Fontenot et al., Nature Immunol 6,1142-51 (2005); D'Cruz and Klein, Nature Immunol 6,1152-59 (2005); Maloy and Powrie, Nature Immunol 6,1171-72 (2005)), causing immunosuppression due to activated Treg cells in patients.

In addition, from years of clinical practical experience, it has been found that although high doses of IL-2 can provide significant clinical efficacy in the treatment of cancer such as melanoma and kidney cancer, they can also cause drug-related serious toxic side effects including cardiovascular toxicities such as vascular leak syndrome and hypotension. Studies have shown that these toxicities most likely result from the over-activation of lymphocytes (especially T cells and NK cells) by IL-2, which stimulates the release of inflammatory factors. For example, this can cause vascular endothelial cells to contract, increasing intercellular gaps, causing the extravasation of tissue fluid and thus causing the vascular leak side effect.

Another limiting problem with the clinical use of IL-2 is that it is difficult to administer due to its extremely short half-life. As an IL-2 molecule weighs only 15 kDa, it will be eliminated primarily by glomerular filtration, having a half-life of only about 1 hour in the human body. In order to achieve a sufficiently high exposure in the human body, a large dose of IL-2 is clinically required to be infused every 8 hours. However, frequent dosing places a heavy burden on patients, and more importantly, infusion of large doses of IL-2 can cause high peak plasma concentrations (Cₘₐₓ), which is probably another critical factor contributing to drug toxicity. Rodrigo Vazquez-Lombardi et al. (Nature Communications, 8:15373, DOI: 10.1038/ncomms15373) have proposed preparing an interleukin-2-Fc fusion to improve the pharmacodynamic properties of interleukins. However, the expression yield of the fusion protein is low, and it easily forms aggregates.

In the production of IL-2, native IL-2 molecules are very hard to express in mammalian cells (CHO or HEK293) due to the characteristics of its amino acid sequence, and the stability of the molecules is poor. Therefore, Proleukin, the currently approved IL-2 molecule on the market, is produced in prokaryotic bacterial expression systems. However, IL2-Fc fusion proteins cannot be expressed in bacterial systems. Therefore, there is a need in the art to improve the expression of IL-2 molecules and IL-2-Fc molecules in mammalian cells.

Several schemes for engineering IL-2 molecules have been proposed in the art. For example, Helen R. Mott et al. disclosed a mutant protein of human IL-2, F42A, which has an eliminated ability to bind to IL-2Rα. Rodrigo Vazquez-Lombardi et al. (Nature Communications, 8:15373, DOI: 10.1038/ncomms15373) have also proposed a triple mutant human IL-2 mutant protein IL-2^{3X} with an eliminated ability to bind to IL-2Rα, which has residue mutations R38D+K43E+E61R at amino acid residue positions 38, 43 and 61, respectively. CN1309705A discloses mutations at positions D20, N88 and Q126 that result in reduced binding of IL-2 to IL-2Rβγ. These mutant proteins are still deficient in their pharmacokinetic and/or pharmacodynamic properties and also confronted with low expression yields and/or poor molecular stability when expressed in mammalian cells.

In view of the above-mentioned problems associated with IL-2 immunotherapy and production, there remains a need in the art to further develop new IL-2 molecules with improved properties, particularly IL-2 molecules that are advantageous to production and purification and have improved pharmacokinetic and pharmacodynamic properties.

### BRIEF SUMMARY

The present invention satisfies the above needs by providing a long-acting IL-2 mutant protein molecule with improved druggability and/or an improved IL-2 receptor binding property.

Thus, in one aspect, the present invention provides a novel IL-2 mutant protein. In some embodiments, the IL-2 mutant protein disclosed herein has one or more of the following properties, preferably at least properties (i) and (ii):
(i) improved druggability, particularly improved expression yield and/or purification performance when expressed in mammalian cells;
(ii) weakened binding to IL-2Rβγ; and
(iii) reduced or eliminated binding to IL-2Rα.

In some embodiments, the IL-2 mutant protein disclosed herein has properties (i) and (ii) and maintains binding to IL-2Rα relative to the wild-type IL-2 protein. In other embodiments, the IL-2 mutant protein disclosed herein has properties (i) to (iii).

In some embodiments, the present invention provides an IL-2 mutant protein comprising an IL-2Rβγ binding interface mutation and further comprising an IL-2Rα binding interface mutation and/or a shortened B'C' loop region. In some preferred embodiments, the present invention provides an IL-2 mutant protein comprising an IL-2Rβγ binding interface mutation and a shortened B'C' loop region but not comprising an IL-2Rα binding interface mutation.

In addition, the present invention provides a fusion protein, a dimer protein and an immunoconjugate comprising the IL-2 mutant protein, a pharmaceutical composition, and a combination product; a nucleic acid encoding same, and a vector and a host cell comprising the nucleic acid; and a method for producing the IL-2 mutant protein, the fusion protein, the dimer protein and the immunoconjugate disclosed herein.

Furthermore, the present invention further provides a method for treating diseases, and a method and use for stimulating the immune system in a subject using the IL-2 mutant protein, the fusion protein, the dimer protein and the immunoconjugate disclosed herein.

The present invention is further illustrated in the following drawings and specific embodiments. However, these drawings and specific embodiments should not be construed as limiting the scope of the present invention, and modifications easily conceived by those skilled in the art will be included in the spirit of the present invention and the protection scope of the appended claims.

### BRIEF DESCRIPTION OF THE DRAWINGS

FIG. 1 shows the crystal structure of a complex of IL-2 and IL-2Rα (PDB: 1Z92).
FIG. 2 shows (A) the crystal structure of IL-2 (PBD: 2ERJ) and (B) the B'C' loop structure superpose of human IL-2 and human IL-15.
FIG. 3 shows IL-2 mutant proteins screened from the mutant library IBYDL029 and sequences thereof.
FIG. 4 shows the crystal structures of IL-2 and IL-2Rβγ (PBD: 2ERJ) and a contact interface thereof.
FIG. 5 shows molecular formats of an IL-2-Fc dimer protein.
FIG. 6 shows the structures of some exemplary IL-2 weakened molecules of the present invention.
FIG. 7 shows signal curves of activation of p-STATS by selected and constructed IL-2^{mutant}-Fc dimer proteins on (A-E) non-activated normal T lymphocytes CD4⁺ T cells and CD8⁺ T cells, as well as on (F) activated T lymphocytes CD4⁺ CD25⁺ T cells.
FIG. 8 shows signal curves of activation of p-STATS by weakened IL-2^{mutant}-Fc dimer proteins on different lymphocyte subpopulations, relative to a control rhIL-2 protein.
FIG. 9 shows the effects of IL-2-Fc dimer proteins constructed from weakened IL-2^{mutant} molecules with reduced binding affinity for CD25, when administered in tumor-bearing C57 mice, on (A) the tumor volume; and (B and C) the body weight and changes in the body weight of the animals.
FIG. 10 shows the effects of IL-2-Fc dimer proteins constructed from weakened IL-2^{mutant} molecules that maintain binding affinity for CD25, when administered in tumor-bearing C57 mice, on (A) the tumor volume; and (B and C) the body weight and changes in the body weight of the animals.
FIG. 11 shows (A and B) the measurement results of the body weight and changes in the body weight of mice and (C-F) the results of Treg, NK, CD4⁺ and CD8⁺ T cell assays in blood, before and on days 3 and 7 after the administration of IL-2^{mutant}-Fc dimer proteins.

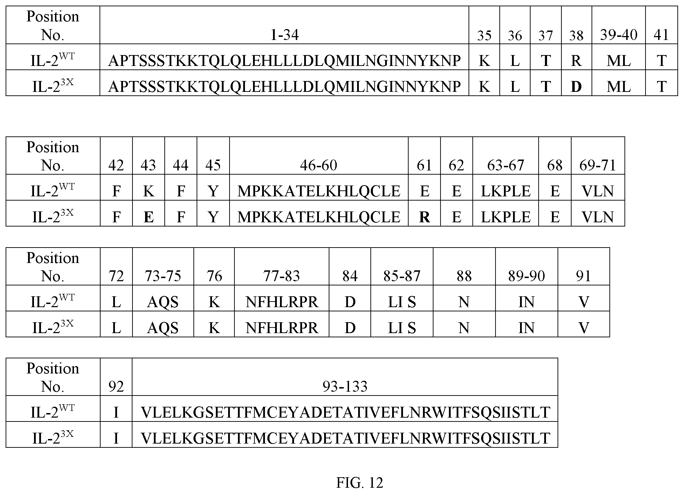

FIG. 12 shows the amino acid sequence of the wild-type IL-2 protein IL-2^{WT} (SEQ ID NO: 1) and the numbering of amino acid residues thereof, and shows the sequence alignment with the mutant protein IL-2^{3X}.

### DETAILED DESCRIPTION

Unless defined otherwise, all technical and scientific terms used herein have the same meaning as commonly understood by those of ordinary skill in the art. For the purposes of the present invention, the following terms are defined below.

The term "about" used in combination with a numerical value is intended to encompass the numerical values in a range from a lower limit less than the specified numerical value by 5% to an upper limit greater than the specified numerical value by 5%.

The term "and/or" should be understood to refer to any one of the options or any two or more of the options.

As used herein, the term "comprise" or "include" is intended to include the described elements, integers or steps, but not to exclude any other elements, integers or steps. As used herein, the term "comprise" or "include", unless indicated otherwise, also encompasses the situation where the entirety consists of the described elements, integers or steps. For example, when an IL-2 mutant protein "comprising" or "including" a mutation or a combination of mutations is mentioned, it is also intended to encompass IL-2 mutant proteins having the mutation or combination of mutations only.

As used herein, wild-type "interleukin-2" or "IL-2" refers to a parent IL-2 protein, preferably a naturally-occurring IL-2 protein, e.g., a native IL-2 protein derived from a human, mouse, rat, or non-human primate, serving as a template to which a mutation or a combination of mutations disclosed herein is introduced, including both unprocessed (e.g., without the removal of the signal peptide) and processed (e.g., with the removal of the signal peptide) forms. A full-length native human IL-2 sequence comprising a signal peptide is set forth in SEQ ID NO: 2 and the sequence of its mature protein is set forth in SEQ ID NO: 3. In addition, this term also includes naturally-occurring allelic and splice variants, isotypes, homologs, and species homologs of IL-2. This term also includes variants of native IL-2, which may, for example, have at least 95%-99% or more identity to the native IL-2 or have no more than 1-10 or 1-5 amino acid mutations (e.g., conservative substitutions) and preferably have substantially the same binding affinity for IL-2Rα and/or IL2Rβγ as the native IL-2 protein. Therefore, in some embodiments, compared to the native IL-2 protein, the wild-type IL-2 protein may comprise amino acid mutations that do not affect its binding to the IL-2 receptor. For example, a native human IL-2 protein (UniProt: P60568) with a mutation C125S introduced at position 125 is a wild-type IL-2 protein disclosed herein. An example of a wild-type human IL-2 protein comprising the C125S mutation is set forth in SEQ ID NO: 1. In some embodiments, the wild-type IL-2 sequence may have at least more than 85% or 95%, or even at least 96%, 97%, 98%, or 99% amino acid sequence identity to the amino acid sequence set forth in SEQ ID NO: 1, 2, or 3.

As used herein, the amino acid mutation may be an amino acid substitution, deletion, insertion, and addition. Any combination of substitution, deletion, insertion and addition may be made to obtain a final mutant protein construct with the desired properties, such as reduced binding affinity for IL-2Rα and/or improved druggability and/or weakened IL-2Rβγ. Amino acid deletions and insertions include amino- and/or carboxyl-terminal deletions and insertions of a polypeptide sequence, as well as deletions and insertions within the polypeptide sequence. For example, an alanine residue can be deleted at position 1 of a full-length human IL-2, or one or more amino acids can be deleted from a B'C' loop region to shorten the length of the loop region. In some embodiments, the preferred amino acid mutations are amino acid substitutions, e.g., the combination of single amino acid substitutions or the replacement of segments of an amino acid sequence. For example, the entirety or a part of the B'C' loop region sequence of the wild-type IL-2 can be replaced with a different sequence, preferably to obtain a shortened B'C' loop region sequence.

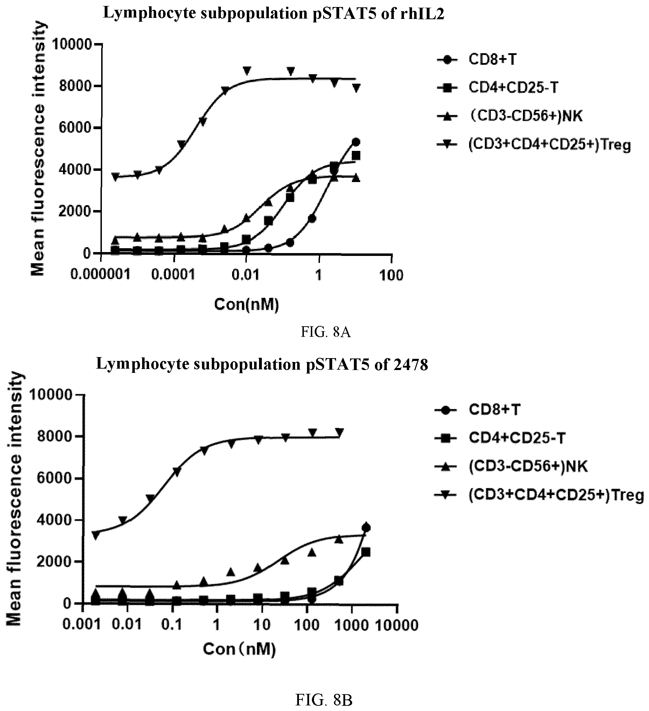

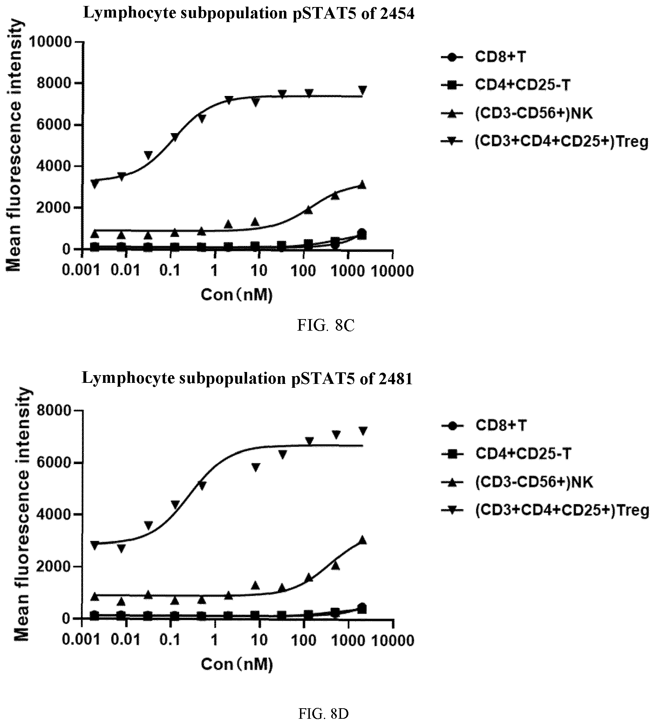

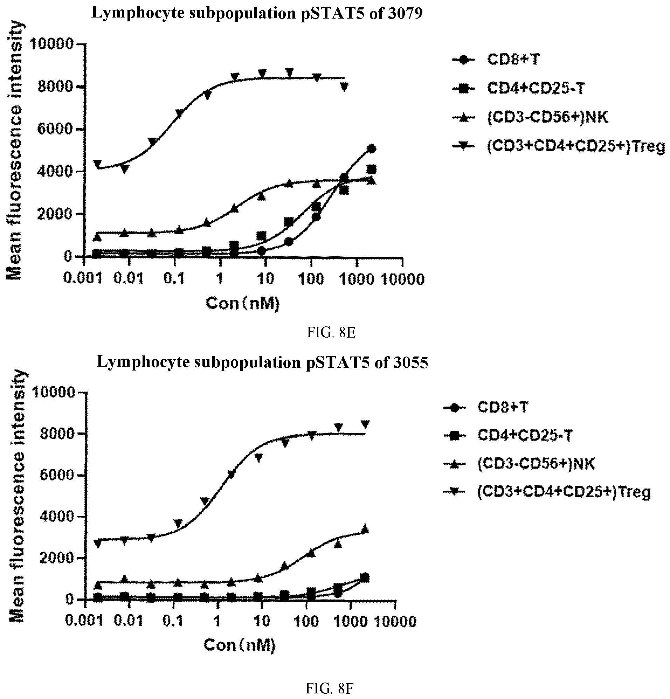

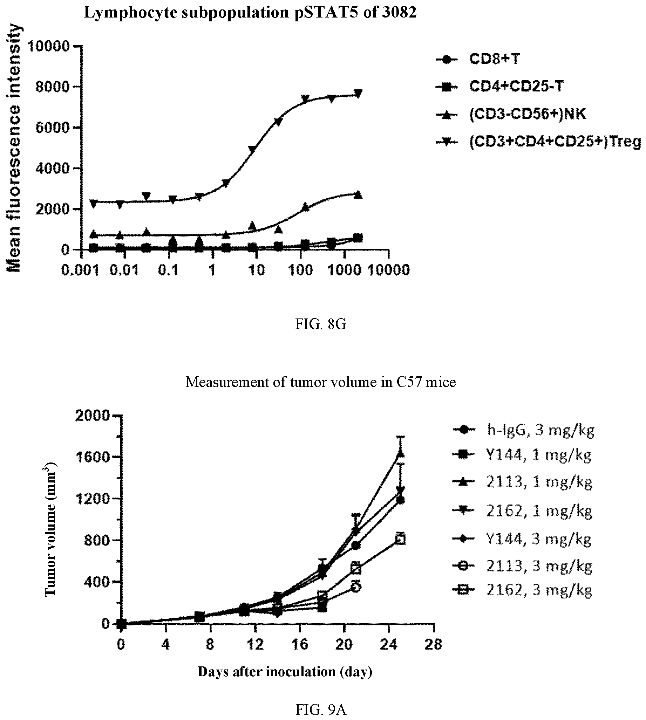

In the present invention, when the amino acid position in the IL-2 protein or IL-2 sequence segments is mentioned, it is determined by referring to the amino acid sequence of the wild-type human IL-2 protein (also referred to as IL-2^{WT}) set forth in SEQ ID NO: 1 (as shown in FIG. 8). The corresponding amino acid position in other IL-2 proteins or polypeptides (including full-length sequences or truncated fragments) can be identified by amino acid sequence alignment with SEQ ID NO: 1. Therefore, in the present invention, unless otherwise stated, an amino acid position in an IL-2 protein or polypeptide is an amino acid position numbered according to SEQ ID NO: 1. For example, when "F42" is mentioned, it refers to a phenylalanine residue F at position 42 of SEQ ID NO: 1, or an amino acid residue at corresponding positions in other IL-2 polypeptide sequences by alignment. To perform a sequence alignment for determining an amino acid position, Basic Local Alignment Search Tool available at https://blast.ncbi.nlm.nih.gov/Blast.cgi can be used with default parameters.

When an IL-2 mutant protein is mentioned herein, a single amino acid substitution is described as [original amino acid residue/position/amino acid residue for substitution]. For example, the substitution of lysine at position 35 with glutamate can be indicated as K35E. When there are multiple optional amino acid substitutions (e.g., D and E) at a given position (e.g., K35), the amino acid substitutions can be indicated as K35D/E. Correspondingly, single amino acid substitutions can be linked together by "+" or "-" to indicate a combinatorial mutation at multiple given positions. For example, the combinatorial mutation of positions K35E, T37E, R38E and F42A can be indicated as K35E+T37E+R38E+F42A or K35E-T37E-R38E-F42A.

As used herein, the "percent sequence identity" can be determined by comparing two optimally aligned sequences over a comparison window. Preferably, the sequence identity is determined over the full length of a reference sequence (e.g., SEQ ID NO: 1). Methods of sequence alignment for comparison are well known in the art. Algorithms suitable for determining the percent sequence identity include, for example, BLAST and BLAST 2.0 algorithms (see Altschul et al., Nuc. Acids Res.25: 3389-402, 1977 and Altschul et al., J.mol. Biol. 215: 403-10, 1990). Software for performing BLAST analysis is publicly available from the National Center for Biotechnology Information. For the purpose of the present application, the percent identity can be determined by using Basic Local Alignment Search Tool available at https://blast.ncbi.nlm.nih.gov/Blast.cgi with default parameters.

As used herein, the term "conservative substitution" means an amino acid substitution that does not adversely affect or alter the biological function of a protein/polypeptide comprising an amino acid sequence. For example, a conservative substitution may be introduced by standard techniques known in the art, such as site-directed mutagenesis and PCR-mediated mutagenesis. A typical conservative amino acid substitution involves a substitution of an amino acid by another amino acid having similar chemical properties (e.g., charge or hydrophobicity). Conservative replacement tables of functionally similar amino acids are well known in the art. In the present invention, residues for conservative substitutions are from the conservative substitution table X below, particularly from the preferred residues for conservative amino acid substitutions in Table X.

**Table X**

| Original residues | Exemplary substitution | Preferred conservative amino acid substitution |
|---|---|---|
| Ala (A) | Val; Leu; Ile | Val |
| Arg (R) | Lys; Gln; Asn | Lys |
| Asn (N) | Gln; His; Asp; Lys; Arg | Gln |
| Asp (D) | Glu; Asn | Glu |
| Cys (C) | Ser; Ala | Ser |
| Gln (Q) | Asn; Glu | Asn |
| Glu (E) | Asp; Gln | Asp |
| Gly (G) | Ala | Ala |
| His (H) | Asn; Gln; Lys; Arg | Arg |
| Ile (I) | Leu; Val; Met; Ala; Phe; Nle | Leu |
| Leu (L) | Nle; Ile; Val; Met; Ala; Phe | Ile |
| Lys (K) | Arg; Gln; Asn | Arg |
| Met (M) | Leu; Phe; Ile | Leu |
| Phe (F) | Trp; Leu; Val; Ile; Ala; Tyr | Tyr |
| Pro (P) | Ala | Ala |
| Ser (S) | Thr | Thr |
| Thr (T) | Val; Ser | Ser |
| Trp (W) | Tyr; Phe | Tyr |
| Tyr (Y) | Trp; Phe; Thr; Ser | Phe |
| Val (V) | Ile; Leu; Met; Phe; Ala; Nle | Leu |

For example, relative to one of SEQ ID NOs: 1-3, the wild-type IL-2 protein may have conservative amino acid substitutions, or only have conservative amino acid substitutions; and in one preferred embodiment, the conservative substitutions involve no more than 10 amino acid residues, e.g., 1, 2, 3, 4, 5, 6, 7, 8, 9 or 10 residues. As another example, relative to the IL-2 mutant protein sequences specifically given herein (e.g., any one of SEQ ID NOs: 37-638), the IL-2 mutant protein disclosed herein may have conservative amino acid substitutions, or only have conservative amino acid substitutions; and in one preferred embodiment, the conservative substitutions involve no more than 10 amino acid residues, e.g., 1, 2, 3, 4, 5, 6, 7, 8, 9 or 10 residues.

"Affinity" or "binding affinity" refers to the inherent binding ability that reflects the interaction between members of a binding pair. The affinity of a molecule X for its binding partner Y can be represented by an equilibrium dissociation constant (K_{D}), which is the ratio of a dissociation rate constant (k_{dis}) to an association rate constant (kₒₙ). The binding affinity can be measured by common methods known in the art. One particular method for measuring the affinity is the ForteBio affinity assay technique described herein.

As used herein, an antigen-binding molecule is a polypeptide molecule that can specifically bind to an antigen, e.g., an immunoglobulin molecule, an antibody, or an antibody fragment (e.g., an Fab fragment and an scFv fragment).

As used herein, an antibody Fc fragment refers to a C-terminus region of an immunoglobulin heavy chain that contains at least a portion of the constant region, and may include Fc fragments of native sequences and variant Fc fragments. Fc fragments of native sequences include various naturally-occurring Fc sequences of immunoglobulins, such as various Ig subclasses or the Fc regions of allotypes thereof (Gestur Vidarsson et al., IgG subclasses and allotypes: from structure to effector functions, 20 October 2014, doi: 10.3389/fimmu.2014.00520.). In one embodiment, the heavy chain Fc fragment of human IgG extends from Cys226 or Pro230 of the heavy chain to the carboxy-terminus. In another embodiment, the C-terminal lysine (Lys447) of the Fc fragment may or may not be present. In other embodiments, the Fc fragment is a variant Fc fragment comprising a mutation, for example, a L234A-L235A mutation. Unless otherwise indicated herein, amino acid residues in the Fc fragment are numbered according to the EU numbering system, also called the EU index, as described in Kabat, E.A. et al., Sequences of Proteins of Immunological Interest, 5th edition, Public Health Service, National Institutes of Health, Bethesda, MD (1991), NIH Publication 91-3242. In some embodiments, the antibody Fc fragment may bear an IgG1 hinge sequence or a part of the IgG1 hinge sequence at the N-terminus, e.g., the sequence of E216 to T225 or the sequence of D221 to T225 according to the EU numbering. Mutations may be comprised in the hinge sequence.

The IL-2 protein is a member of the short chain type I cytokine family with four α-helical bundles (A, B, C, and D). As used herein, the terms "B'C' loop", "B'C' loop region" and "B'C' loop sequence" are used interchangeably, referring to a linker sequence between the B and C helices of the IL-2 protein. The B'C' loop sequence of an IL-2 protein can be determined by performing analysis of the IL-2 crystal structure (e.g., PDB: 2ERJ). For the purpose of the present invention, according to the numbering of SEQ ID NO: 1, the B'C' loop sequence refers to a sequence linking the residue at position 72 to the residue at position 84 in the IL-2 polypeptide. In the wild-type IL-2 proteins set forth in SEQ ID NOs: 1, 2 and 3, the linker sequence comprises 11 amino acids, namely A73-R83. Accordingly, as used herein, the term "shortened loop region" or "shortened B'C' loop region" means that a mutant protein has a B'C' loop sequence with a reduced length relative to the wild-type IL-2 protein, i.e., the linker sequence between the amino acid residues aa72 and aa84 is shortened according to the numbering of SEQ ID NO: 1. A "shortened loop region" can be achieved by replacement or truncation of the loop sequence. The replacement or truncation may occur in any region or portion of the B'C' loop sequence. For example, the replacement or truncation may be the replacement of the sequence A73-R83 in the loop region or the truncation of the sequence by one or more amino acid residues at the C-terminus. As another example, the replacement or truncation may be the replacement of the sequence Q74-R83 in the loop region or the truncation of the sequence by one or more amino acid residues at the C-terminus. After the replacement or truncation, if necessary, a single amino acid substitution, e.g., an amino acid substitution for eliminating glycosylation and/or a reverse mutation, can be further introduced into the loop region sequence to further improve the performance of the mutant protein, e.g., the druggability. Therefore, herein, the mutated shortened B'C' loop region can be described through a sequence linking the residue at position 72 to the residue at position 84 after a mutation is introduced.

As used herein, an "IL-2Rα binding interface" mutation refers to a mutation occurred at amino acid sites where IL-2 interacts with IL-2Rα (i.e., CD25). These interaction sites can be determined by analyzing the crystal structure of the complex of IL-2 and its receptor (e.g., PDB: 1Z92). In some embodiments, the mutation refers particularly to mutations in the region of amino acid residues 35-72 of IL-2, particularly to mutations at the following amino acid sites: 35, 37, 38, 41, 42, 43, 45, 61, 62, 68 and 72. Preferably, an IL-2 protein comprising the mutation has reduced or eliminated binding to IL-2Rα compared to the corresponding protein before introduction of the mutation.

As used herein, an "IL-2βγ binding interface" mutation refers to a mutation occurred at amino acid sites where IL-2 interacts with IL-2Rβγ (i.e., CD122 and CD132). These interaction amino acid sites can be determined by analyzing the crystal structure of the complex of IL-2 and its receptor (e.g., PDB: 2ERJ). In some embodiments, the mutation refers particularly to mutations in the regions of amino acid residues 12-20, 84-95 and 126 of IL-2, particularly to mutations at the following amino acid sites: 12, 15, 16, 19, 20, 84, 87, 88, 91, 92, 95 and 126. Preferably, an IL-2 protein comprising the mutation has weakened binding to IL-2Rβγ compared to the corresponding protein before introduction of the mutation.

As used herein, with respect to binding to an IL-2Rβγ receptor, a "weakened" IL-2 protein molecule means introducing a mutation into a binding interface to IL-2Rβγ that leads to reduced binding affinity for the IL-2Rβγ receptor relative to the corresponding IL-2 protein before the introduction of the mutation. Further preferably, the weakened molecule has reduced activation activity for T cells (e.g., CD25⁻ T cells or CD25⁺ T cells) and/or NK cells relative to the corresponding protein. For example by measuring the ratio of EC₅₀ values of activation of pSTAT5 signals in T cells by the weakened molecule and the corresponding protein, the activation activity may be reduced by 5 times or more, e.g., 10 times or more, or 50 times or more, or 100 times or more, or even 1000 times or more. For example, the activation activity of the weakened molecule for T cells can be reduced by 10-50 times, or 50-100 times, or 100-1000 times, or more, relative to the corresponding protein. Thus, in the present invention, in some embodiments, the weakened molecule of the present invention has "weakened" binding affinity for the IL-2Rβγ receptor and "weakened" activation activity for T cells.

All aspects of the present invention are further detailed in the following sections.

### 1. IL-2 Mutant Protein Disclosed Herein

### Advantageous biological properties of the IL-2 mutant protein disclosed herein

The inventors have found through long-term research that the following molecular mutations and engineering can be combined and implemented to improve the efficacy of IL-2, reduce the toxic side effects of IL-2 and achieve a good production performance at the same time:
(i) introducing a certain residue mutation into the binding interface of IL-2 to the IL-2Rβγ receptor to weaken the binding to the IL-2Rβγ receptor and to down-regulate the activity of IL-2 to some extent. By including such mutations that weaken the binding to the IL-2Rβγ receptor, the IL-2 mutant protein disclosed herein can activate lymphocytes to kill tumors, while avoiding the release of a large amount of inflammatory factors, and thus drug-related toxicity, caused by over-activation of lymphocytes. In addition, by reducing the binding affinity of the IL-2 mutant protein disclosed herein for the IL-2 receptors extensively occurred on lymphocytes, the weakening mutations can also reduce the clearance of the IL-2 mutant protein mediated by the IL-2 receptors and prolong the period of action of the IL-2 mutant protein;
(ii) constructing the IL-2 mutant protein disclosed herein into an IL-2-Fc dimer. The formation of this dimer can increase the molecular weight of the IL-2 mutant protein disclosed herein, greatly reducing renal clearance and further extending the half-life of the IL2-Fc fusion protein by FeRn-mediated *in vivo* recycling. Thus, the high peak plasma concentration problem caused by the short half-life and high-frequency large-dose administration of IL-2 is overcome;
(iii) engineering the B'C' loop structure of IL-2, for example, by replacing with the loop of the IL-15 molecule or by truncating the B'C' loop of the IL-2 molecule. The B'C' loop mutation can significantly enhance the stability of the B'C' loop structure in the IL-2 mutant protein disclosed herein, and significantly improve the production performance of the IL-2 mutant protein and the IL-2-Fc dimeric molecule constructed thereof, e.g., significantly improving the expression yield and purity.

Furthermore, the inventors have found that in the mutant protein disclosed herein, the binding activity of the IL-2 mutant protein to IL-2Rα can be regulated as needed, while the excellent properties described above are kept, to meet different drug-forming requirements of IL-2 in multiple aspects such as in anti-tumor therapy or in the treatment of autoimmune diseases, and thus to further impart excellent pharmacodynamic properties to the mutant protein disclosed herein.

For example, the mutant protein disclosed herein can maintain substantially comparable binding activity to IL-2Rα as the wild-type IL-2; or can combine the following mutations: (iv) one or more specific mutations at the binding interface of IL-2 to the IL-2Rα receptor, to change the binding performance of the IL-2 mutant protein to IL-2Rα.

Thus, through the engineering of the sequence, the IL-2-Fc molecules disclosed herein, in one aspect, have weakened binding affinity for the IL2Rβ/γ receptor and achieve more excellent pharmacokinetic experimental results and pharmacodynamic results, and, in another aspect, have significantly improved druggability such as the protein expression yield and purity.

Thus, the present invention provides an IL-2 mutant protein with improved druggability and improved IL-2 receptor binding properties. IL-2-Fc molecules comprising the IL-2 mutant protein disclosed herein can effectively avoid excessive release of inflammatory factors caused by strong agonizing of lymphocytes, and has more stable and long-acting pharmacokinetic properties. Thus, a sufficiently high drug exposure can be achieved in the human body with a lower single dose, which avoids drug-related toxicity resulting from high Cₘₐₓ. Furthermore, it is more significant that, although the long-acting IL-2-Fc molecule of the present invention has weakened immunostimulatory activity for lymphocytes relative to native IL-2, the *in vivo* effective drug concentration of the molecule of the present invention is more lasting due to the significant improvements to the pharmacokinetic properties and it can achieve a long period of constant stimulation of lymphocytes, a comparable pharmacodynamic effect to or even a better pharmacodynamic effect than native IL-2 molecules, and better anti-tumor efficacy and tolerance in animals.

### Improved druggability

In some embodiments, the IL-2 mutant protein disclosed herein has improved druggability. For example, when expressed in mammalian cells such as HEK293 cells or CHO cells, particularly in the form of an Fc fusion protein, the IL-2 mutant protein has one or more properties selected from the following: (i) a superior expression yield to the wild-type IL-2 protein; and (ii) ease of purification to a higher protein purity.

In some embodiments disclosed herein, the IL-2 mutant protein disclosed herein shows an increased expression level compared to the wild-type IL-2. In some embodiments disclosed herein, the increased expression occurs in a mammalian cell expression system. The expression level can be determined by any suitable method that allows for quantitative or semi-quantitative analysis of the amount of recombinant IL-2 protein in cell culture supernatant, preferably the supernatant purified by one-step affinity chromatography. For example, the amount of recombinant IL-2 protein in a sample can be assessed by Western blotting or ELISA. In some embodiments, the expression yield of the IL-2 mutant protein disclosed herein in mammalian cells is increased by at least 1.1 times, or at least 1.5 times, or at least 2 times, 3 times or 4 times or more, or at least 5, 6, 7, 8 or 9 times, or even 10 times or more, e.g., about 10, 15, 20, 25, 30 or 35 times, compared to that of the wild-type IL-2.

In some embodiments, as shown by determining the purity of the protein purified by protein A affinity chromatography, the IL-2 mutant protein-Fc fusion disclosed herein has higher purity, relative to the wild-type IL-2 protein fusion. In some embodiments, the purity of the protein is determined by SEC-HPLC. In some preferred embodiments, the IL-2 mutant protein-Fc fusion disclosed herein can have a purity of up to 70%, or 80%, or 90% or higher, preferably 92%, 93%, 94%, 95%, 98% or 99% or higher, after being purified by one-step protein A affinity chromatography.

In some embodiments, as shown by determining the purity of the protein purified by protein A affinity chromatography, the IL-2-Fc dimer protein disclosed herein has higher purity, relative to the corresponding IL-2-Fc dimer protein formed of the wild-type IL-2 protein. In some embodiments, the purity of the protein is determined by SEC-HPLC. In some preferred embodiments, the IL-2-Fc dimer protein disclosed herein can have a purity of up to 70%, or 80%, or 90% or higher, preferably 92%, 93%, 94%, 95%, 98% or 99% or higher, after being purified by one-step protein A affinity chromatography.

### Weakened binding to IL-2βγ receptor

By introducing a mutation into the binding interface to IL-2Rβγ, in some embodiments, the IL-2 mutant protein disclosed herein has weakened binding affinity for IL-2βγ relative to the corresponding protein before the introduction of the mutation.

In some embodiments, the IL-2 mutant protein disclosed herein has reduced binding affinity for the IL-2RP and/or IL-2Rβγ receptor relative to that before weakening by introducing an IL-2Rβγ binding interface mutation. In some embodiments, the IL-2 mutant protein disclosed herein has reduced binding affinity for the IL-2RP receptor relative to that before weakening; for example, the binding affinity is reduced by 1-20 times or more. In some embodiments, binding to the IL-2RP receptor is eliminated. In some embodiments, the IL-2 mutant protein disclosed herein has reduced binding affinity for the IL-2Rβγ receptor relative to that before weakening; for example, the binding affinity is reduced by 1-100 times or more. In some embodiments, the IL-2 mutant protein disclosed herein does not bind to the IL-2RP receptor, but is still capable of binding to the IL-2Rβγ receptor. Preferably, the binding to the IL-2Rβγ receptor is reduced by 1-100 times, e.g., about 20-80 times, compared to that before weakening. The binding affinity can be determined by measuring the equilibrium dissociation constant (K_{D}) of the binding of the IL-2 mutant protein disclosed herein, such as the IL-2 mutant protein disclosed herein fused to an Fc fragment or the dimeric molecule thereof, to the IL-2RP or IL-2Rβγ receptor using the ForteBio affinity assay technique.

By introducing mutations into the binding interface to IL-2Rβγ, in some embodiments, the IL-2 mutant protein disclosed herein, relative to the corresponding protein before the introduction of the mutations, has weakened IL-2 activity, e.g., at least one IL-2 activity selected from the following:
- reduced activation of T cells (e.g. CD4⁺ and CD8⁺ T cells, e.g. CD4⁺/CD8⁺ CD25⁻T cells, or CD4⁺ CD25⁺ T cells), compared to that before weakening;
- reduced activation of NK cells, compared to that before weakening; and
- reduced IL-2-stimulated release of inflammatory factors from T cells/NK cells, compared to that before weakening.

In one embodiment, the IL-2 mutant protein disclosed herein leads to reduced IL-2-mediated activation and/or proliferation of lymphocytes (e.g., T cells and/or NK cells) relative to that before weakening. In one embodiment, the lymphocytes are CD4⁺ and CD8⁺ T cells, such as CD25⁻ T cells. In one embodiment, in the STATS phosphorylation assay, the ability of the IL-2 mutant protein to activate CD4⁺ and CD8⁺ T cells is identified by measuring the activation of STATS phosphorylation signals by the IL-2 mutant protein in lymphocytes such as T cells or NK cells. For example, as described in the examples of this application, STATS phosphorylation in cells can be analyzed by flow cytometry to determine the half maximum effective concentration (EC₅₀). For example, by measuring the ratio of EC₅₀ values of activation of STATS phosphorylation signals by the IL-2 weakened molecule disclosed herein and the corresponding protein, the IL-2 mutant molecule disclosed herein has "weakened" activation activity for T cells. According to the ratio, the activation activity of the IL-2 mutant molecule disclosed herein for T cells can be reduced by, e.g., 5 times or more, e.g., 10 times or more, or 50 times or more, or 100 times or more, or even 1000 times or more. For example, the activation activity of the IL-2 mutant molecule disclosed herein for T cells can be reduced by 10-50 times, or 50-100 times, or 100-1000 times, or more, relative to the corresponding protein. In some preferred embodiments, the IL-2 mutant protein disclosed herein has reduced cell surface IL-2 receptor-mediated clearance of IL-2 and an increased *in vivo* half-life, relative to the wild-type IL-2.

In some preferred embodiments, the IL-2 mutant protein disclosed herein has reduced *in vivo* toxicity mediated by IL-2 and its receptors relative to the wild-type IL-2.

### Maintained or altered binding to IL-2Rα receptor

The IL-2 protein triggers signaling and functions by interacting with IL-2 receptors. Wild-type IL-2 exhibits different affinities for different IL-2 receptors. IL-2β and IL-2γ receptors having a low affinity for wild-type IL-2 are expressed on resting effector cells, including CD8⁺ T cells and NK cells. IL-2Rα receptors having a high affinity for wild-type IL-2 are expressed on regulatory T cell (Treg) cells and activated effector cells. Due to high affinity, the wild-type IL-2 will preferentially bind to IL-2Rα on the cell surface and then recruit IL-2Rβγ. Treg cells and activated effector cells are stimulated by downstream p-STAT5 signals released through the IL-2Rβγ. Without being bound by theory, altering the affinity of IL-2 for the IL-2Rα receptor will alter the preference of IL-2 for preferentially activating CD25⁺ cells and the IL-2-mediated immune downregulation of Treg cells.

In some embodiments, the IL-2 mutant protein disclosed herein has a maintained or an altered ability to bind to the IL-2Rα receptor relative to the wild-type IL-2.

In some embodiments, the IL-2 mutant protein disclosed herein maintains binding to the IL-2Rα receptor relative to the wild-type IL-2. As used herein, the expression "maintain binding to the IL-2Rα receptor" means that the IL-2 mutant protein has a comparable binding activity to the IL-2Rα receptor relative to the wild-type IL-2 protein. Preferably, "comparable binding activity" means that when measured in the same manner, the binding activity values (e.g., binding affinity K_{D}) of the IL-2 mutant protein and the wild-type IL-2 protein are in a ratio between 1:20 and 20:1, preferably between 1:10 and 10:1. Preferably, the IL-2 mutant protein does not have an IL-2Rα binding interface mutation relative to the wild-type IL-2.

In some embodiments, the IL-2 mutant protein disclosed herein is a weakened IL-2 mutant molecule which maintains binding to the IL-2Rα receptor. In still other embodiments, the weakened IL-2 mutant protein disclosed herein does not have an IL-2Rα binding interface mutation relative to the wild-type IL-2. Preferably, the weakened IL-2 mutant molecule has improved selectivity for Treg and/or improved selectivity for NK cells (e.g., CD3⁻ CD56⁺ NK cells). In one embodiment, in the STATS phosphorylation assay, the selectivity of the IL-2 mutant protein for lymphocytes is identified by measuring the activation of STATS phosphorylation signals by the IL-2 mutant protein in different lymphocytes such as Treg cells, NK cells and CD4⁺ and CD8⁺ effector T cells. In one embodiment, in the STATS phosphorylation assay, the selectivity of the IL-2 mutant protein can be reflected by a dose window of the IL-2 mutant protein that selectively activates specific (one or more types of) lymphocytes without substantially activating other lymphocytes. For example, in some embodiments, the weakened IL-2 mutant protein disclosed herein may exhibit improved selectivity for Treg and/or improved selectivity for NK cells (CD3⁻ CD56⁺ NK cells) relative to that for effector T cells, such as CD25^{-/low} CD4⁺ and/or CD8⁺ effector T lymphocytes. In still other embodiments, the improved selectivity may be reflected by low drug-related toxicity of the IL-2 mutant protein.

In other embodiments, the IL-2 mutant protein disclosed herein has a mutation introduced into the binding interface to IL-2Rα relative to the wild-type IL-2, the mutation causing the IL-2 mutant protein to have reduced or eliminated binding to the IL-2Rα receptor.

In still other embodiments, the IL-2 mutant protein disclosed herein reduces the preference of IL-2 for preferentially activating CD25⁺ cells relative to the wild-type IL-2. In still other embodiments, the IL-2 mutant protein disclosed herein reduces IL-2-mediated immune downregulation of Treg cells relative to the wild-type IL-2.

In other embodiments, the IL-2 mutant protein disclosed herein has an immune downregulation effect. In still other embodiments, the IL-2 mutant protein disclosed herein can be used to treat autoimmune diseases. Therefore, in some embodiments, the IL-2 mutant protein disclosed herein has one or more improved properties selected from the following:
- maintained or altered (e.g. reduced or increased) binding affinity for a high affinity IL-2R receptor (IL-2Rαβγ), compared to the wild-type IL-2;
- maintained or altered (e.g. reduced or increased) activation of CD25⁺ cells (e.g., CD8⁺ T cells and Treg cells), compared to the wild-type IL-2;
- maintained or altered (e.g. eliminated or reduced, or increased) preference of IL-2 for preferentially activating CD25⁺ cells (e.g. Treg cells), compared to wild-type IL-2; and
- maintained or altered (e.g. reduced or increased) IL-2-induced downregulation of the immune response by Treg cells, compared to wild-type IL-2.

In some embodiments, the binding affinity of the IL-2 mutant protein disclosed herein for the IL-2Rα receptor is reduced by at least 5 times, at least 10 times, or at least 25 times, particularly at least 30 times, 50 times or 100 times or more, relative to the wild-type IL-2 (e.g., IL-2^{WT} set forth in SEQ ID NO: 1). In a preferred embodiment, the mutant protein disclosed herein does not bind to the IL-2Rα receptor. The binding affinity can be determined by measuring the equilibrium dissociation constant (K_{D}) of the binding of the IL-2 mutant protein disclosed herein, such as the IL-2 mutant protein disclosed herein fused to an Fc fragment or the dimeric molecule thereof, to the IL-2Rα receptor using the ForteBio affinity assay technique.

In one embodiment, the IL-2 mutant protein disclosed herein reduces IL-2-mediated activation and/or proliferation of CD25⁺ cells relative to the wild-type IL-2. In one embodiment, the CD25⁺ cells are CD25⁺ CD8⁺ T cells. In another embodiment, the CD25⁺ cells are Treg cells. In one embodiment, in the STATS phosphorylation assay, the ability of the IL-2 mutant protein to activate CD25⁺ cells is identified by measuring the activation of STATS phosphorylation signals by the IL-2 mutant protein in CD25⁺ cells. For example, as described in the examples of this application, STATS phosphorylation in cells can be analyzed by flow cytometry to determine the half maximum effective concentration (EC₅₀).

In one embodiment, the IL-2 mutant protein disclosed herein eliminates or reduces the preference of IL-2 for preferentially activating CD25⁺ cells relative to the wild-type IL-2. In one embodiment, the CD25⁺ cells are CD25⁺ CD8⁺ T cells. In another embodiment, the CD25⁺ cells are Treg cells. In one embodiment, in the STATS phosphorylation assay, the ability of the IL-2 mutant protein to activate CD25⁻ cells is identified by measuring the EC₅₀ values of the IL-2 mutant protein in activating STATS phosphorylation signals in CD25⁻ cells and in CD25⁺ cells, respectively. For example, the activation preference of the IL-2 mutant protein for CD25⁺ cells is determined by calculating the ratio of EC₅₀ values of the IL-2 mutant protein in activating STATS phosphorylation signals in CD25⁻ and in CD25⁺ T cells. Preferably, the preference of the mutant protein for CD25⁺ cells is reduced by at least 10 times, preferably at least 100 times, 150 times, 200 times, or 300 times or more, relative to the wild-type protein.

### The mutant protein disclosed herein

In one aspect, the present invention provides an IL-2 mutant protein, which, compared to a wild-type IL-2 (preferably a human IL-2, and more preferably an IL-2 comprising the sequence of SEQ ID NO: 1), comprises mutations:
(i) a mutation that eliminates or reduces binding affinity for an IL-2Rα receptor, at a binding interface of IL-2 to IL-2Rα, particularly at at least one position selected from positions 35, 37, 38, 41, 42, 43, 45, 61, 68 and 72; and/or
(ii) a shortened B'C' loop region (i.e., a sequence linking amino acid residues aa72 and aa84), wherein preferably, the shortened loop region has less than 10, 9, 8, 7, 6 or 5 amino acids in length, and more preferably has 7 amino acids in length; preferably, the shortened B'C' loop region leads to an improved protein expression yield and/or purity;
and comprises a mutation:
(iii) a mutation that weakens binding to an IL-2Rβγ receptor, at a binding interface of IL-2 to IL-2Rβγ, particularly at at least one position selected from positions 12, 15, 16, 19, 20, 84, 87, 88, 91, 92, 95 and 126; wherein, the amino acid positions are numbered according to SEQ ID NO: 1;
preferably, the mutant protein comprises the mutations (i) and (iii), or comprises the mutations (ii) and (iii), or comprises the mutations (i), (ii) and (iii).

### IL-2Rβγ binding interface mutation

An IL-2Rβγ binding interface mutation suitable for the mutant protein disclosed herein may be any mutation that can be combined with the other mutations of the present invention and leads to weakened binding affinity for IL-2Rβγ and/or weakened activation activity for lymphocytes (e.g., T cells/NK cells).

Examples of such mutations include, but are not limited to: mutations that leads to weakened binding to the IL-2Rβγ receptor, at the binding interface of IL-2 to IL-2Rβγ, particularly at at least one position selected from positions 12, 15, 16, 19, 20, 84, 87, 88, 91, 92, 95 and 126.

In some embodiments, the IL-2Rβγ binding interface mutation includes mutations selected from the following: L12R, L12K, L12E, L12Q, E15Q, E15R, E15A, E15S, H16N, H16T, H16Y, H16A, H16E, H16D, H16R, L19D, L19E, L19R, L19S, D20N, D20Q, D20E, D20A, D20R, D20S, D84N, D84E, D84Q, D84T, D84S, D84R, D84G, D84M, D84F, D84L, D84K, D84H, S87T, S87R, S87K, S87L, S87M, S87H, N88D, N88T, N88Q, N88R, N88E, N88K, N88H, N88M, N88S, N88L, V91I, V91L, V91D, V91E, V91N, V91Q, V91S, V91H, I92E, I92T, 192K, I92R, 192L, E95Q, E95G, E95D, E95N, Q126E, Q126D, Q126A, Q126S, D84N+E95Q, D84E+E95Q, D84T+E95Q, D84Q+E95Q, D84T+H16T, D84N+V91I, D84T+Q126E, D84N+Q126E, H16T+D84Q and H16T+V91I.

In some preferred embodiments, the IL-2Rβγ binding interface mutation includes mutations selected from the following:
D20N, D84E, D84N, D84N+E95Q, D84N+Q126E, D84N+V91I, D84Q, D84T, D84T+H16T, D84T+Q126E, E15Q, E95N, E95Q, H16N, H16N+D84N, H16T, H16T+D84Q, H16T+V91I, N88R, N88D, N88Q, N88T, Q126E and V91I.

In still other preferred embodiments, the IL-2Rβγ binding interface mutation includes
- a mutation selected from the following: D84N, E95Q and H16N; or
- a mutation selected from the following: D84Q and H16T+V91I; or
- a mutation selected from the following: D84T, Q126E, D84N+V91I and D84N+E95Q; or
- a mutation selected from the following: N88D, N88R, D20N, D84N+E95Q, D84T+H16T, D84T+Q126E, D84N+Q126E and H16T+D84Q.

In still other preferred embodiments, the IL-2Rβγ binding interface mutation includes mutations selected from the following: D20N, N88D and N88R.

### B'C' loop region mutations

In one aspect, the IL-2 mutant protein disclosed herein, relative to the wild-type IL-2, comprises a B'C' loop region mutation; preferably, the mutation leads to a B'C' loop region with increased stability; more preferably, the mutation leads to improved druggability of the IL-2 mutant protein disclosed herein, e.g., an increased expression yield and/or purity.

In some embodiments, due to the mutation introduced, the mutant protein comprises a shortened B'C' loop region (i.e., a shortened linker sequence between the amino acid residues aa72 and aa84) compared to the wild-type IL-2 (preferably the human IL-2, and more preferably the IL-2 comprising the sequence of SEQ ID NO: 1). Preferably, the shortened loop region has less than 10, 9, 8, 7, 6 or 5 amino acids in length, and more preferably has 7 amino acids in length, wherein the amino acid residues are numbered according to SEQ ID NO: 1.

Herein, B'C' loop region mutations suitable for the present invention include truncations and replacements of the B'C' loop region. In one embodiment, the mutations include truncations or replacements of the amino acid residues aa73 to aa83 in the B'C' loop region, e.g., a truncation to form A(Q/G)SKN(F/I)H, or a replacement with SGDASIH. In another embodiment, the mutations include truncations or replacements of the amino acid residues aa74 to aa83 in the B'C' loop region, e.g., a truncation to form (Q/G)SKN(F/I)H, or a replacement with GDASIH. In some embodiments, the IL-2 mutant protein disclosed herein comprises a B'C' loop chimeric mutation. The mutant protein, relative to the wild-type IL-2, comprises a substitution of the entirety or a part of the sequence linking aa72 to aa84, for example, with a short B'C' loop sequence from other four-helical short-chain cytokine family members. The short B'C' loop suitable for the substitution of the wild-type IL-2 can be identified from other four-helical short-chain cytokine IL family members, such as IL-15, IL-4, IL-21, or IL family members from non-human species such as mice, by the superpose of a crystal structure. In one embodiment, the sequence used for substitution is a B'C' loop sequence from interleukin IL-15, particularly human IL-15. In one embodiment, the substitution includes substitutions of the amino acid residues aa73 to aa83 in the B'C' loop region. In another embodiment, the substitution includes substitutions of the amino acid residues aa74 to aa83 in the B'C' loop region. Preferably, the IL-2 mutant protein disclosed herein, after the substitutions, has a B'C' loop sequence (i.e., a sequence linking aa72 to aa84) selected from the following: SGDASIH and AGDASIH.

In some embodiments, the IL-2 mutant protein disclosed herein comprises a B'C' loop truncation mutation. The mutant protein, relative to the wild-type IL-2, comprises a truncation of the sequence linking aa72 to aa84. In one embodiment, the truncation includes truncations of the amino acid residues aa73 to aa83 in the B'C' loop region. In another embodiment, the truncation includes truncations of the amino acid residues aa74 to aa83 in the B'C' loop region. For example, the sequence may be truncated by 1, 2, 3 or 4 amino acids at the C-terminus. Preferably, after the truncation, the IL-2 mutant protein disclosed herein has a B'C' loop region having a sequence of A(Q/G)SKN(F/I)H. Preferably, after the truncation, the IL-2 mutant protein disclosed herein has a B'C' loop sequence (i.e., a sequence linking aa72 to aa84) selected from the following:

| B'C' loop sequence |
|---|
| AQSKNFH |
| AGSKNFH |
| AQSANFH |
| AQSANIH |

In one preferred embodiment, the IL-2 mutant protein disclosed herein comprises a B'C' loop region sequence (i.e., a sequence linking aa72 to aa84) selected from the following: AQSKNFH, SGDASIH and AGDASIH.

For B'C' loop mutations suitable for the present invention, see also the applicant's co-pending application PCT/CN2019/107054, which is incorporated herein by reference in its entirety.

### IL-2Rα binding interface mutation

In one aspect, the IL-2 mutant protein disclosed herein, relative to the wild-type IL-2, comprises one or more mutations at the binding interface to IL-2Rα, preferably at positions 35, 37, 38, 41, 42, 43, 45, 61, 68 and 72. Preferably, the mutation eliminates or reduces binding affinity for the IL-2Rα receptor.

In some embodiments, the IL-2Rα binding interface mutation of the present invention includes a combination of mutations selected from one of the following combinations (1)-(9):

| Combinations | Mutations |
|---|---|
| 1 | K35D/**E**+T37E/**D**+R38Y/**W**+F42N/**Q**+Y45R/**K**+E61R/**K**+E68K/**R**, preferably K35D/**E**+T37E/**D**+R38**W**+F42**Q**+Y45R/**K**+E61R/**K**+E68K/**R**, and more preferably K35D/**E**+T37E/**D**+R38**W**+F42**Q**+Y45**K**+E61**K**+E68**R** |
| 2 | K35D/**E**+T37D/**E**+R38D/**E**+F42V/L/I/**A**, preferably K35D/**E**+T37D/**E**+R38D/**E**+F42V/**A**, and more preferably K35D/**E**+T37D/**E**+R38D/**E**+F42**A** |
| 3 | K35E/**D**+R38D/**E**+T41D/**E**+K43D/**E**, preferably K35E/**D**+R38D/**E**+T41**E**+K43**E** |
| 4 | K35E/**D**+T37D/**E**+R38E/**D**+K43F/**Y**+Y45**K**+L72**F**, more preferably K35E/**D**+T37D/**E**+R38E/**D**+K43**Y**+Y45**K**+L72**F** |
| 5 | K35D/**E**+R38D/**E**+T41D/**E**+K43**Y**+Y45R/**K**+L72Y/**F**, more preferably K35D/**E**+R38D/**E**+T41D/**E**+K43**Y**+Y45**K**+L72**F** |
| 6 | K35E/**D**+T37D/**E**+R38E/**D**+T41D/**E**+K43D/**E**+L72Y/**F**, more preferably K35E/**D**+T37D/**E**+R38E/**D**+T41D/**E**+K43D/**E**+L72**F** |
| 7 | K35E/**D**+T37D/**E**+R38E/**D**+K43D/**E**+L72Y/**F**, more preferably K35E/**D**+T37D/**E**+R38E/**D**+K43D/**E**+L72**F** |
| 8 | K35D/**E**+T37E/**D**+R38E/**D**+K43D/**E**+L72Y/**F**, more preferably K35D/**E**+T37E/**D**+R38E/**D**+K43D/**E**+L72**F** |
| 9 | K35D/**E**+R38E/**D**+T41D/**E**+K43D/**E**+E61R/**K**+L72Y/**F**, more preferably K35D/**E**+R38E/**D**+T41D/**E**+K43D/**E**+E61**K**+L72**F** |

In some preferred embodiments, the IL-2Rα binding interface mutation of the present invention includes or consists of the combination of mutations K35**E**+T37**E**+R38**E**+F42**A**.

In other preferred embodiments, the IL-2Rα binding interface mutation of the present invention includes or consists of the combination of mutations K35**E**+T37**E**+R38**E**.

In other preferred embodiments, the IL-2Rα binding interface mutation of the present invention includes or consists of the mutation F42**A**.

In other embodiments, the IL-2 mutant protein disclosed herein comprising the IL-2Rα binding interface mutation of the present invention has altered binding to IL-2Rα, as determined, e.g., by a ForteBio affinity assay.

For IL-2Rα binding interface mutations suitable for the present invention, see also the applicant's co-pending application PCT/CN2019/107055, which is incorporated herein by reference in its entirety.

### Preferred exemplary combinations of mutations

In some preferred embodiments, the IL-2 mutant protein disclosed herein has weakened binding to IL-2Rβγ and has improved properties selected from one or both of: (i) reduced (or eliminated) binding to IL-2Rα; and (ii) improved expression level and purity.

In some embodiments, the present invention provides an IL-2 mutant protein, which comprises, relative to the wild-type IL-2:
(a) a combination of mutations K35**E**+T37**E**+R38**E**+F42**A**;
(b) an IL-2Rβγ binding interface mutation, selected from the following:
   D20N, D84E, D84N, D84N+E95Q, D84N+Q126E, D84N+V91I, D84Q, D84T, D84T+H16T, D84T+Q126E, E15Q, E95N, E95Q, H16N, H16N+D84N, H16T, H16T+D84Q, H16T+V91I, N88D, N88R, N88Q, N88T, Q126E and V91I;
and optionally, (iv) a mutation T3A.

In some embodiments, the present invention provides an IL-2 mutant protein, which comprises, relative to the wild-type IL-2:
(a) a combination of mutations K35**E**+T37**E**+R38**E**;
(b) an IL-2Rβγ binding interface mutation, selected from the following:
   D20N, D84E, D84N, D84N+E95Q, D84N+Q126E, D84N+V91I, D84Q, D84T, D84T+H16T, D84T+Q126E, E15Q, E95N, E95Q, H16N, H16N+D84N, H16T, H16T+D84Q, H16T+V91I, N88D, N88R, N88Q, N88T, Q126E and V91I;
and optionally, (iv) a mutation T3A.

In some embodiments, the present invention provides an IL-2 mutant protein, which comprises, relative to the wild-type IL-2:
(a) a mutation F42A;
(b) an IL-2Rβγ binding interface mutation, selected from the following:
   D20N, D84E, D84N, D84N+E95Q, D84N+Q126E, D84N+V91I, D84Q, D84T, D84T+H16T, D84T+Q126E, E15Q, E95N, E95Q, H16N, H16N+D84N, H16T, H16T+D84Q, H16T+V91I, N88D, N88R, N88Q, N88T, Q126E and V91I;
and optionally, (iv) a mutation T3A.

In some embodiments, the present invention provides an IL-2 mutant protein, which comprises, relative to the wild-type IL-2:
(a) a B'C' loop region sequence AQSKNFH;
(b) an IL-2Rβγ binding interface mutation, selected from the following:
   D20N, D84E, D84N, D84N+E95Q, D84N+Q126E, D84N+V91I, D84Q, D84T, D84T+H16T, D84T+Q126E, E15Q, E95N, E95Q, H16N, H16N+D84N, H16T, H16T+D84Q, H16T+V91I, N88D, N88R, N88Q, N88T, Q126E and V91I;
and optionally, (iv) a mutation T3A. Preferably, the IL-2 mutant protein maintains an ability to bind to the IL-2Rα receptor relative to the wild-type IL-2 protein.

In some embodiments, the present invention provides an IL-2 mutant protein, which comprises, relative to the wild-type IL-2:
(a) a B'C' loop region sequence SGDASIH or AGDASIH;
(b) an IL-2Rβγ binding interface mutation, selected from the following:
   D20N, D84E, D84N, D84N+E95Q, D84N+Q126E, D84N+V91I, D84Q, D84T, D84T+H16T, D84T+Q126E, E15Q, E95N, E95Q, H16N, H16N+D84N, H16T, H16T+D84Q, H16T+V91I, N88D, N88R, N88Q, N88T, Q126E and V91I;
and optionally, (iv) a mutation T3A. Preferably, the IL-2 mutant protein maintains binding to the IL-2Rα receptor relative to the wild-type IL-2 protein.

In some embodiments, the IL-2 mutant protein disclosed herein comprises, relative to the wild-type IL-2:
(i) a combination of mutations K35**E**+T37**E**+R38**E**+F42**A**, **or a combination of mutations** K35**E**+T37**E**+R38**E**, **or a mutation** F42**A**;
(ii) a B'C' loop region sequence: AQSKNFH; or SGDASIH or AGDASIH;
(iii) an IL-2Rβγ binding interface mutation, selected from the following:
   D20N, D84E, D84N, D84N+E95Q, D84N+Q126E, D84N+V91I, D84Q, D84T, D84T+H16T, D84T+Q126E, E15Q, E95N, E95Q, H16N, H16N+D84N, H16T, H16T+D84Q, H16T+V91I, N88D, N88R, N88Q, N88T, Q126E and V91I;
and optionally, (iv) a mutation T3A.

In some embodiments, the IL-2 mutant protein disclosed herein comprises, relative to the wild-type IL-2:
(i) a combination of mutations K35**E**+T37**E**+R38**E**+F42**A**;
(ii) a B'C' loop region sequence: AQSKNFH;
(iii) an IL-2Rβγ binding interface mutation, selected from the following:
   D20N, D84E, D84N, D84N+E95Q, D84N+Q126E, D84N+V91I, D84Q, D84T, D84T+H16T, D84T+Q126E, E15Q, E95N, E95Q, H16N, H16N+D84N, H16T, H16T+D84Q, H16T+V91I, N88D, N88R, N88Q, N88T, Q126E and V91I;
and optionally, (iv) a mutation T3A.

In some embodiments, the IL-2 mutant protein disclosed herein comprises, relative to the wild-type IL-2:
(i) a combination of mutations K35**E**+T37**E**+R38**E**+F42**A**;
(ii) a B'C' loop region sequence: SGDASIH or AGDASIH;
(iii) an IL-2Rβγ binding interface mutation, selected from the following:
   D20N, D84E, D84N, D84N+E95Q, D84N+Q126E, D84N+V91I, D84Q, D84T, D84T+H16T, D84T+Q126E, E15Q, E95N, E95Q, H16N, H16N+D84N, H16T, H16T+D84Q, H16T+V91I, N88D, N88R, N88Q, N88T, Q126E and V91I;
and optionally, (iv) a mutation T3A.

In some embodiments, the IL-2 mutant protein disclosed herein comprises, relative to the wild-type IL-2:
(i) **a combination of mutations** K35**E**+T37**E**+R38**E**;
(ii) a B'C' loop region sequence: AQSKNFH;
(iii) an IL-2Rβγ binding interface mutation, selected from the following:
   D20N, D84E, D84N, D84N+E95Q, D84N+Q126E, D84N+V91I, D84Q, D84T, D84T+H16T, D84T+Q126E, E15Q, E95N, E95Q, H16N, H16N+D84N, H16T, H16T+D84Q, H16T+V91I, N88D, N88R, N88Q, N88T, Q126E and V91I;
and optionally, (iv) a mutation T3A.

In some embodiments, the IL-2 mutant protein disclosed herein comprises, relative to the wild-type IL-2:
(i) **a combination of mutations** K35**E**+T37**E**+R38**E**;
(ii) a B'C' loop region sequence SGDASIH or AGDASIH;
(iii) an IL-2Rβγ binding interface mutation, selected from the following:
   D20N, D84E, D84N, D84N+E95Q, D84N+Q126E, D84N+V91I, D84Q, D84T, D84T+H16T, D84T+Q126E, E15Q, E95N, E95Q, H16N, H16N+D84N, H16T, H16T+D84Q, H16T+V91I, N88D, N88R, N88Q, N88T, Q126E and V91I;
and optionally, (iv) a mutation T3A.

In some preferred embodiments, the IL-2 mutant protein disclosed herein comprises, relative to the wild-type IL-2:
(i) **a combination of mutations** K35**E**+T37**E**+R38**E**+F42**A**;
(ii) a B'C' loop region sequence SGDASIH or AGDASIH;
(iii) IL-2Rβγ binding interface mutations: D84N or D84N+E95Q;
and optionally, (iv) a mutation T3A. More preferably, the IL-2 mutant protein disclosed herein comprises, relative to the wild-type IL-2: (i) **a combination of mutations** K35**E**+T37**E**+R38**E**+F42**A**; (ii) a B'C' loop region sequence AGDASIH; (iii) IL-2Rβγ binding interface mutations: D84N+E95Q.

In still other preferred embodiments, the IL-2 mutant protein disclosed herein, relative to the wild-type IL-2 protein, maintains binding to the IL-2Rα receptor and comprises:
(i) a B'C' loop region sequence AQSKNFH; SGDASIH or AGDASIH;
(ii) an IL-2Rβγ binding interface mutation, selected from the following: D20N, N88R and N88D;
and optionally (iii) a mutation T3A. Preferably, the IL-2 mutant protein does not have an IL-2Rα binding interface mutation relative to the wild-type IL-2.

In other preferred embodiments, the IL-2 mutant protein disclosed herein, relative to the wild-type IL-2 protein, maintains binding to the IL-2Rα receptor and comprises:
(i) a B'C' loop region sequence SGDASIH or AGDASIH;
(ii) an IL-2Rβγ binding interface mutation: D20N, or N88R, or N88D;
and optionally (iii) a mutation T3A. Preferably, the IL-2 mutant protein does not have an IL-2Rα binding interface mutation relative to the wild-type IL-2.

In other preferred embodiments, the IL-2 mutant protein disclosed herein, relative to the wild-type IL-2 protein, maintains binding to the IL-2Rα receptor and comprises:
(i) a B'C' loop region sequence AGDASIH;
(ii) an IL-2Rβγ binding interface mutation: D20N, or N88R, or N88D;
and optionally (iii) a mutation T3A. Preferably, the IL-2 mutant protein does not have an IL-2Rα binding interface mutation relative to the wild-type IL-2.

In still other preferred embodiments, the IL-2 mutant protein disclosed herein, relative to the wild-type IL-2 (e.g., SEQ ID NO: 1), maintains binding to the IL-2Rα receptor and comprises:
(i) a B'C' loop region sequence AGDASIH;
(ii) an IL-2Rβγ binding interface mutation: N88R;
and (iii) a mutation T3A. Preferably, the IL-2 mutant protein does not have an IL-2Rα binding interface mutation relative to the wild-type IL-2. In one embodiment, the IL-2 mutant protein disclosed herein has a mature region having at least 85% or 90% identity in the amino acid sequence to that of the wild-type IL-2 protein set forth in one of SEQ ID NOs: 1-3.

In some preferred embodiments, the IL-2 mutant protein disclosed herein comprises an amino acid sequence having at least 90%, 92%, 93%, 94%, 95%, 96%, 97%, 98% or 99% identity to an amino acid sequence selected from one of SEQ ID NOs: 37-638 (particularly SEQ ID NOs: 469-553 or 554-638, preferably SEQ ID NOs: 148 and 197, more preferably SEQ ID NOs: 489, 513 and 516). In some embodiments, the mutant protein comprises or consists of an amino acid sequence selected from SEQ ID NOs: 37-638. In some preferred embodiments, the mutant protein comprises or consists of an amino acid sequence selected from SEQ ID NOs: 469-553 or 554-638, preferably SEQ ID NOs: 148 and 197, and more preferably SEQ ID NOs: 489, 513 and 516.

### Other mutations

In addition to the "IL-2Rβγ binding interface mutations", "B'C' loop region mutations" and "IL-2Rα binding interface mutations" described above, the IL-2 mutant protein disclosed herein can also have one or more mutations in other regions or positions, as long as it retains one or more beneficial properties described above. For example, the IL-2 mutant protein disclosed herein may also comprise a substitution at position 125, such as C125S, C125A, C125T, or C125V, so as to provide additional advantages, such as improved expression or homogeneity or stability (see, e.g., U.S. Patent No. 4,518,584). As another example, the IL-2 mutant protein disclosed herein can also comprise a substitution at position 3, e.g., T3A, to remove the O-glycosylation at the N-terminus of IL2. Those skilled in the art know how to determine additional mutations that can be incorporated into the IL-2 mutant protein disclosed herein.

The sequence difference between the IL-2 mutant protein and the wild-type protein can be expressed in terms of sequence identity or in terms of the number of different amino acids between the two. In one embodiment, the IL-2 mutant protein has at least 85%, 86%, 87%, 88%, or 89% identity, preferably more than 90% (preferably 95%) but preferably no more than 97%, and more preferably no more than 96% identity to the wild-type protein. In another embodiment, in addition to the three types of mutations described above in the present invention, the IL-2 mutant protein may also have no more than 15, e.g., 1-10, or 1-5, e.g., 0, 1, 2, 3 or 4, mutations relative to the wild-type protein. In one embodiment, the additional mutations may be conservative substitutions.

### 2. Fusion Protein and IL-2-Fc Dimer Protein

In one aspect, the present invention also provides a fusion protein comprising the IL-2 mutant protein disclosed herein. In one preferred embodiment, the IL-2 mutant protein disclosed herein is fused to another polypeptide, such as albumin, and preferably an antibody Fc fragment, which can provide improved pharmacokinetic properties.

In one embodiment, the present invention provides an IL-2 mutant protein fusion protein, which comprises the IL-2 mutant protein disclosed herein fused to an antibody Fc fragment.

The Fc fragment for use in the present invention may comprise a mutation that reduces or eliminates effector functions. In one preferred embodiment, the Fc fragment has reduced Fc-mediated effector functions, such as reduced or eliminated ADCC or ADCP or CDC effector functions. For example, in some particular embodiments, the Fc fragment for use in the present invention has mutations L234A/L235A or L234A/L235E/G237A that reduce binding to the Fcy receptor.

In yet another preferred embodiment, the Fc fragment may have a mutation that leads to an increased serum half-life, e.g., a mutation that improves binding of the Fc fragment to FcRn.

In some embodiments, the Fc fragment fused to the IL-2 mutant protein is a human IgG Fc, e.g., human IgG1 Fc, human IgG2 Fc or human IgG4 Fc. In one embodiment, the Fc fragment comprises the amino acid sequence of SEQ ID NO: 12, or has at least 90% identity, e.g., 95%, 96%, 97%, 99% or higher identity, thereto.

In some embodiments, the IL-2 mutant protein is fused to the Fc via a linker. In some embodiments, the linker may be selected to enhance the activation of the Fc fusion protein on CD25⁻ T cells. In one embodiment, the linker is GSGS, preferably (G4S)₂.

In a further aspect, the present invention also provides a dimeric molecule comprising the IL-2 mutant protein disclosed herein fused to an Fc fragment. With such a dimeric molecule, the molecular weight can be increased to 60-80 kDa, and the renal clearance is greatly reduced. In addition, the half-life of the IL2-Fc fusion protein can be further extended by FeRn-mediated *in vivo* recycling. Preferably, the dimeric molecule, compared to a corresponding dimeric molecule comprising a wild-type IL-2-Fc fusion protein, has one or more of the following properties:
- an increased expression yield and/or purity, when expressed in mammalian cells (e.g., CHO or HEK293 cells);
- reducing or avoiding over-activation of lymphocytes and/or release of inflammatory factors caused by IL-2;
- more excellent pharmacokinetic properties, such as a delayed *in vivo* half-life; and
- low toxicity when administered *in vivo,* e.g., low cardiovascular toxicity (e.g., a low vascular leak side effect).

Preferably, the dimeric molecule of the IL-2 mutant protein disclosed herein shows a good anti-tumor effect and tolerance when administered in animals. The anti-tumor effect can be determined by measuring the tumor inhibition rate in a tumor-bearing animal experiment as described in the examples. The tolerance can be determined by measuring the body weight and changes in the body weight of an animal model after administration as described in the examples.

In some embodiments, the present invention provides an IL-2-Fc dimer protein, which is a homodimer, wherein a first monomer and a second monomer comprise, from N-terminus to C-terminus, i) an IL-2 mutant protein; ii) a linker; and iii) an Fc fragment.

In other embodiments, the present invention provides an IL-2-Fc dimer protein, which is a heterodimer and comprises:
a) a first monomer, comprising, from N-terminus to C-terminus, i) an IL-2 mutant protein; ii) a linker; and iii) a first Fc fragment; and
b) a second monomer, comprising a second Fc fragment.

In some embodiments, the first Fc fragment and the second Fc fragment comprise a first heterodimerization mutation and a second heterodimerization mutation that promote the formation of the heterodimer from the first monomer and the second monomer, respectively. In some preferred embodiments, the first and second heterodimerization mutations comprise a combination of Knob:Hole mutations, such as the combination of mutations T366W/S354C:Y349C/T366S/L368A/Y407V.

In some preferred embodiments, the first heterodimerization mutation in the first Fc fragment includes a Knob mutation and the second heterodimerization mutation in the second Fc fragment includes a Hole mutation; alternatively, the first heterodimerization mutation in the first Fc fragment includes a Hole mutation and the second heterodimerization mutation in the second Fc fragment includes a Knob mutation.

As understood by those skilled in the art, Fc fragments suitable for the fusion protein and the dimeric molecule disclosed herein may be any antibody Fc fragment. In one embodiment, the Fc fragment of the present invention is effector function-silenced.

In one embodiment, the Fc fragment is modified in one or more properties selected from: the effector function of the Fc region and the complement activation function of the Fc region. In one embodiment, the effector function or complement activation function has been reduced or eliminated relative to a wild-type Fc region of the same isotype. In one embodiment, the effector function is reduced or eliminated by using a method selected from: reducing glycosylation of the Fc region, using an Fc isotype that naturally has a reduced or an eliminated effector function, and Fc region modification.

In one embodiment, the effector function is reduced or eliminated by reducing glycosylation of the Fc region. In one embodiment, the glycosylation of the Fc region is reduced by using a method selected from: producing the fusion protein or the dimeric molecule disclosed herein in an environment that does not allow wild-type glycosylation; removing carbohydrate groups already present in the Fc region; and modifying the Fc region so that wild-type glycosylation does not occur. In one embodiment, the glycosylation of the Fc region is reduced by using the method of modifying the Fc region so that wild-type glycosylation does not occur, e.g., including a mutation at position 297 in the Fc region so that the wild-type asparagine residue at that position is replaced with another amino acid that interferes with glycosylation at that position, e.g., an N297A mutation.

In one embodiment, the effector function is reduced or eliminated by at least one Fc region modification. In one embodiment, the at least one Fc region modification is selected from: an Fc region point mutation that impairs binding to one or more Fc receptors, selected from the following positions: 238, 239, 248, 249, 252, 254, 265, 268, 269, 270, 272, 278, 289, 292, 293, 294, 295, 296, 297, 298, 301, 303, 322, 324, 327, 329, 333, 335, 338, 340, 373, 376, 382, 388, 389, 414, 416, 419, 434, 435, 437, 438 and 439; an Fc region point mutation that impairs binding to C1q, selected from the following positions: 270, 322, 329 and 321; and an Fc region point mutation at position 132 of a CH1 domain. In one embodiment, the modification is an Fc region point mutation that impairs binding to C1q selected from the following positions: 270, 322, 329 and 321. In another embodiment, the modification is the elimination of some Fc regions.

As understood by those skilled in the art, to facilitate the formation of the heterodimer of the present invention, the Fc fragment of the dimeric molecule disclosed herein may comprise mutations that favor the dimerization of the first monomer and the second monomer. Preferably, corresponding Knob and Hole mutations are introduced into the first and second monomers based on the Knob-in-Hole technique.

Thus, in one embodiment, the dimeric molecule disclosed herein comprises:
i) a homodimeric Fc-region of the human IgG1 subclass, optionally with mutations P329G, L234A and L235A, or
ii) a homodimeric Fc-region of the human IgG4 subclass, optionally with mutations P329G, S228P and L235E, or
iii) a heterodimeric Fc-region, wherein
   a) one Fc-region polypeptide comprises a mutation T366W, and the other Fc-region polypeptide comprises mutations T366S, L368A and Y407V, or
   b) one Fc-region polypeptide comprises mutations T366W and Y349C, and the other Fc-region polypeptide comprises mutations T366S, L368A, Y407V and S354C, or
   c) one Fc-region polypeptide comprises mutations T366W and S354C, and the other Fc-region polypeptide comprises mutations T366S, L368A, Y407V and Y349C,
   or
iv) a heterodimeric Fc-region of the human IgG4 subclass, wherein both Fc-region polypeptides comprise mutations P329G, L234A and L235A, and
   a) one Fc-region polypeptide comprises a mutation T366W, and the other Fc-region polypeptide comprises mutations T366S, L368A and Y407V, or
   b) one Fc-region polypeptide comprises mutations T366W and Y349C, and the other Fc-region polypeptide comprises mutations T366S, L368A, Y407V and S354C, or
   c) one Fc-region polypeptide comprises mutations T366W and S354C, and the other Fc-region polypeptide comprises mutations T366S, L368A, Y407V and Y349C,
   or
v) a heterodimeric Fc-region of the human IgG4 subclass, wherein both Fc-region polypeptides comprise mutations P329G, S228P and L235E, and
   a) one Fc-region polypeptide comprises a mutation T366W, and the other Fc-region polypeptide comprises mutations T366S, L368A and Y407V, or
   b) one Fc-region polypeptide comprises mutations T366W and Y349C, and the other Fc-region polypeptide comprises mutations T366S, L368A, Y407V and S354C, or
   c) one Fc-region polypeptide comprises mutations T366W and S354C, and the other Fc-region polypeptide comprises mutations T366S, L368A, Y407V and Y349C.

In some embodiments, the Fc region further comprises additional mutations that favor the purification of the heterodimer. For example, an H435R mutation (Eric J. Smith, Scientific Reports | 5:17943 | DOI: 10.103 8/srep 17943) can be introduced into one of the Fc regions of the heterodimer (e.g., an Fc region with Hole mutations) to facilitate the purification of the heterodimer using protein A. In other embodiments, for heterodimeric monomers comprising a hinge region, mutations such as C220S may also be introduced into the hinge region to facilitate the formation of the heterodimer.

As will be appreciated by those skilled in the art, linkers suitable for linking the IL-2 mutant protein and the Fc fragment in the fusion protein and dimeric molecule disclosed herein may be any linker known in the art. In some embodiments, the linker may comprise an IgG1 hinge, or may comprise a linker sequence selected from the following: (GS)n, (GSGGS)n, (GGGGS)n and (GGGS)n, wherein n is an integer of at least 1. Preferably, the linker comprises (G4S)2, i.e., GGGGSGGGGS.

Thus, in one preferred embodiment, each monomer in the dimer of the present invention comprises an IL-2 mutant protein selected from SEQ ID NOs: 37-638 (particularly SEQ ID NOs: 469-553 or 554-638, preferably SEQ ID NOs: 489, 513 and 516) linked at the C-terminus to the amino acid sequence of SEQ ID NO: 12 by a linker (G4S)2. In some preferred embodiments, the first monomer of the dimeric molecule comprises an IL-2 mutant protein selected from SEQ ID NOs: 37-638 (particularly SEQ ID NOs: 469-553 or 554-638, preferably SEQ ID NOs: 489, 513 and 516) linked at the C-terminus to the amino acid sequence of SEQ ID NO: 9 by a linker (G4S)2; and the second monomer comprises an amino acid sequence of SEQ ID NO: 10.

### 3. Immunoconjugate

The present invention also provides an immunoconjugate comprising the IL2 mutant protein disclosed herein and an antigen-binding molecule. Preferably, the antigen-binding molecule is an immunoglobulin molecule, particularly an IgG molecule, an antibody, or an antibody fragment, and more particularly an Fab molecule or an scFv molecule. In some embodiments, the antigen-binding molecule specifically binds to an antigen present on a tumor cell or in tumor environment, such as an antigen selected from: fibroblast activation protein (FAP), A1 domain of tenascin-C (TNC A1), A2 domain of tenascin-C (TNC A2), extra domain B (EDB) of fibronectin, carcinoembryonic antigen (CEA), and melanoma-associated chondroitin sulfate proteoglycan (MCSP). Thus, the immunoconjugate disclosed herein can target the tumor cell or the tumor environment after being administrated to a subject, thereby providing further therapeutic benefits, such as the feasibility of treatment at lower doses and the consequent low side effects, and enhanced anti-tumor effects.

In the immunoconjugate disclosed herein, the IL-2 mutant protein disclosed herein can be linked, either directly or through a linker, to another molecule or antigen-binding molecule, and in some embodiments, a proteolytic cleavage site is provided therebetween.

### 4. Polynucleotide, Vector, and Host

The present invention provides a nucleic acid encoding any of the IL-2 mutant proteins, fusion proteins, dimeric molecules or conjugates above. The polynucleotide sequence encoding the mutant protein disclosed herein can be generated by *de novo* solid phase DNA synthesis or by PCR mutagenesis of an existing sequence encoding the wild-type IL-2 using methods well known in the art. In addition, the polynucleotide and the nucleic acid disclosed herein may comprise a segment encoding a secretion signal peptide and are operably linked to a segment encoding the mutant protein disclosed herein so that secretory expression of the mutant protein disclosed herein can be directed.

The present invention also provides a vector comprising the nucleic acid disclosed herein. In one embodiment, the vector is an expression vector, such as a eukaryotic expression vector. The vector includes, but is not limited to, a virus, a plasmid, a cosmid, a λ phage, or a yeast artificial chromosome (YAC). In a preferred embodiment, the expression vector disclosed herein is a pYDO_017 expression vector.

In addition, the present invention also provides a host cell comprising the nucleic acid or the vector. Host cells suitable for replicating and supporting the expression of the IL-2 mutant protein, the fusion, the dimer or the immunoconjugate are well known in the art. Such cells can be transfected or transduced with a particular expression vector, and a large number of cells comprising vectors can be cultivated for inoculation in large-scale fermenters, so as to obtain sufficient IL-2 mutants, fusions, dimers or immunoconjugates for clinical application. In one embodiment, the host cell is eukaryotic. In another embodiment, the host cell is selected from a yeast cell and a mammalian cell (e.g., a CHO cell or a 293 cell). Examples of available mammalian host cell lines include SV40 transformed monkey kidney CV1 lines (COS-7), human embryonic kidney lines (293 or 293T cells, as described, for example, in Graham et al., JGenVirol 36,59 (1977)), baby hamster kidney cells (BHK), mouse Sertoli cells (TM4 cells, as described, for example, in Mather, BiolReprod 23,243-251 (1980)), monkey kidney cells (CV1), African green monkey kidney cells (VERO-76), human cervical cancer cells (HELA), canine kidney cells (MDCK), buffalo rat liver cells (BRL3A), human lung cells (W138), human liver cells (HepG2), mouse mammary tumor cells (MMT060562), TRI cells (as described, for example, in Mather et al., AnnalsN.Y.AcadSci 383,44-68 (1982)), MRC5 cells, and FS4 cells. Other available mammalian host cell lines include Chinese hamster ovary (CHO) cells, including dhfr-CHO cells (Urlaub et al., Proc. Natl. Acad. Sci. USA 77,4216 (1980)), and myeloma cell lines such as YO, NS0, P3X63, and Sp2/0. In one embodiment, the host cell is a eukaryotic cell, preferably a mammalian cell such as a Chinese hamster ovary (CHO) cell, a human embryonic kidney (HEK) cell, or a lymphocyte (e.g., Y0, NS0, and Sp20 cells).

### 5. Preparation Method

In a further aspect, the present invention provides a method for preparing the IL-2 mutant protein, the fusion, the dimer or the conjugate disclosed herein, wherein the method comprises culturing a host cell comprising a nucleic acid encoding the protein, the fusion, the dimer or the conjugate under conditions suitable for expression of the IL-2 mutant protein, the fusion, the dimer or the conjugate, as provided above, and optionally isolating the protein, the fusion, the dimer or the conjugate from the host cell (or the host cell culture medium).

### 6. Assay

The IL-2 mutant protein provided herein can be identified, screened, or characterized for its physical/chemical properties and/or biological activities through a variety of assays known in the art.

In one aspect, the IL-2 mutant protein disclosed herein can be tested for its binding activity to an IL-2 receptor. For example, the binding to a human IL-2Rα or β protein, IL-2Rβγ or IE-2Kαβγ can be determined by methods known in the art, such as ELISA and Western blotting, or by the exemplary methods disclosed in the examples herein. For example, the flow cytometry can be used, wherein cells such as yeast display cells that are transfected to express the mutant protein on the cell surface react with a labeled (e.g., biotin-labeled) IL-2Rα or β protein, IL-2Rβγ or 1E-2Kαβγ complex. Alternatively, the binding of the mutant protein to the receptor, including the binding kinetics (e.g., the K_{D} value), can be determined by a ForteBio assay in IL-2-Fc fusion or dimeric molecule form.

In a further aspect, the ability of the IL-2 mutant protein to bind to the IL-2 receptor can be measured indirectly by measuring the signaling and/or immune activation at the downstream of receptor binding.

Thus, in some embodiments, an assay for identifying the IL-2 mutant protein having a biological activity is provided. The biological activities may include, for example, the ability to induce proliferation of T cells and/or NK cells and/or Treg cells with IL-2 receptors, the ability to induce IL-2 signaling in T cells and/or NK cells and/or Treg cells with IL-2 receptors, reduced ability to induce apoptosis in T cells, the ability to induce tumor regression and/or to improve survival, and reduced *in vivo* toxicity properties, such as reduced vascular permeability. The present invention also provides an IL-2 mutant protein having such biological activities *in vivo* and/or *in vitro,* an Fc fusion thereof and a dimeric molecule comprising same.

Various methods known in the art can be used for determining the biological activities of the IL-2. For example, an assay suitable for testing the ability of the IL-2 mutant protein disclosed herein (e.g., in dimeric molecule form) to stimulate IFN-γ production by NK cells may comprise the steps of: incubating the cultured NK cells with the IL-2 mutant protein disclosed herein, and measuring the IFN-γ concentration in the culture medium by ELISA. IL-2 signaling induces several signaling pathways and involves JAK (Janus kinase) and STAT (signal transducers and activators of transcription) signaling molecules.

The interaction of the IL-2 with the β and γ subunits of the receptor results in phosphorylation of the receptor and JAK1 and JAK3 (which bind to the β and γ subunits, respectively). STATS then binds to the phosphorylated receptor and is phosphorylated on a very important tyrosine residue. This results in dissociation of STATS from the receptor, dimerization of STATS, and translocation of STATS dimers to the nucleus where they facilitate the transcription of target genes. Thus, the ability of the mutant IL-2 polypeptide to induce signaling via the IL-2 receptor can be assessed, for example, by measuring the phosphorylation of STATS. Details of this method have been disclosed in the examples. For example, PBMCs can be treated with the mutant IL-2 polypeptide, the fusion, the dimer or the immunoconjugate disclosed herein, and the level of phosphorylated STATS is determined by flow cytometry.

Furthermore, the effect of the mutant IL-2 on tumor growth and survival can be assessed in a variety of animal tumor models known in the art. For example, heterografts of cancer cell lines can be implanted into immunodeficient mice and treated with the mutant IL-2 polypeptide, the fusion, the dimer or the immunoconjugate disclosed herein. The *in vivo* anti-tumor effects of the mutant IL-2 polypeptide, the fusion, the dimeric molecule and the immunoconjugate disclosed herein can be determined based on tumor growth inhibition (e.g., calculated relative to an isotype control antibody). In addition, the *in vivo* toxicity of the mutant IL-2 polypeptide, the fusion, the dimeric molecule and the immunoconjugate disclosed herein can be determined based on changes in the body weight of animals (e.g., changes in the absolute body weight or percent changes in the body weight relative to the body weight before administration). The *in vivo* toxicity can also be determined based on mortality, life-time observations (visible symptoms of adverse effects, e.g., behavior, body weight, and body temperature), and clinical and anatomical pathology (e.g., measurement of blood chemistry values and/or histopathological analysis).

In a further aspect, the druggability (e.g., expression yield and product purity) of the mutant protein disclosed herein can be characterized by using methods known in the art. For the determination of the expression yield, when the mutant protein is secreted from the cultured cells and expressed in the culture supernatant, the cell culture fluid collected by centrifugation can be assayed for the protein content. Alternatively, the assay may be performed after one-step purification of the collected cell culture fluid, for example, after one-step affinity chromatography purification. For the determination of the purity of the product, the purity can be determined after one-step affinity chromatography purification of the collected culture supernatant of the production cells to determine the purification performance of the mutant protein. Preferably, the mutant protein disclosed herein, after being purified by this one-step affinity chromatography, has significantly higher purity than the wild-type protein, indicating that the mutant protein disclosed herein has a better purification performance. The purity determination method can be any conventional method known in the art, including, but not limited to, the SEC-HPLC method.

In a further aspect, the pharmacokinetic properties, e.g., half-life, of the mutant proteins, fusions, and dimeric molecules disclosed herein can be characterized by using methods known in the art.

### 7. Method for Engineering IL-2 Proteins

In one aspect, the present invention provides a method for obtaining an IL-2 mutant protein with improved properties and the IL-2 mutant protein obtained by using this method.

In one embodiment, the method disclosed herein comprises the following steps:
(1) shortening a sequence of a B'C' loop region of IL-2 by mutation, and optionally introducing one or more mutations into a binding interface of IL-2 to IL-2Rβγ and/or introducing one or more mutations into a binding interface of IL-2 to IL-2Rα; and
(2) expressing the IL-2 mutant protein in a mammalian cell (e.g., an HEK293 or CHO cell), for example, in the form of an Fc fusion (e.g., an FcLALA fusion).

In yet another embodiment, the method disclosed herein comprises the following steps:
(1) introducing one or more mutations into a binding interface of IL-2 to IL-2Rβγ, and optionally shortening a sequence of a B'C' loop region of IL-2 by mutation and/or introducing one or more mutations into a binding interface of IL-2 to IL-2Rα; preferably in one embodiment, not introducing a mutation into the binding interface of IL-2 to IL-2Rα; and
(2) expressing the IL-2 mutant protein in a mammalian cell (e.g., an HEK293 or CHO cell), for example, in the form of an Fc fusion (e.g., an FcLALA fusion).

In any of the above embodiments, preferably, the shortened loop region has less than 10, 9, 8, 7, 6 or 5 amino acids in length, and more preferably has 7 amino acids in length;
more preferably, the B'C' loop region mutation includes:
(a) a substitution of aa74 to aa83 in a B'C' loop region, for example, with a short B'C' loop sequence from four-helical short-chain cytokine IL family members, such as a B'C' loop sequence from IL-15, preferably, with a sequence GDASIH; or
(b) a truncation of aa74 to aa83 in the B'C' loop region, for example, by 1, 2, 3 or 4 amino acids at the C-terminus; preferably a truncation to form a sequence (Q/G)SKN(F/I)H, and more preferably a truncation to form a sequence selected from the following:

| B'C' loop sequence |
|---|
| QSKNFH |
| GSKNFH |
| QSANFH |
| QSANIH |

preferably, the IL-2Rβγ binding interface mutation includes the IL-2Rβγ binding interface mutation described above;
preferably, the IL-2Rα binding interface mutation includes the IL-2Rα binding interface mutation described above;
preferably, the mutant protein has the following improved properties: (i) improved expression yield and/or protein purity (e.g., purity after one-step affinity chromatography as determined by SEC-HPLC); and optionally (ii) weakened binding to IL2RP and/or (iii) altered binding to IL-2Rα.

In one embodiment, the method of the present invention further comprises the following steps: after steps (1) and (2), identifying the following properties of the mutant protein: (i) the expression yield and/or protein purity after purification (e.g., purity after one-step affinity chromatography as determined by SEC-HPLC); and optionally (ii) the weakened binding to IL2RP, and/or (iii) the altered binding to IL-2Rα. In yet another embodiment, the method of the present invention further comprises the following steps: after steps (1) and (2), identifying the following properties of the mutant protein: (i) the expression yield and/or protein purity after purification (e.g., purity after one-step affinity chromatography as determined by SEC-HPLC); and (ii) the weakened binding to IL2RP, and optionally the maintained binding to IL-2Rα.

In one embodiment, the method comprises: identifying the IL-2 mutation that improves druggability (e.g., expression yield and/or product stability and/or homogeneity, such as one-step Fc affinity chromatography purity) before the combination of mutations in step (1) is performed. In one preferred embodiment, the druggability of the mutant protein is improved by substituting the B'C' loop with a shortened B'C' loop or truncating the B'C' loop to form a shortened B'C' loop.

In one embodiment, the method comprises: identifying the IL-2Rβγ binding interface mutation and/or IL-2Rα binding interface mutation that impart weakened binding to IL2RP and/or an altered ability to bind to IL-2Rα relative to the wild-type IL-2 before the combination of mutations in step (1) is performed.

As will be appreciated by those skilled in the art, these mutations can be combined with mutations imparting further improved druggability or other improved properties to obtain an IL-2 mutant protein with multiple improved properties.

In one embodiment, the combination of an (e.g., known) IL-2Rβγ binding interface mutation that results in weakened binding to IL-2Rβγ and a (e.g., known) B'C' loop region mutation that improves the druggability is introduced into the IL-2 protein, and the properties are identified. In one preferred embodiment, the identification includes weakened binding to IL-2Rβγ and improved druggability (e.g., improved expression and/or purity, and/or product stability and/or homogeneity), and optionally substantially unchanged or weakened or enhanced binding affinity for IL-2Rα, relative to the wild-type IL-2.

In some embodiments, the parental wild-type IL-2 protein used as a mutation template preferably has at least 85%, or at least 90% or 95% identity to SEQ ID NO: 1, and more preferably is an IL-2 protein derived from human.

### 8. Pharmaceutical Composition and Pharmaceutical Preparation

The present invention also comprises a composition (including a pharmaceutical composition or a pharmaceutical preparation) comprising the IL-2 mutant protein or the fusion, dimeric molecule or immunoconjugate thereof, and a composition comprising the polynucleotide encoding the IL-2 mutant protein or the fusion, dimeric molecule or immunoconjugate thereof. Such compositions can further optionally comprise suitable pharmaceutical adjuvants, such as a pharmaceutical carrier and a pharmaceutical excipient known in the art, including buffers.

### 9. Combination Product

In one aspect, the present invention further provides a combination product comprising the mutant protein or the fusion, dimeric molecule or immunoconjugate thereof disclosed herein, and one or more other therapeutic agents (e.g., a chemotherapeutic agent, other antibodies, a cytotoxic agent, a vaccine, and an anti-infective active agent). The combination product disclosed herein can be used in a therapeutic method disclosed herein.

In some embodiments, the present invention provides a combination product, wherein the aforementioned other therapeutic agents refer to, for example, a therapeutic agent, such as an antibody, which is effective to stimulate an immune response and thus further enhance, stimulate or upregulate the immune response in a subject.

In some embodiments, the combination product is used for preventing or treating cancer. In some embodiments, the cancer is, e.g., a gastrointestinal cancer, e.g., rectal cancer, colon cancer, or colorectal cancer. In some embodiments, the combination product is used for preventing or treating an infection, such as bacterial infection, viral infection, fungal infection, and protozoal infection.

### 10. Therapeutic Method and Use

As used herein, the terms "individual" and "subject" are used interchangeably and refer to a mammal. Mammals include, but are not limited to, domesticated animals (e.g., cows, sheep, cats, dogs, and horses), primates (e.g., humans and non-human primates such as monkeys), rabbits, and rodents (e.g., mice and rats). In particular, a subject is a human.

As used herein, the term "treating" refers to a clinical intervention intending to alter the natural progress of a disease in an individual being treated. Desired therapeutic effects include, but are not limited to, preventing the occurrence or recurrence of diseases, alleviating symptoms, reducing any direct or indirect pathological outcomes of diseases, preventing metastasis, delaying disease progression, improving or alleviating conditions, and alleviating or improving prognosis.

In one aspect, the present invention provides a method for stimulating the immune system of a subject, comprising administering to the subject an effective amount of a pharmaceutical composition comprising the IL-2 mutant protein, the fusion, the dimeric molecule or the immunoconjugate disclosed herein.

The weakened IL-2-Fc molecule of the present invention can effectively avoid excessive release of inflammatory factors caused by strong agonizing of lymphocytes, and has more stable and long-acting pharmacokinetic properties. Thus, in one embodiment, a sufficiently high drug exposure can be achieved in the human body with a lower single dose to avoid drug-related toxicity resulting from high Cₘₐₓ.

Thus, in some embodiments, the present invention relates to a method for enhancing the immune response of the body of a subject, comprising administering to the subject an effective amount of any of the IL-2 mutant proteins or the fusions or immunoconjugates thereof described herein. In some embodiments, the IL-2 mutant protein or the fusion or immunoconjugate thereof disclosed herein is administered to a subject with a tumor to stimulate an anti-tumor immune response. In other embodiments, the antibodies or the antigen-binding fragments thereof disclosed herein are administered to a subject with an infection to stimulate an anti-infection immune response.

In another aspect, the present invention relates to a method for treating a disease, such as cancer, in a subject, wherein the method comprises administering to the subject an effective amount of any of the IL-2 mutant proteins or the fusions or immunoconjugates thereof described herein. The cancer may be at an early, intermediate or advanced stage, or may be a metastatic cancer. In some embodiments, the cancer may be, e.g., a gastrointestinal cancer, e.g., rectal cancer, colon cancer, or colorectal cancer.

In another aspect, the present invention relates to a method for treating an infectious disease, e.g., chronic infection, in a subject, wherein the method comprises administering to the subject an effective amount of any of the IL-2 mutant proteins or the fragments thereof, or an immunoconjugate, a multispecific antibody, or a pharmaceutical composition comprising the antibodies or the fragments described herein. In one embodiment, the infection is virus infection.

In another aspect, the present invention relates to a method for treating a disease associated with Treg regulation in a subject, wherein the method comprises administering to the subject an effective amount of any of the IL-2 mutant proteins or the fragments thereof, or an immunoconjugate, a multispecific antibody, or a pharmaceutical composition comprising the antibodies or the fragments described herein. In one embodiment, the disease is associated with IL-2-mediated immune downregulation via Treg cells. In one embodiment, the disease is an autoimmune disease.

The mutant protein disclosed herein (or the pharmaceutical composition comprising the same, or the fusion, dimeric molecule or immunoconjugate thereof, and optionally an additional therapeutic agent) can be administered by any suitable method, including parenteral administration, intrapulmonary administration, intranasal administration, and, if required by locoregional treatment, intralesional administration. Parenteral infusion includes intramuscular, intravenous, intra-arterial, intraperitoneal or subcutaneous administration. The administration is carried out by any suitable means, such as injection, e.g., intravenous or subcutaneous injection, to some extent depending on whether the treatment is short-term or long-term. Various administration schedules are encompassed herein, including, but not limited to, single administration or multiple administrations at multiple time points, bolus injection, and pulse infusion.

In order to prevent or treat a disease, the appropriate dosage of the mutant protein disclosed herein (used alone or in combination with one or more additional therapeutic agents) will depend on the type of the disease to be treated, the type of the antibody, severity and progression of the disease, the purpose for which the antibody is administered (prevention or treatment), previous treatments, clinical history of a patient, responses to the antibody, and the discretion of an attending physician. The antibody is suitably administered to a patient through a single treatment or through a series of treatments.

In a further aspect, the present invention also provides use of the IL-2 mutant protein, composition, immunoconjugate, fusion and dimeric molecule disclosed herein in preparation of a drug for use in the aforementioned method (e.g., for treatment).

The following examples are described to assist in understanding the present invention. The examples are not intended to be and should not be interpreted in any way as limiting the protection scope of the present invention.

Examples

### Example 1: Design and Construction of IL-2Rα Binding Interface Mutant of Interleukin-2 Design and construction of an interleukin-2 mutant library

### Design of a mutant library

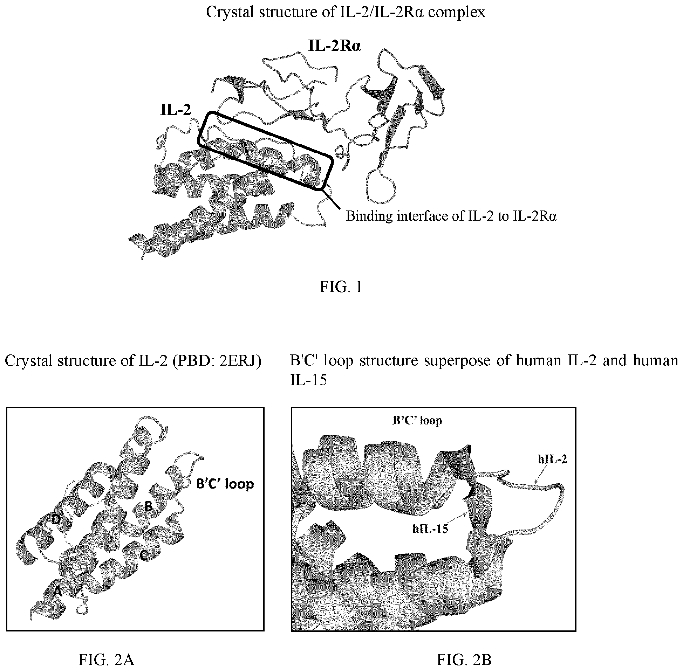

According to the crystal structure (PDB: 1Z92) (as shown in FIG. 1) of the complex of interleukin-2 (referred to as IL-2) and its alpha receptor CD25 (referred to as IL-2Rα), the listed IL-2 residues at interaction sites were mutated as per Table 1. The wild-type IL-2 (UniProt: P60568, aa21-153, C125S, referred to as IL-2^{WT}) was used as a template for mutant library construction. According to the 50% original amino acids contained in each site, the remaining 50% was evenly shared by the "mutant amino acids" in Table 1. A yeast-based IL-2^{mutant} display library was designed and produced for the binding site of IL-2 to IL-2Rα and designated IBYDL029 (Innoventbio Yeast Display Library). The library was screened for IL-2 mutants that did not bind to IL-2Rα by flow cytometry FACS.

**Table 1. Mutation sites for the IBYDL029 library**

| Sites | Amino acid residues | Mutant amino acids | Diversity |
|---|---|---|---|
| 35 | Lys(K) | D,E | 3 |
| 37 | Thr(T) | D,E,R,K,F,Y,W | 8 |
| 38 | Arg(R) | D,E,F,Y,W,A,V | 8 |
| 41 | Thr(T) | K,R,M,F,Y,W,Q,E | 9 |
| 42 | Phe(F) | K,R,A,E,Q | 6 |
| 43 | Lys(K) | E,D,F,Y,W | 6 |
| 45 | Tyr(Y) | R,K | 3 |
| 61 | Glu(E) | R,K,W,Y,L | 6 |
| 62 | Glu(E) | R,K,W,Y,L | 6 |
| 68 | Glu(E) | R,K,W,Y | 5 |
| 72 | Leu(L) | R,K,F,Y,W | 6 |

The sequence of IL-2^{WT} was set forth in SEQ ID NO: 1 in the present application. A C125S mutation was introduced at position 125 of the sequence to avoid the formation of a disulfide-bridged IL-2 dimer.

According to existing literature, the IL-2 mutant IL-2^{3X} does not bind to IL-2Rα, and has unchanged binding to IL-2Rβ (Rodrigo Vazquez-Lombardi et al., Nature Communications, 8:15373, DOI: 10.1038/ncomms15373). IL-2^{3X} was displayed on the surface of yeasts and used as a control. The sequence of IL-2^{3X} was set forth in SEQ ID NO: 4. Similar to IL-2^{WT} the protein also comprised a C125S mutation.

### Identification of IL-2Rα binding interface mutants

### Expression and purification of IL-2 receptors

### Vector construction

An avi tag (GLNDIFEAQKIEWHE, the tag peptide can be biotinylated under BirA enzyme catalysis) and 6 histidine tags (HHHHHH) were linked to the C-terminus of the sequences of the IL-2 receptors IL-2Rα (UniProt: P01589, aa22-217) and IL-2RP (UniProt: P14784, aa27-240), which were constructed separately into pTT5 vectors (Addgene). The sequences of the receptors constructed were set forth in SEQ ID NOs: 5 and 6.

The IL-2 receptor βγ complex was an Fc heterodimer based on Knobs in holes. The sequence of IL-2RP was constructed into the N-terminus of Fc-Knob (SEQ ID NO: 7), and the sequence of IL-2Rγ was constructed into the N-terminus of Fc-Hole (SEQ ID NO: 8). They were constructed separately into pcDNA3.1 vectors, then co-transfected into Expi293 cells and expressed.

### Plasmid transfection

Expi293 cells (Invitrogen) were passaged according to a desired transfection volume. The cell density was adjusted to 1.5 × 10⁶ cells/mL the day before transfection. The cell density on the day of transfection was approximately 3 × 10⁶ cells/mL. 1/10 (v/v) of the final volume of Opti-MEM medium (Gibco, Catalog No. 31985-070) was taken as a transfection buffer, added with expression plasmids constructed as described above, mixed well, and filtered with a 0.22 µm filter for later use. An appropriate amount of polyethylenimine (PEI) (Polysciences, 23966) was added to the plasmids from the previous step (the mass ratio of plasmids to PEI was 1:3), mixed and incubated at room temperature for 10 min to give a DNA/PEI mixture. The DNA/PEI mixture was gently poured into HEK293 cells, mixed well, and cultured at 37 °C, 8% CO₂ for 24 h, followed by the addition of VPA (Sigma, Catalog No. P4543-100G) to reach a final concentration of 2 mM. Then 2% (v/v) Feed (1 g/L Phytone Peptone + 1 g/L Difco Select Phytone) was added and the resulting mixture was cultivated for another 6 days.

### Purification of IL-2Rα-His and IL-2Rβ-His proteins

Before purification, the collected cultures were centrifuged at 4500 rpm for 30 min, and the cells were discarded. The supernatant was filtered through a 0.22 µm filter. A nickel column (5 mL Histrap excel, GE, 17-3712-06) used for purification was soaked with 0.1 M NaOH for 2 h, and then washed with 5-10 column volumes of ultra-pure water to remove alkali liquor. The column was equilibrated with 5 column volumes of binding buffer (20 mM Tris pH 7.4, 300 mM NaCl) before purification. The cell supernatant was passed through the equilibrated column and then 10 column volumes of wash buffer (20 mM Tris 7.4, 300 mM NaCl, 10 mM imidazole) was loaded on the column to remove non-specific binding impurity proteins. The target protein was then eluted with 3-5 column volumes of eluent (20 mM Tris 7.4, 300 mM NaCl, 100 mM imidazole). The collected protein was buffer-exchanged into PBS (Gibco, 70011-044) by ultrafiltration concentration, and further separated and purified using superdex200 increase (GE, 10/300GL, 10245605). The elution peak of the monomer was collected, and the equilibration buffer and elution buffer for the column were PBS (Gibco, 70011-044). 100 µg of the purified protein sample was taken and the protein purity was determined using a gel filtration column SW3000 (TOSOH Catalog No. 18675).

### Purification of IL-2 receptor βγ protein

After culture, the mixture was centrifuged at 13000 rpm for 20 min, and the supernatant was collected and purified by a pre-packed column Hitrap Mabselect Sure (GE, 11-0034-95). The procedures were as follows: the packing column was equilibrated with 5 column volumes of equilibration buffer (0.2 M Tris, 1.5 M NaCl, pH 7.2) before purification; the collected supernatant was passed through the column, and then the column was washed with 10 column volumes of equilibration buffer to remove non-specific binding proteins; the packing was washed with 5 column volumes of elution buffer (1 M sodium citrate, pH 3.5), and the eluent was collected. The collected protein was buffer-exchanged into PBS (Gibco, 70011-044) by ultrafiltration concentration, and further separated and purified using superdex200 increase (GE, 10/300GL, 10245605). The elution peak of the monomer was collected, and the equilibration buffer and elution buffer for the column were PBS (Gibco, 70011-044). 100 µg of the purified protein sample was taken and the protein purity was determined using a gel filtration column SW3000 (TOSOH Catalog No. 18675).

### Biotin labeling of IL-2Rα, IL-2RP and IL-2Rβγ proteins

Biotin labeling was performed using an enzymatic method, of which the procedures were as follows: an appropriate amount of each of the solutions of the IL-2Rα, IL-2RP and IL-2Rβγ proteins expressed and purified above was added with 1/10 (m/m) mass of His-BirA protein (UniProt: P06709), followed by ATP (Sigma, Catalog No. A2383-10G) with a final concentration of 2 mM, MgCl₂ with a final concentration of 5 mM, and D-biotin (AVIDITY, Catalog No. K0717) with a final concentration of 0.5 mM; the mixtures were incubated at 30 °C for 1 h, and purified by Superdex200 increase (GE, 10/300GL, 10245605) to remove excess biotin and His-BirA; the purified samples were verified by Streptavidin (SA) sensor (PALL, 18-5019) from Fortebio to confirm the successful biotin labeling.

### Expression, purification and identification of IL-2^{mutant}-FC fusion protein

### Construction of expression plasmids

The yeast-based IL-2^{mutant} display library IBYDL029 was screened by flow cytometry FACS for an IL-2^{mutant} that did not bind to IL-2Rα. The IL-2^{mutant} sequence obtained from the library screening was linked to FcLALA via two GGGGS and constructed into a vector of pCDNA3.1 (Addgene) to express the IL-2^{mutant}-FC fusion protein.

In addition, as controls, IL-2^{WT} and IL-2^{3X} gene sequences were linked to FcLALA via two GGGGS and constructed into pCDNA3.1 to express the IL-2^{WT}-FC and IL-2^{3X}-FC fusion proteins. The Fc used in this example was the Fc of human IgG1 with the mutations L234A and L235A (referred to as FcLALA, SEQ ID NO: 12).

Expression and purification of fusion proteins of IL-2 and FC

A vector containing the gene encoding the fusion protein was transferred into HEK293 cells using a chemical transfection method. The cultured HEK293 cells were transiently transfected using chemical transfection reagent PEI according to a scheme provided by the manufacturer. First, the plasmid DNA and the transfection reagent were prepared in a superclean bench. 3 mL of Opti-MEM medium (Gibco, Catalog No. 31985-070) was added to a 50 mL centrifuge tube, followed by 30 µg of the corresponding plasmid DNA. The Opti-MEM medium containing the plasmid was filtered with a 0.22 µm filter, and then added with 90 µg of PEI (1 g/L), and the mixture was let stand for 20 min. The DNA/PEI mixture was gently poured into 27 mL of HEK293 cells, mixed well, and cultured at 37 °C with 8% CO₂ for 20 h, followed by the addition of VPA to reach a final concentration of 2 mM. Then 2% (v/v) of Feed was added, and the resulting mixture was cultured for another 6 days.

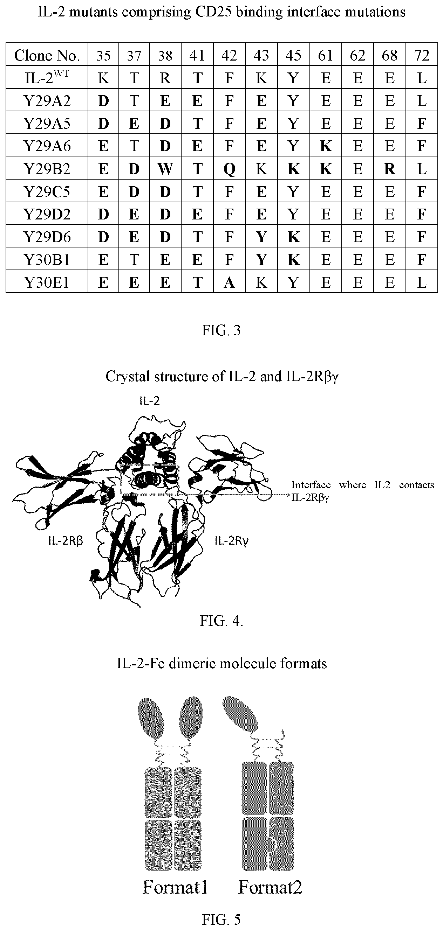

After culture, the mixture was centrifuged at 13000 rpm for 20 min, and the supernatant was collected, and purified by pre-packed column Hitrap Mabselect Sure. The procedures were as follows: the packing column was equilibrated with 5 column volumes of equilibration buffer (0.2 M Tris, 1.5 M NaCl, pH 7.2) before purification; the collected supernatant was passed through the column, and then the column was washed with 10 column volumes of equilibration buffer to remove non-specific binding proteins; the packing was washed with 5 column volumes of elution buffer (1 M sodium citrate, pH 3.5), and the eluent was collected; 80 µL of Tris (2 M Tris) was added per 1 mL of eluent, and the mixture was buffer-exchanged into PBS using an ultrafiltration concentration tube, and then the concentration was determined. 100 µg of purified protein was taken with its concentration adjusted to 1 mg/mL. The purity of IL-2-Fc dimer protein was determined by gel filtration chromatography. The results are shown in Table 2. The sequences of the IL-2 mutants of the present invention listed in Table 2 are shown in FIG. 3.

**Table 2. Expression yield and purity of IL-2-FC in 293 cells**

| IL-2 | Expression yield (mg/L) | Purity (SEC-HPLC) |
|---|---|---|
| IL-2^{WT} | 18 | 45% |
| Mutant IL-2^{3x} | 34 | 70% |
| Mutant Y29A2 | 37 | 79% |
| Mutant Y29A5 | 12 | 73% |
| Mutant Y29A6 | 1 | 37% |
| Mutant Y29B2 | 77 | 88% |
| Mutant Y29C5 | 10 | 60% |
| Mutant Y29D2 | 17 | 80% |
| Mutant Y29D6 | 23 | 60% |
| Mutant Y30B1 | 20 | 70% |
| Mutant Y30E1 | 60 | 78% |

### Determination of affinity of IL-2^{mutant}-FC fusion proteins for their receptors

The equilibrium dissociation constants (K_{D}) for binding of the IL-2^{mutant}-FC fusion proteins of the present invention to their receptors were measured by bio-layer interferometry (ForteBio).

The ForteBio affinity assay was conducted according to the method (Estep, P et al., High throughput solution Based measurement of antibody-antigen affinity and epitope binding. mAbs, 2013.5(2): p. 270-8) known in the art. Briefly, the affinity of candidate IL-2^{mutant}-FC for each of IL-2Rα and IL-2RP was measured as follows: a sensor was equilibrated offline in an assay buffer for 20 min and equilibrated online for 120 s to establish a baseline; the human biotin-labeled IL-2Rα or IL-2RP was loaded onto an SA sensor (PALL, 18-5019) for ForteBio affinity assay; the sensor loaded with the biotin-labeled IL-2Rα or IL-2RP was placed into a solution containing 100 nM IL-2^{mutant}-FC until to a plateau, and then transferred to an assay buffer for dissociation for at least 2 min to measure the association and dissociation rates. The kinetic analysis was performed using a 1:1 binding model.

In the assay described above, the affinity K_{D} values *of IL-2^{mutant}-FC* fusion proteins expressed by HEK293-F cells for their receptors are shown in Table 3. As a control, the affinity K_{D} values of the IL-2^{WT}-FC and IL-2^{3X}-FC fusion proteins measured using the same method are also shown in Table 3.

**Table 3. Affinity K_{D} values of IL-2^{mutant}-FC fusion proteins for their receptors**

| IL-2 | IL-2Rα avidity | IL-2RP avidity |
|---|---|---|
| IL-2^{WT} | 1.0E-08 | 4.1E-08 |
| IL-2^{3x} | N.B | 1.6E-08 |
| Y29A2 | Very weak | 4.8E-10 |
| Y29A5 | N.B | 3.4E-10 |
| Y29A6 | N.B | 1.8E-09 |
| Y29B2 | N.B | 3.4E-10 |
| Y29C5 | N.B | 4.2E-10 |
| Y29D2 | Very weak | 8.5E-11 |
| Y29D6 | N.B | 1.9E-10 |
| Y30B1 | N.B | 1.9E-10 |
| Y30E1 | N.B | 5.9E-09 |

| | | |
|---|---|---|
| N.B: none binding | | |

It can be seen from the affinity data, the binding of all of the above mutants obtained from the library IBYDL029 to IL-2Rα was blocked.

### Example 2: Construction, Screening and Identification of IL-2 Chimeric and Truncated Mutants

### Design of an IL-2 B'C' loop chimera and an IL-2 B'C' loop truncate

B'C' loop: The linker sequence (FIG. 2A) of the B helix and C helix of the IL-2, comprising 11 amino acids, namely A73-R83.

By comparing the crystal structure of an IL-2 monomer (PDB: 1M47) with that of a complex (PDB: 2ERJ), it was found that the B'C' loop was absent from the crystal structure of the IL-2 monomer since it was very active in a solution and could not form a relatively stable conformation.

By genetically engineering the B'C' loop, the stability of the B'C' loop, and thus that of the IL-2, was increased. We therefore observed the crystal structure of the human IL-15 (PDB: 2Z3Q) and found that its B'C' loop was relatively short and stable (FIG. 2B). Therefore, we designed an IL-2 chimeric molecule (L017) and 4 truncated molecules (L057-L060) (see Table 4).

**Table 4. Optimized sequences of IL-2 B'C' loop**

| Name | B'C' loop sequence |
|---|---|
| L 001(IL-2^{WT}) | AQSKNFHLRPR |
| L 017(IL-2^{hyb15BCL}) | SGDASIH |
| L 057(IL-2^{truncate1}) | AQSKNFH |
| L 058(IL-2^{truncate2}) | AGSKNFH |
| L 059(IL-2^{truncate3}) | AQSANFH |
| L 060(IL-2^{truncate4}) | AQSANIH |

### Construction of expression plasmids

The wild-type IL-2 (UniProt: P60568, aa21-153, C125S, IL-2^{WT} for short), the IL-2 mutant IL-2^{3X} (R38D, K43E, E61R), and the B'C' loop chimeras and truncates were linked to the Fc of human IgG1 (L234A, L235A, FcLALA for short, SEQ ID NO: 12) via a GSGS linker sequence and constructed into pTT5 vectors to express the following proteins:

| Protein | Structure | SEQ ID NOs |
|---|---|---|
| Y001 | IL-2^{WT}-GSGS-FcLALA | SEQ ID NO: 13 |
| Y002 | IL-2^{.3X}-GSGS-FcLALA | SEQ ID NO: 14 |
| Y017 | IL- 2^{hyb15BCL}-GSGS-FcLALA | SEQ ID NO: 15 |
| Y057 | IL-2^{truncate1}-GSGS⁻FCLALA | SEQ ID NO: 16 |
| Y058 | IL-2^{truneate2}-GSGS⁻FCLALA | SEQ ID NO: 17 |
| Y059 | IL-2^{truneate3}-GSGS-FCLALA | SEQ ID NO: 18 |
| Y060 | IL-2^{truncate4}-GSGS-FcLALA | SEQ ID NO: 19 |

The B'C' loop chimera (Y017) or truncate (Y057), combined with the mutant Y30E1 (K35E, T37E, R38E, F42A) obtained by library screening, was linked to FcLALA via two GGGGS and constructed into pCDNA3.1 vectors to express the following proteins. Among them, Y092 had a chimeric B'C' loop sequence AGDASIH; Y144 had an additional T3A amino acid substitution on the basis of Y092, which was intended to remove the O-glycosylation at the N-terminus of IL-2.

| Protein | Structure | SEQ ID NOs |
|---|---|---|
| Y089 | IL-2^{.Y30E1.truncate1}-2*(G4S)-FcLALA | SEQ ID NO: 20 |
| Y092 | IL-2^{.Y30E1.15BCL}-2^{∗}(G4S)-FcLALA | SEQ ID NO: 21 |
| Y144 | IL-2^{.Y30E1.15BCL.T3A}-2^{∗}(G4S)-FcLALA | SEQ ID NO: 22 |

Moreover, the IL-2^{WT} and the IL-2^{3X} were linked to FcLALA via two GGGGS and constructed into pCDNA3.1 vectors to express the following proteins:

| Protein | Structure | SEQ ID NOs |
|---|---|---|
| Y040 | IL-2^{.3X}-2^{∗}(G4S)-FcLALA | SEQ ID NO: 23 |
| Y045 | IL-2^{.WT}-2^{∗}(G4S)-FcLALA | SEQ ID NO: 24 |

The specific sequence information of the above protein molecules is shown in the sequence listing.

### Expression and purification of fusion proteins

The above protein molecules were expressed in 293 cells and CHO cells, respectively. The expression in HEK293 cells was performed by using the method for expressing IL-2-Fc fusion proteins in Example 1. The expression in CHO cells was performed as follows.

ExpiCHO cells (Invitrogen) were passaged according to a desired cell volume. The cell density was adjusted to 3.5 × 10⁶ cells/mL the day before transfection. The cell density (about 8-10 × 10⁶ cells/mL) was measured on the day of transfection. The viability reached 95% or more. The cell density was adjusted to 6 × 10⁶ cells/mL using ExpiCHO^{™} Expression Medium (Gibco, Catalog No. A29100-01). OptiPRO^{™} SFM (Gibco, Catalog No. 12309-019) at a final volume of 8% (v/v) was taken as a transfection buffer. A corresponding amount (0.8 µg/mL cell) of plasmid was added, and the mixture was well mixed and filtered through a 0.22 µm filter membrane to remove the bacteria. The reagent in ExpiFectamine^{™} CHO Transfection Kit (Gibco, Catalog No. A29130) was added in a cell ratio of 3.2 µL/mL. The complex formed with transfection reagent and the plasmid DNA was incubated at room temperature for 1-5 min, and slowly added to the cells. The cells were cultured at 37 °C with 8% CO₂ for 18 h, and then 0.6% (v/v) Enhancer and 30% (v/v) Feed were added. The cells were cultured for another 6 days.

After culture, the cell culture fluid was centrifuged at 13000 rpm for 20 min, and the supernatant was collected and purified by a pre-packed column Hitrap Mabselect Sure (GE, 11-0034-95). The procedures were as follows: the packing column was equilibrated with 5 column volumes of equilibration buffer (20 mM Tris, 150 mM NaCl, pH 7.2) before purification; the collected supernatant was passed through the column, and then the column was washed with 10 column volumes of equilibration buffer to remove non-specific binding proteins; the packing was washed with 5 column volumes of elution buffer (100 mM sodium citrate, pH 3.5), and the eluent was collected. 80 µL of Tris (2 M Tris) was added per 1 mL of eluent, and the mixture was buffer-exchanged into PBS (Gibco, Catalog No. 70011-044) using an ultrafiltration concentration tube (MILLIPORE, Catalog No. UFC901096), and then the concentration was determined. 100 µg of purified protein was taken with its concentration adjusted to 1 mg/mL. The protein purity was determined by gel filtration column SW3000 (TOSOH Catalog No. 18675).

The fusion proteins of the B'C' loop chimera (Y017) and truncate (Y057/058/059) had greatly improved expression yields and one-step affinity chromatography purity in HEK293 cells compared to the wild-type IL-2 fusion protein (Y001). The results are shown below in Table 5.

**Table 5. Expression yield and purity of IL-2 mutants in HEK293**

| Protein | Expression yield (mg/L) | Purity (SEC-HPLC) |
|---|---|---|
| Y001 | 16.35 | 44.74% |
| Y002 | 23.92 | 69.85% |
| Y017 | 54.47 | 93.45% |
| Y057 | 52.36 | 92.77% |
| Y058 | 49.86 | 99.09% |
| Y059 | 36.52 | 86.95% |
| Y060 | 21.20 | 66.33% |

The fusion proteins comprising the B'C' loop chimeras (Y092 and Y144), truncate (Y089) and further mutant Y30E1 also had greatly improved expression yields and one-step affinity chromatography purity in CHO cells compared to the wild-type IL-2 fusion protein (Y045). The results are shown below in Table 6.

**Table 6. Expression yield and purity of IL-2 mutants in CHO**

| Protein | Expression yield (mg/L) | Purity (SEC-HPLC) |
|---|---|---|
| Y040 | 20.28 | 40.75% |
| Y045 | 2.44 | 50.85% |
| Y089 | 249.6 | 99.11% |
| Y092 | 118.8 | 99.07% |
| Y144 | 114 | 97.78% |

According to the above data, it can be seen that: 1) the point mutation molecules such as Y30E1 obtained by yeast library screening could block the binding to IL-2Rα; 2) the B'C' loop chimeric molecule and truncate molecule improved the molecule expression yield and purity; 3) the combination molecules of Y30E1 and the B'C' loop mutant achieved the blocking of IL-2Rα and also the improved molecule expression yield and purity.

### In vitro functional assays of mutants

The activation effect of each mutant on CD25⁺ cells and CD25⁻ cells was tested by detecting the activation effect of each IL-2^{mutant}-FC on p-STAT5 signals of primary human CD8⁺ T cells. The specific steps are as follows:
1. Thawing PBMC cells:
   a) PBMC cells (Allcells, Catalog No. PB005F, 100M package) were taken out from liquid nitrogen, and then rapidly placed in a 37 °C water bath for thawing;
   b) the cells were added to 10 mL of preheated X-VIVO15 (Lonza, Catalog No. 04-418Q) culture medium containing 5% human AB serum (GemCell, Catalog No. 100-512) and 1 %o DNase (STRMCELL, Catalog No. 07900), centrifuged at 400 G and 25 °C for 10 min (the subsequent centrifugation was under the same condition) and washed once;
   c) 20 mL of culture medium was added to resuspend the cells, and the cells were cultured overnight in a 37 °C carbon dioxide incubator.
2. Purifying human CD8⁺ T cells:
   a) the cell suspension obtained in step 1 was pipetted, and after centrifugation, the supernatant was discarded;
   b) a mixture of 1 mL of Robosep buffer (STEMCELL, Catalog No. 20104), 100 µL of human AB serum, and 100 µL of negative screening antibody in human CD8⁺ T cell purification kit (Invitrogen, Catalog No. 11348D) was added to resuspend the cells;
   c) after mixing well, the cells were incubated for 20 min at 4 °C and shaken every 5 min;
   d) after incubation, 10 mL of Robosep buffer was added, and the cells were centrifuged and washed twice;
   e) meanwhile, 1 mL of magnetic microspheres (human CD8⁺ T cell purification kit) was taken, and 7 mL of Robosep buffer was added; the mixture was placed on a magnetic frame for 1 min to discard the supernatant, and the magnetic microspheres were pre-washed;
   f) the microspheres and the cells were resuspended with 1 mL of Robosep buffer, and after mixing well, the mixture was subjected to rotary incubation for 30 min at room temperature;
   g) after incubation, 6 mL of Robosep buffer was added and the mixture was placed on a magnetic frame for 1 min, followed by the collection of the supernatant;
   h) the collected liquid was placed on the magnetic frame for 1 min, and the supernatant was collected;
   i) centrifugation was performed to discard the supernatant, the cells were resuspended using a preheated T culture medium, and the cell density was adjusted to 1 × 10⁶/mL;
   j) 1/3 of the cells were taken to stimulate the expression of CD25 later, and the remaining cells were placed in a 37 °C carbon dioxide incubator for static culture overnight.
3. Stimulating CD8⁺ T cells to express CD25:
   a) 1/3 of the CD8⁺ T cells purified in step 2 were taken, into which magnetic microspheres of an anti-human CD3/CD28 antibody (GIBCO, Catalog No. 11131D) were added (the ratio of cells to microspheres was 3:1);
   b) the mixture was placed in a 37 °C carbon dioxide incubator for static culture for three days;
   c) 10 mL of culture medium was added to wash the cells twice;
   d) the culture medium was added to adjust the cell density to 1 × 10⁶/mL, and the cells were placed in a 37 °C carbon dioxide incubator for static culture for 2 days.
4. Detecting the purity and expression level of the cells:
   a) an anti-human CD8-PE antibody (Invitrogen, Catalog No. 12-0086-42), an anti-human CD25-PE antibody (eBioscience, Catalog No. 12-0259-42), and an isotype control antibody (BD, Catalog No. 556653) were adopted to detect CD8 and CD25 of the cells;
   b) the cells in step 2 were CD8⁺ CD25⁻ T cells, and the cells in step 3 were CD8⁺ CD25⁺ T cells.
5. Detecting the EC₅₀ value of each IL-2^{mutant}-FC in activating p-STAT5 signals in CD8⁺ CD25⁻ T cells:
   a) CD8⁺ CD25⁻ T cells were added to 96-well U-bottom plates (Costar, Catalog No. CLS3799-50EA) at 1 × 10⁵ cells per well;
   b) the IL-2^{mutant}-FC, the commercialized IL-2 (R&D, Catalog No. 202-IL-500), the IL-2^{WT}-FC, and the IL-2^{3X}-FC, each of 100 µL, were added and 4-fold diluted in gradient from a maximum concentration of 266.7 nM, for a total of 12 dilution gradients, and the cells were incubated in a 37 °C incubator for 20 min;
   c) 55.5 µL of 4.2% formaldehyde solution was added to immobilize the above cells at room temperature for 10 min;
   d) centrifugation was performed to discard the supernatant, and 200 µL of ice methanol (Fisher, Catalog No. A452-4) was added to resuspend the cells, which were then incubated in a 4 °C refrigerator for 30 min;
   e) centrifugation was performed to discard the supernatant, and the residue was washed 3 times with 200 µL of staining buffer (BD, Catalog No. 554657);
   f) 200 µL of permeabilization/fixation buffer (BD, Catalog No. 51-2091KZ) containing anti-p-STAT5-AlexFlour647 (BD, Catalog No. 562076, 1:200 dilution) was added, and the cells were incubated in the dark for 3 h at room temperature;
   g) the cells were washed with staining buffer for three times, resuspended with 100 µL of staining buffer, and detected using a flow cytometer;
   h) the EC₅₀ values to activate p-STAT5 signals were plotted using the IL-2 molecule concentration as the abscissa and the AlexFlour647 median fluorescence value as the ordinate, and the results are shown below in Table 7.
6. Detecting the EC₅₀ value of each IL-2^{mutant}-FC in activating p-STAT5 signals in CD8⁺ CD25⁺ T cells:
   a) CD8⁺ CD25⁺ T cells were added to 96-well U-bottom plates at 1 × 10⁵ cells per well;
   b) the EC₅₀ values to activate p-STAT5 signals were plotted through steps same as b-h in step 5, and the results are shown in Table 7.

**Table 7. EC₅₀ of IL-2 mutants activating p-STAT5 signals in CD25^{+/-} T cells and ratios thereof**

| | R&D IL2 | Y045 | Y040 | Y30E1 | Y089 | Y092 |
|---|---|---|---|---|---|---|
| CD25-pSTAT5 EC₅₀ | 0.4697 | 3.793 | 8.399 | 3.768 | 2.196 | 0.6644 |
| CD25+pSTAT5 EC₅₀ | 0.0009973 | 0.001919 | 3.717 | 2.186 | 0.3696 | 0.3729 |
| Ratio of CD25⁻ EC₅₀/CD25⁺ EC₅₀ | 470.9716 | 1976.5503 | 2.2596 | 1.7237 | 5.9416 | 1.7817 |

### Example 3: Design of IL-2 Weakened Mutants

### Design of IL-2 weakened molecules

This example was intended to further improve the pharmacokinetics (PK) of IL-2 mutants and to extend the half-lives of the molecules while reducing the toxicity of the molecules. Based on the analysis of the crystal structure of the IL-2 complex (FIG. 4) (PDB: 2ERJ), the amino acids at the interface where IL-2 contacted IL-2RP were selected and mutated to reduce the affinity between them.

Verification was performed based on IL-2Rα binding interface amino acid mutations, IL-2 B'C' loop optimization and IL-2Rβγ binding interface amino acid mutations, as well as two format forms. The design of molecules is shown in Table 8. Optionally, these molecules may further comprise a T3A mutation to remove the O-glycosylation at the N-terminus of IL2.

**Table 8. Design of IL-2 mutants**

| IL-2Rβγ binding interface | IL-2Rα binding interface | B'C' Loop engineering | Molecular sequence |
|---|---|---|---|
| L12, E15, H16, L19, D20, D84, S87, N88, V91, I92, E95, Q126 (single or multiple point mutations) | Y30E1(K35E, T37E, R38E, F42A) | WT (wild-type) | See the sequence listing and the description thereof for specific exemplary molecules. |
| | | Replacement with human IL-15 B'C' loop | |
| | | Truncation of human IL-2 B'C' loop | |
| | K35E, T37E, R38E | WT (wild-type) | |
| | | Replacement with human IL-15 B'C' loop | |
| | | Truncation of human IL-2 B'C' loop | |
| | F42A | Replacement with human IL-15 B'C' loop | |
| | | Truncation of human IL-2 B'C' loop | |
| | WT (wild-type) | Replacement with human IL-15 B'C' loop | |
| | | Truncation of human IL-2 B'C' loop | |

According to the above design, IL-2^{mutant} with the following sequence structure (from N-terminus to C-terminus) was specifically constructed for expression to produce format 1 molecules:
- an IL-2 mutant sequences with amino acid sequences of SEQ ID NOs: 37-638;
- a linker sequence GGGGSGGGGS; and
- an Fc sequence of SEQ ID NO: 12.

According to the above design, IL-2^{mutant}-Fc.Knob and Fc.Hole with the following sequence structure (from N-terminus to C-terminus) was specifically constructed for expression to produce format 2 molecules:
(1) IL-2^{mutant}-Fc.Knob
   - an IL-2 mutant sequences with amino acid sequences of SEQ ID NOs: 37-638;
   - a linker sequence GGGGSGGGGS; and
   - an Fc.Knob sequence of SEQ ID NO: 9; and
(2) Fc.Hole
   - an Fc.Hole sequence of SEQ ID NO: 10.

### Expression and purification of IL-2 weakened mutants and determination of their affinity for receptors

In the case of format 1, bivalent IL-2 mutant molecules were constructed by linking IL-2 mutants to the N-terminus of IgGlFcLALA via two G4S. Format 2 was a Knob-in-hole-based heterodimer of IgG1FcLALA, wherein monovalent IL-2 mutant molecules were constructed by linking IL-2 mutant molecules to the N-terminus of IgG1FcLALA-Knob via two G4S. All sequences were constructed into pcDNA3.1 vectors.

Expression and purification of proteins: the experimental procedure was the same as in Example 2.

The equilibrium dissociation constants (K_{D}) for binding of the IL-2 mutants described above in the present invention to their receptors were measured by biological optical interferometry (ForteBio).

The ForteBio affinity assay was conducted according to the method (Estep, P et al., High throughput solution Based measurement of antibody-antigen affinity and epitope binding. mAbs, 2013.5(2): p. 270-8) known in the art. The affinity of a candidate molecule for each of IL-2Rα, IL-2RP and IL-2Rβγ was measured: a sensor was equilibrated offline in an assay buffer for 20 min and equilibrated online for 120 s to establish a baseline; the human biotin-labeled IL-2Rα, IL-2RP or IL-2Rβγ was loaded onto an SA sensor (PALL, 18-5019) for ForteBio affinity assay; the sensor loaded with the IL-2 receptor was placed into a solution containing 100 nM IL-2 mutant molecules to a plateau, and then transferred to an assay buffer for dissociation for at least 2 min to measure the association and dissociation rates. The kinetic analysis was performed using a 1:1 binding model.

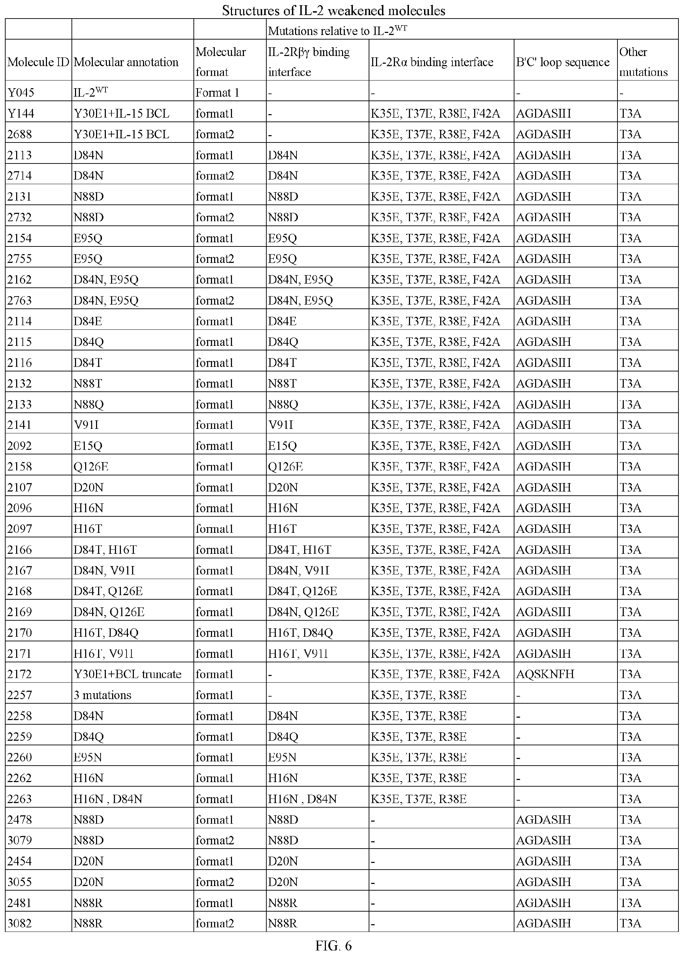

The experimental results are shown in Table 9. The expression yields and purity of the IL-2 mutants were improved relative to the wild-type (Y045), and meanwhile, the introduction of the IL-2Rβγ binding interface amino acid mutations led to different levels of reductions in the affinity for IL-2RP and IL-2Rβγ. The structures of the molecules in the table below are shown in FIG. 6.

**Table 9. Expression of IL-2 mutants in HEK293 and their affinity for IL-2 receptors**

| Molecule name | Expression yield (mg/L) | Purity (SEC-HPLC) | K_{D} (M) for IL-2Rα | K_{D} (M) for IL-2Rβ | K_{D} (M) for IE-2Kβγ |
|---|---|---|---|---|---|
| Y045 | 11.9 | 50.85% | 1.05E-09 | 2.45E-08 | - |
| Y144 | 114 | 97.78% | N.B. | 2.95E-09 | 8.67E-11 |
| 2688 | 156 | 84.38% | - | 7.90E-09 | 1.50E-10 |
| 2113 | 295 | 97.36% | - | 1.36E-08 | 2.47E-10 |
| 2131 | 281 | 97.20% | - | 1.19E-08 | 4.67E-10 |
| 2154 | 273 | 97.22% | - | 6.08E-09 | - |
| 2162 | 167.3 | 96.96% | - | 3.71E-08 | 3.13E-10 |
| 2714 | 158.2 | 85.16% | - | 3.03E-08 | 4.38E-10 |
| 2732 | 166.5 | 84.67% | - | N.B. | 3.18E-09 |
| 2755 | 168.7 | 85.02% | - | 6.32E-09 | 5.37E-10 |
| 2763 | 147.4 | 85.53% | - | 1.71E-08 | 1.60E-09 |
| 2114 | 244.4 | 97.77% | - | 1.13E-08 | 1.72E-10 |
| 2115 | 256.5 | - | - | 1.61E-08 | 4.59E-10 |
| 2116 | 239.4 | 92.05% | - | 1.76E-08 | 3.74E-10 |
| 2132 | 230.1 | 95.23% | - | N.B. | 1.99E-09 |
| 2133 | 240.5 | 97.22% | - | N.B. | 7.68E-09 |
| 2141 | 250.6 | 96.15% | - | 7.56E-09 | 1.12E-10 |
| 2092 | 346.8 | 95.57% | - | 2.31E-09 | 1.53E-10 |
| 2158 | 225 | 96.61% | - | 6.77E-09 | 3.58E-10 |
| 2107 | 307.7 | 91.97% | - | N.B. | 2.63E-09 |
| 2096 | 294.6 | 96.33% | - | 1.39E-08 | 1.12E-10 |
| 2097 | 220.5 | 96.37% | - | 1.35E-08 | 1.18E-10 |
| 2172 | 121.9 | 96.60% | - | 2.78E-09 | - |
| 2257 | 131.4 | 88.47% | - | 7.14E-10 | - |
| 2258 | 145.6 | 77.64% | - | 1.39E-09 | - |
| 2259 | 145.9 | 86.68% | - | - | - |
| 2260 | 124.2 | 83.24% | - | - | - |
| 2262 | 63.9 | 96.89% | - | 1.05E-08 | - |
| 2263 | 100.4 | 70.62% | - | 2.77E-08 | - |
| 2166 | 221.7 | 97.02% | - | 3.30E-07 | 1.72E-09 |
| 2167 | 144.3 | 96.24% | - | 2.85E-08 | 2.87E-10 |
| 2168 | 129.7 | 96.59% | - | 2.33E-08 | 6.97E-09 |
| 2169 | 105.2 | 96.64% | - | 2.47E-08 | 6.68E-09 |
| 2170 | 128.8 | 96.57% | - | 4.96E-08 | 8.61E-10 |
| 2171 | 125.7 | 95.44% | - | 1.53E-08 | 1.58E-10 |
| 2478 | 90.7 | 98.56% | - | N.B. | - |
| 2454 | 100 | 90.47% | - | N.B. | - |
| 2481 | 60 | 97.97% | - | N.B. | - |
| 3079 | 100 | 93.34% | 7.29E-09 | N.B. | 3.79E-08 |
| 3055 | 130 | 85.74% | 8.06E-09 | N.B. | 3.61E-08 |
| 3082 | 100 | 94.43% | 8.17E-09 | N.B. | 2.65E-08 |

| | | | | | |
|---|---|---|---|---|---|
| Note: "-" indicates no detection was performed; "N.B." represents non-bound. | | | | | |

### Example 4: In Vitro Functional Assays of IL-2 Mutants

### Detection of pSTATS signals caused by IL-2 mutants in human T lymphocytes

The binding of IL-2 to an IL-2 receptor on the surface of a T cell will activate the JAK-STAT signaling pathway of the human T lymphocyte. The phosphorylation level of STATS is an important measure for the activation level of the signaling pathway. Normal T lymphocytes do not substantially express IL-2Rα. The activation activity of different IL-2 mutant molecules for human T cells was assessed by detecting the pSTAT5 signal caused by the IL-2 mutant molecule in T lymphocytes.

Experimental materials and methods:
Experimental method:
   1. Thawing of PBMC cells
      (1) PBMCs (All Cells, Catalog No. PB004F-C, No. LP191011A/LP190529) cryopreserved in liquid nitrogen were thawed by rapidly shaking at 37 °C.
      (2) The cells were slowly added to 10 mL of CTS medium (Gibco, Catalog No. A3021002, Lot No. 1989823, preheated at 37 °C and containing 100 µL of DNase).
      (3) The medium was centrifuged at 300 g for 8 min, and the supernatant was removed.
      (4) The residue was resuspended in 10 mL of CTS. The suspension was transferred to a T75 culture flask (NUNC, Catalog No. 156499) and stabilized in a 5% incubator at 37 °C overnight.
   2. pSTAT5 test
      (1) The suspended PBMCs cultured overnight were plated on to a 96-well U-plate (Cornning, Catalog No. CLS3799-50EA) at 5 × 10⁵ cells/well.
      (2) Different diluted test antibodies were added to the 96-well U-plate, and the cells were incubated with the test antibodies at 37 °C for 30 min.
      (3) The mixtures were centrifuged at 400 g for 5 min, and the supernatants were removed.
      (4) A 4% tissue cell fixation solution (Solarbio, Catalog No. P1110) was added at 200 µL/well, and the mixtures were centrifuged at 400 g at room temperature for 30 min.
      (5) A perm buffer (BD, Catalog No. 558050) was added at 200 µL/well, and the plate was let stand at 4 °C for 30 min and centrifuged at 400 g for 10 min.
      (6) A perm/wash Buffer (BD, Catalog No. 558050, Lot No. 7271605) was added at 200 µL/well. The cells were washed twice.
      (7) Antibody stain solutions were prepared. The amount of pSTAT5 antibody (BD, Catalog No. 562076, Lot No. 9141872) was 3 µL/100 µL perm/wash Buffer/well. The amounts of the BV421 anti-human CD3 antibody (Biolegend, Catalog No. 300434, Lot No. B271302), FTIC anti-human CD4 antibody (Biolegend, Catalog No. 300506, Lot No. B283935) and AF700 anti-human CD8a (Biolegend, Catalog No. 300924, Lot No. B253967) were 1 µL/100 µL perm/wash Buffer/well. Incubation was performed at room temperature for 1.5 h, followed by 2 washings with perm/wash Buffer.
      (8) Resuspension was performed in 150 µL perm/wash Buffer/well, followed by a flow cytometry assay.

### Experimental results:

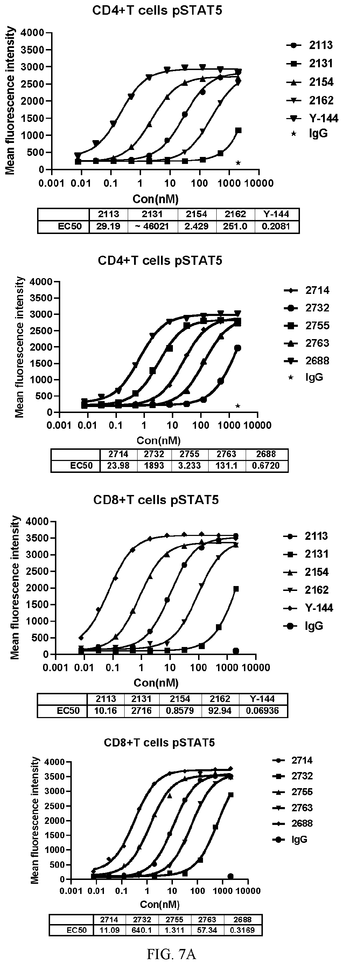

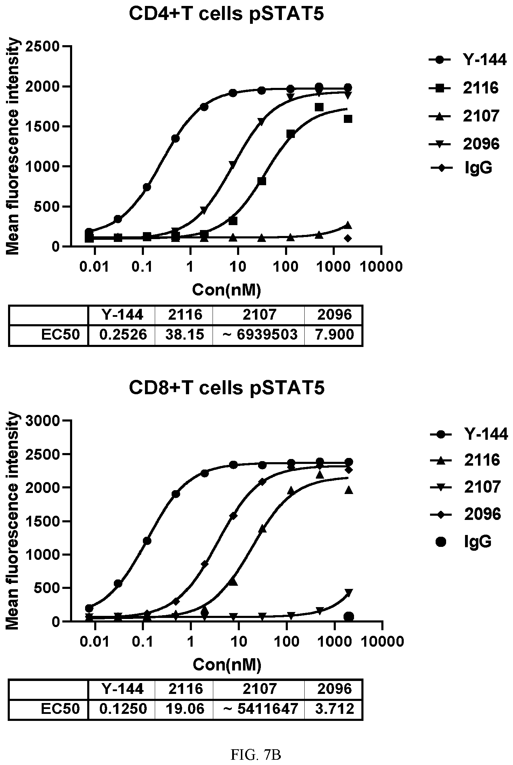

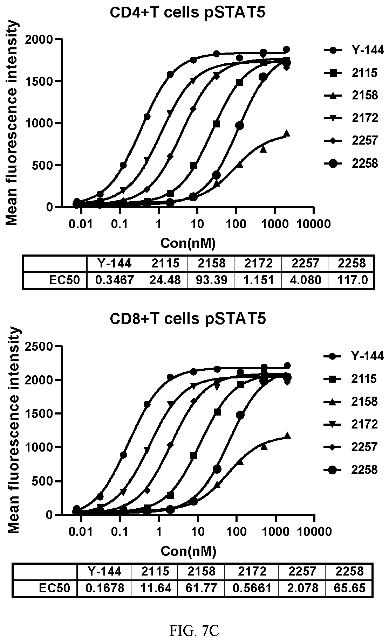

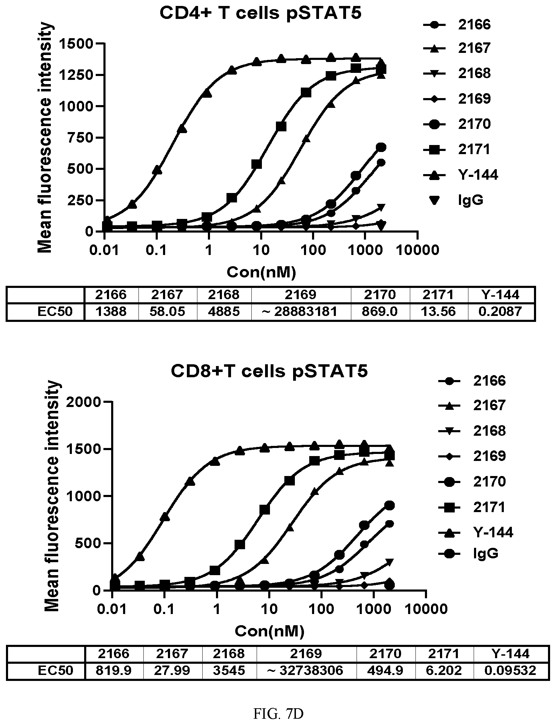

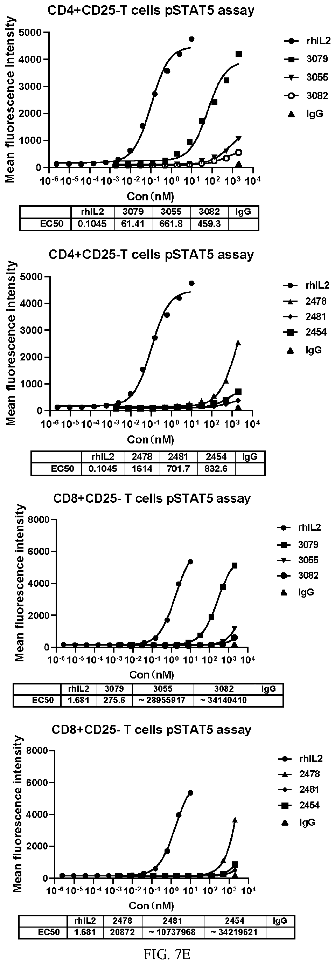

The pSTAT5 effect assay results of IL-2 mutant molecules in normal T lymphocytes (CD4⁺ T cells and CD8⁺ T cells) are shown in FIGs. 7A, 7B, 7C, 7D and 7E.

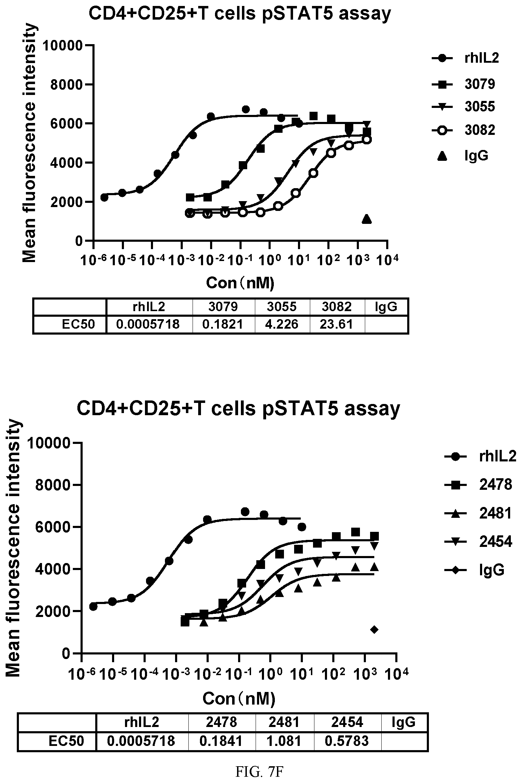

The phosphorylation levels of CD4⁺ and CD8⁺ T cells represent the proliferation capacity of the cells and the activity of the cells. It can be seen from FIG. 7 and Table 10 that the phosphorylation levels of T cells caused by the IL-2 mutants were reduced to different degrees by mutating one or more amino acids at the IL-2RP binding interface, relative to the molecules Y-144 (format 1) and 2688 (format 2) without mutations at the IL-2RP binding interface.

**Table 10. Ratios of reductions in the activation activity of IL-2 mutants for CD4⁺ or CD8⁺ T cells**

| Ratio of reduction | 10-50 times | 50-100 times | 100-1000 times | >1000 times |
|---|---|---|---|---|
| IL-2 mutant EC₅₀ (CD4⁺ or CD8⁺)/Y-144 EC₅₀ | 2154, 2096, 2257 | 2115, 2171 | 2113, 2116, 2158, 2258, 2167 | 2131, 2162, 2107, 2166, 2168, 2169, 2170 |
| IL-2 mutant EC₅₀ (CD4⁺ or CD8⁺)/2688 EC₅₀ | 2714 | | 2763 | 2732 |

### Detection of pSTAT5 signals caused by IL-2 mutants in activated T lymphocytes

The binding of IL-2 to an IL-2 receptor on the surface of a T cell will activate the JAK-STAT signaling pathway of the T lymphocyte. The phosphorylation level of STATS is an important measure for the activation level of the signaling pathway. After the T lymphocyte is activated, the abundance of IL-2Rα (CD25) on the surface of the T cell is significantly increased.

Experimental method:
1. Thawing of PBMC cells
   (1) PBMCs (All Cells, Catalog No. PB004F-C, No. LP191011A) cryopreserved in liquid nitrogen were thawed by rapidly shaking at 37 °C.
   (2) The cells were slowly added to 10 mL of CTS medium (Gibco, Catalog No. A3021002, Lot No. 1989823, preheated at 37 °C and containing 100 µL of DNase).
   (3) The medium was centrifuged at 300 g for 8 min, and the supernatant was removed.
   (4) The residue was resuspended in 10 mL of CTS. The suspension was transferred to a T75 culture flask (NUNC, Catalog No. 156499) and stabilized in a 5% incubator (Thermo, Catalog No. 3111) at 37 °C overnight.
2. Activation and resting of T lymphocytes
   (1) The suspended PBMCs cultured overnight were counted, and activated and stimulated for 48 h by adding an equal number of CD3/CD28 Beads (Invitrogen, Catalog No. 11131D, Lot No. 00783216).
   (2) The beads and medium were removed and the activated cells were washed.
   (3) The activated cells were transferred to a T75 culture flask and rested at 37 °C/5% for 48 h.
3. pSTAT5 test
   (1) The cells were plated on to a 96-well U-plate (Cornning, Catalog No. CLS3799-50EA) at 5 × 10⁵ cells/well.
   (2) Different diluted test antibodies were added to the 96-well U-plate, and the cells were incubated with the test antibodies at 37 °C for 30 min.
   (3) The mixtures were centrifuged at 400 g for 5 min, and the supernatants were removed.
   (4) A 4% tissue cell fixation solution (Solarbio, Catalog No. P1110) was added at 200 µL/well, and the mixtures were centrifuged at 400 g at room temperature for 30 min.
   (5) A perm buffer (BD, Catalog No. 558050) was added at 200 µL/well, and the plate was let stand at 4 °C for 30 min and centrifuged at 400 g for 10 min.
   (6) A perm/wash Buffer (BD, Catalog No. 558050, Lot No. 7271605) was added at 200 µL/well. The cells were washed twice.
   (7) Antibody stain solutions were prepared. The amount of pSTAT5 antibody (BD, Catalog No. 562076, Lot No. 9141872) was 3 µL/100 µL perm/wash Buffer/well. The amounts of the BV421 anti-human CD3 antibody (Biolegend, Catalog. No. 300434, Lot No. B271302), FTIC anti-human CD4 antibody (Biolegend, Catalog No. 300506, Lot No. B283935) and AF700 anti-human CD8a (Biolegend, Catalog No. 300924, Lot No. B253967) were 1 µL/100 µL perm/wash Buffer/well. Incubation was performed at room temperature for 1.5 h, followed by 2 washings with perm/wash Buffer.
   (8) Resuspension was performed in 150 µL perm/wash Buffer/well, followed by a flow cytometry assay.

### Experimental results

The pSTAT5 effect assay results of the IL-2 mutant molecules in CD8⁺T cells, CD4⁺ CD25⁻T cells, NK cells (CD3⁻ CD56⁺) and Treg cells (CD3⁺ CD4⁺ CD25⁺) are shown in FIGs. 8A-G and Tables 11-12. It can be seen from the results that the IL-2 mutant molecules of this study had weaker pSTAT5 activity on lymphocytes than human wild-type IL-2 (rhIL-2, R&D systems, Catalog No. 202-IL) while showing great selectivity for Treg cells relative to other lymphocytes.

**Table 11. EC₅₀ of pSTAT5 of rhIL-2 and IL-2 mutants on different lymphocytes**

| EC50 (nM) | CD8⁺T | CD4⁺CD25⁻T | NK | Treg |
|---|---|---|---|---|
| rhIL-2 | 1.685 | 0.1024 | 0.02422 | 0.0003881 |
| 2478 | 21740 | 1475 | 22.08 | 0.06178 |
| 2454 | ∼ 34240531 | 636.5 | 147.6 | 0.1146 |
| 2481 | ∼ 34240531 | 636.5 | 147.6 | 0.1146 |
| 3079 | 275.4 | 63.16 | 2.298 | 0.08149 |
| 3055 | ∼ 28361571 | 578.4 | 83.79 | 1.165 |
| 3082 | ∼ 33933462 | 319.6 | 82.27 | 9.002 |

**Table 12. Ratios of selectivity of IL-2 mutants for Treg cells to that for other lymphocytes**

| Ratio (other lymphocytes EC₅₀/Treg cells) | CD8⁺T | CD4⁺CD25⁻T | NK | Treg |
|---|---|---|---|---|
| 2478 | 351893.8168 | 23875.04047 | 357.3972159 | 1 |
| 2454 | 298782993 | 5554.101222 | 1287.958115 | 1 |
| 2481 | 28232534.83 | 1632.687734 | 1290.859667 | 1 |
| 3079 | 3379.555774 | 775.0644251 | 28.19977911 | 1 |
| 3055 | 24344696.14 | 496.4806867 | 71.92274678 | 1 |
| 3082 | 3769546.99 | 35.50322151 | 9.139080204 | 1 |

### Example 5: In Vivo Anti-tumor Efficacy of IL-2 Mutant Molecule

To demonstrate the *in vivo* anti-tumor efficacy of the IL-2 mutant molecules, C57 mice were inoculated with MC38 cells to determine the anti-tumor efficacy of the IL-2 mutant molecules (Y144, 2113 and 2162) of the present invention (the structures of the molecules 2113 and 2162 are shown in FIG. 6). SPF female C57 mice (14-17 g, purchased from Beijing Vital River Laboratory Animal Technology Co., Ltd.) with certificate No. 110011201109499961 were used in the experiment.

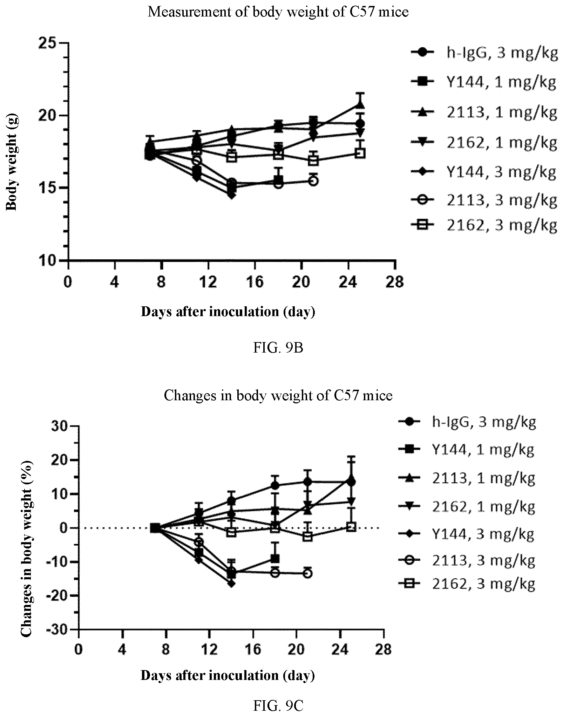

The MC38 cells were subcultured conventionally for subsequent *in vivo* study. The MC38 cells were collected by centrifugation and dispersed in PBS (1×) to form a cell suspension with a cell concentration of 5×10⁶ cells/mL. On day 0, 0.2 mL of the cell suspension was subcutaneously inoculated into the right abdominal region of the C57 mice to establish MC38 tumor-bearing mouse models. 7 days after the tumor cell inoculation, the tumor volume in each mouse was measured. The mice were divided into groups of 6. The dosages and routes of administration are shown in Tables 13 and 14. Administration was performed on days 7, 14 and 21 after the inoculation. The tumor volume and body weight of the mice were monitored twice a week for 25 days, as shown in FIGs. 9A and 9B. On day 25 after inoculation, the relative tumor growth inhibition (TGI%) was calculated by the following formula: TGI% = 100% × (control group tumor volume - treatment group tumor volume)/(control group tumor volume - control group tumor volume before administration). Tumor volume measurement: The maximum length of major axis (L) and maximum length of minor axis (W) of tumors were measured with a vernier caliper, and tumor volume was calculated using the following formula: V = L × W²/2. The mice were weighted using an electronic balance.

**Table 13. Experimental design (note: administration was performed every 7 days, 3 times in total)**

| Groups | Dosage of administration | Number of administration | Route of administration |
|---|---|---|---|
| h-IgG* | 3 mg/kg | Q7Dx3 | Intraperitoneal |
| Y144 | 1 mg/kg | Q7Dx3 | Intraperitoneal |
| Y144 | 3 mg/kg | Q7Dx3 | Intraperitoneal |
| 2113 | 1 mg/kg | Q7Dx3 | Intraperitoneal |
| 2113 | 3 mg/kg | Q7Dx3 | Intraperitoneal |
| 2162 | 1 mg/kg | Q7Dx3 | Intraperitoneal |
| 2162 | 3 mg/kg | Q7Dx3 | Intraperitoneal |

| | | | |
|---|---|---|---|
| * h-IgG: purchased from Equitech-Bio, Lot No. 161206-0656, 1 g/vial, prepared at 10 mg/mL with PBS. | | | |

The tumor volume measurements are shown in FIG. 9A. On day 25 after inoculation, compared to h-IgG, the weakened IL-2 molecules 2113 (3 mg/kg) and 2162 (3 mg/kg) had single-drug inhibition rates of 71.4% and 33.8%, respectively. The results show that the engineered IL2 molecules (2113 and 2162) had an anti-tumor effect and achieved a dose-dependent response. Meanwhile, the body weight of the mice was measured. As shown in FIGs. 9B and 9C, there was no significant difference in body weight of mice in the molecule 2113 (1 mg/kg), 2162 (1 mg/kg) and 2162 (3 mg/kg) groups. In contrast, in the group where the molecule Y144 not weakened was administered, the mice were intolerant and died before the end of the 25-day monitoring period.

The anti-tumor *in vivo* efficacy of several additional IL-2 mutant molecules (Y045, 2478 and 2454) was demonstrated in another MC38 tumor model (see FIG. 6 for the structures of molecules 2478 and 2454). SPF female C57 mice (14-17 g, purchased from Beijing Vital River Laboratory Animal Technology Co., Ltd.) with certificate No. 110011201109499826 were used to prepare MC38 tumor-bearing mice in the experiment. The experiment was conducted substantially as described above.

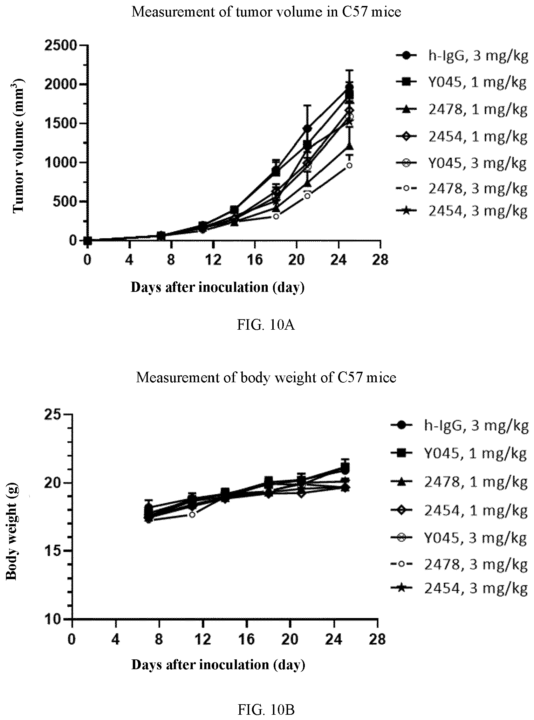

7 days after the tumor cell inoculation, the tumor volume in each mouse was measured. The mice were divided into groups of 7. The dosages and routes of administration are shown in Table 14. Administration was performed on days 7, 14 and 21 after the inoculation. The tumor volume and body weight of the mice were monitored 2-3 times a week for 25 days, as shown in FIGs. 10A and 10B. On day 25 after inoculation, the relative tumor growth inhibition (TGI%) was calculated by the following formula: TGI% = 100% × (control group tumor volume - treatment group tumor volume)/(control group tumor volume - control group tumor volume before administration). Tumor volume measurement: The maximum length of major axis (L) and maximum length of minor axis (W) of tumors were measured with a vernier caliper, and tumor volume was calculated using the following formula: V = L × W²/2. The mice were weighted using an electronic balance.

**Table 14. Experimental design (note: administration was performed every 7 days, 3 times in total)**

| Groups | Dosage of administration | Number of administration | Route of administration |
|---|---|---|---|
| h-IgG | 3 mg/kg | Q7Dx3 | Intraperitoneal |
| Y045 | 1 mg/kg | Q7Dx3 | Intraperitoneal |
| Y045 | 3 mg/kg | Q7Dx3 | Intraperitoneal |
| 2478 | 1 mg/kg | Q7Dx3 | Intraperitoneal |
| 2478 | 3 mg/kg | Q7Dx3 | Intraperitoneal |
| 2454 | 1 mg/kg | Q7Dx3 | Intraperitoneal |
| 2454 | 3 mg/kg | Q7Dx3 | Intraperitoneal |

| | | | |
|---|---|---|---|
| * h-IgG: purchased from Equitech-Bio, Lot No. 161206-0656, 1 g/vial, prepared at 10 mg/mL with PBS. | | | |

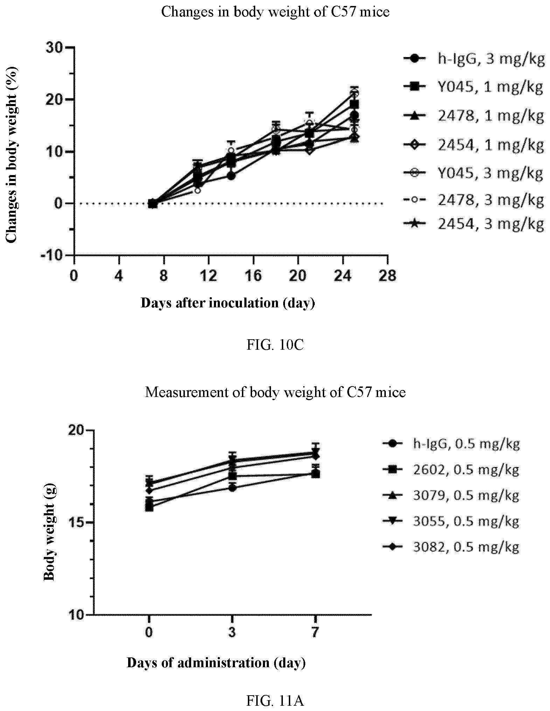

The tumor growth inhibition results are shown in Table 15: on day 25 after inoculation, compared to h-IgG, the molecules Y045 (1 mg/kg), 2478 (1 mg/kg), 2454 (1 mg/kg), Y045 (3 mg/kg), 2478 (3 mg/kg) and 2454 (3 mg/kg) had single-drug inhibition rates of 5.3%, 39.1%, 15.6%, 19.8%, 52.6% and 23.0%, respectively. The results show that the IL2 molecule not engineered (Y045) had an anti-tumor effect and achieved a dose-dependent response; the weakened IL2 molecules obtained by engineering (2478, 2454) had anti-tumor effects and achieved dose-dependent responses, and the efficacy was better than that of the IL2 molecule not engineered (Y045). Meanwhile, the body weight of the mice was measured. As shown in FIGs. 10B and 10C, there was no significant difference in body weight of mice.

**Table 15. Tumor growth inhibition on day 25**

| Groups | Tumor volume (mm³) | Tumor growth inhibition (%) |
|---|---|---|
| h-IgG, 3 mg/kg | 2473 | N/A |
| Y045, 1 mg/kg | 2377 | 5.3 |
| 2478, 1 mg/kg | 1772 | 39.1 |
| 2454, 1 mg/kg | 2323 | 15.6 |
| Y045, 3 mg/kg | 2131 | 19.8 |
| 2478, 3 mg/kg | 1478 | 52.6 |
| 2454, 3 mg/kg | 2354 | 23.0 |

### Example 6: In Vivo Study of IL-2 Mutant Molecules in Autoimmunity

To demonstrate the potential of IL-2 weakened molecules in autoimmunity, the effects of the IL-2 weakened molecules of the present invention on immune cells were determined using the blood collected from C57 mice before administration and on days 3 and 7 after administration. SPF female C57 mice (14-17 g, purchased from Beijing Vital River Laboratory Animal Technology Co., Ltd.) with certificate No. 110011201109500076 were used in the experiment.

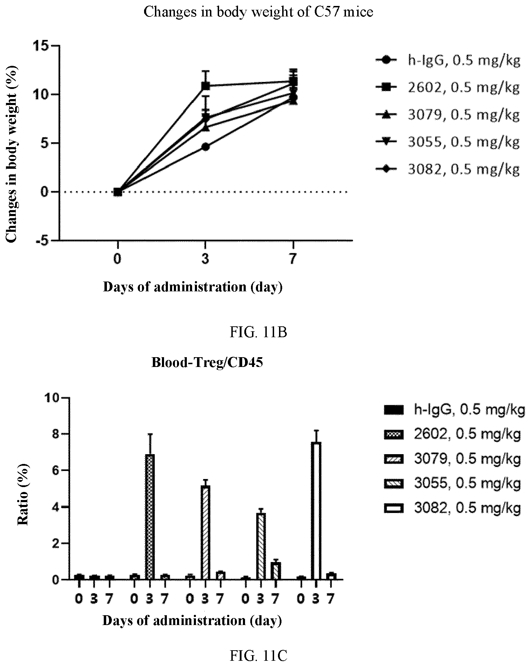

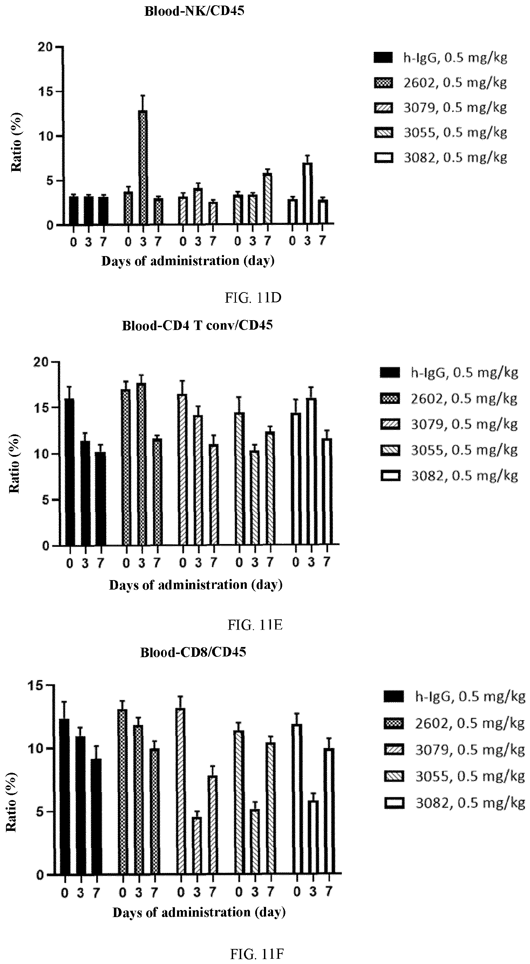

30 C57 mice were randomized into groups of 6. The dosages and routes of administration are shown in Table 16. A single dose was administered. The body weight of the mice was monitored twice a week for 7 days, as shown in FIGs. 11A and 11B. Blood was collected from the mice before administration and on days 3 and 7 after administration and tested for changes in the percentages of Treg, NK, CD4 T conv (CD4⁺ Foxp3⁻) and CD8⁺ T cells in the total number of CD45⁺ T lymphocytes, as shown in FIGs. 11C, 11D, 11E and 11F.

The tested molecule 2602 is an IL-2-Fc dimeric molecule of format 2 comprising the wild-type IL-2^{WT} sequence SEQ ID NO: 1; the structures of the weakened molecules 3079, 3055 and 3082 are shown in FIG. 6, and they are all IL-2-Fc dimeric molecules of format 2 as well.

It can be concluded from the results that the IL-2 mutant molecules of this study has weakened binding to IL-2RP, so that the IL-2 mutant molecules had weaker stimulatory effects on the proliferation of pro-inflammatory NK cells than the wild-type IL-2 molecule 2602 (IL-2^{WT}), but maintained the expansion of the immunosuppression cells Treg cells; the IL-2 mutant molecules showed similar Treg proliferation levels. In addition, the IL-2 mutant molecules had no significant proliferation effect on CD4 T conv and CD8 T cells, which suggests that its selectivity for Treg and other immune cells can be greatly improved by mutating the binding sites of IL-2 to IL-2Rβ.

**Table 16. Experimental design (note: SD: Single dose)**

| Groups | Dosage of administration | Number of administration | Route of administration |
|---|---|---|---|
| h-IgG | 0.5 mg/kg | SD | Intraperitoneal |
| 2602 | 0.5 mg/kg | SD | Intraperitoneal |
| 3079 | 0.5 mg/kg | SD | Intraperitoneal |
| 3055 | 0.5 mg/kg | SD | Intraperitoneal |
| 3082 | 0.5 mg/kg | SD | Intraperitoneal |

| | | | |
|---|---|---|---|
| * h-IgG: purchased from Equitech-Bio, Lot No. 161206-0656, 1 g/vial, prepared at 10 mg/mL with PBS. | | | |

Sequence listing description

| SEQ ID NO | Name | Description |
|---|---|---|
| 1 | Wild-type IL-2 | IL-2^{wt} with mutation C125S |
| 2 | Full-length human IL-2 | A native hIL-2 |
| 3 | Mature human IL-2 | A native hIL-2 with signal peptides removed |
| 4 | IL-2 mutant | IL-2^{3X} with mutations C125S, R38D, K43E and E61R |
| 5 | IL-2Rα receptor | An IL-2Rα receptor with an avi tag and an His6 tag at the C-terminus |
| 6 | IL-2RP receptor | An IL-2RP receptor with an avi tag and an His6 tag at the C-terminus |
| 7 | IL-2Rβ-Fc.Knob | An IL-2RP with Fc-Knob connected at the C-terminus |
| 8 | IL-2Ry-Fc.Hole | An IL-2Rγ with Fc-Hole connected at the C-terminus |
| 9 | Fc.Knob | A human IgG1 Fc region with mutation LALA and Knob mutations T366W and S354C, for format 2 |
| 10 | Fc.Hole | A human IgG1 Fc region with mutation LALA and hole mutations Y349C, T366S, L368A and Y407V, as well as a hinge region, for format 2 |
| 11 | Fc.Hole | A human IgG1 Fc region with mutation LALA and hole mutations Y349C, T366S, L368A and Y407V, as well as mutation H435R, for IE-2βγ complex |
| 12 | Mutant Fc region | A human IgG1 Fc with mutation LALA, for format 1 |
| 13 | Y001 | IL-2^{wt}-GSGS-FcLALA |
| 14 | Y002 | IL-^{2.3X}-GSGS-FcLALA |
| 15 | Y017 | IL-2^{hyb15BCL}-GSGS-FcLALA |
| 16 | Y057 | IL-2^{truncate1}-GSGS-FcLALA |
| 17 | Y058 | IL-2^{truncate2}-GSGS-FcLALA |
| 18 | Y059 | IL-2^{truncate3}-GSGS-FcLALA |
| 19 | Y060 | IL-2^{truncate4}-GSGS-FcLALA |
| 20 | Y089 | IL-2^{.Y30E1truncate1}-2^{∗}(G4S)-FcLALA |
| 21 | Y092 | IL-2^{.Y30E1.15BCL}-2^{∗}(G4S)-FcLALA |
| 22 | Y144 | IL-2 ^{Y30E1.15BCL.T3A}-2^{∗}(G4S)-FcLALA |
| 23 | Y040 | IL-^{2.3X}-2^{∗}(G4S)-FcLALA |
| 24 | Y045 | IL-2^{WT}-2^{∗}(G4S)-FcLALA |
| 25 | IL-2 ^{15BCL} | An IL-2 mutant with a chimeric loop sequence AGDASIH |
| 26 | IL-2^{.truncate1} | An IL-2 mutant with a truncated loop sequence AQSKNFH |
| 27 | IL-2 ^{Y30E1} | An IL-2 mutant with K35E, T37E, R38E and F42A |
| 28 | *IL-2* ^{Y30E1.T3A} | An IL-2 mutant with K35E, T37E, R38E, F42A and T3A |
| 29 | IL-2 ^{Y30E1.15BCL.T3A} | An IL-2 mutant with K35E, T37E, R38E, F42A and T3A, as well as a chimeric loop sequence AGDASIH |
| 30 | IL-2 ^{Y30E1.truncate1.T3A} | An IL-2 mutant sequence with K35E, T37E, R38E, F42A and T3A, as well as a truncated loop sequence AQSKNFH |
| 31 | IL-2^{K35E.T37E.R38E.T3A} | An IL-2 mutant sequence with K35E, T37E, R38E and T3A |
| 32 | IL-2^{K35E.T37E.R38E.T3A.15BCL} | An IL-2 mutant sequence with K35E, T37E, R38E and T3A, as well as a chimeric loop sequence AGDASIH |
| 33 | IL-2^{K35E. T37E. R38E. T3A.truncate1} | An IL-2 mutant sequence with K35E, T37E, R38E and T3A, as well as a truncated loop sequence AQSKNFH |
| 34 | IL-2 ^{15BCL.T3A} | An IL-2 mutant sequence with T3A and a chimeric loop sequence AGDASIH |
| 35 | IL-2^{.truncate1.T3A} | An IL-2 mutant sequence with T3A and a truncated loop sequence AQSKNFH |
| 36-638 | - | An IL-2 sequence for forming a dimeric molecule |

The IL-2 dimeric molecules constructed in the present invention are described in detail in the table below. The "IL-2 sequence SEQ ID NO" column shows the amino acid sequences of the IL-2 portions of the molecules. The "annotations for IL-2 portions" column describes, through the amino acid sequences in the "SEQ ID NO:" column and the mutations in the "mutations" column, that the IL-2 amino acid sequences of the molecules consist of the SEQ ID NO: amino acid sequences with the mutations. "Format 1" indicates that the molecules are homodimers, and each monomer (from N-terminus to C-terminus) is formed by linking the IL-2 amino acid sequence, the linker sequence (G4S)2 and SEQ ID NO: 12 in order. "Format 2" indicates that the molecules are heterodimers, in which one monomer (from N-terminus to C-terminus) is formed by linking the IL-2 amino acid sequence, the linker sequence (G4S)2 and SEQ ID NO: 9 in order, and the other monomer consists of SEQ ID NO: 10.

| Molecule_ID | | Annotations for IL-2 portions | | IL-2 sequence SEQ ID NO | Molecule_ID | | Annotations for IL-2 portions | | IL-2 sequence SEQ ID NO |
|---|---|---|---|---|---|---|---|---|---|
| Format 1 | Format 2 | SEQ ID NO: | Mutations | | Format 1 | Format 2 | SEQ ID NO: | Mutations | |
| Y045 | 2602 | SEQ ID NO:1 | - | 36 | | | | | |
| Y30E1 | 2603 | SEQ ID NO:27 | - | 37 | Y144 | 2688 | SEQ ID NO:29 | - | 122 |
| 2003 | 2604 | SEQ ID NO:28 | L12R | 38 | 2088 | 2689 | SEQ ID NO:29 | L12R | 123 |
| 2004 | 2605 | SEQ ID NO:28 | L12K | 39 | 2089 | 2690 | SEQ ID NO:29 | L12K | 124 |
| 2005 | 2606 | SEQ ID NO:28 | L12E | 40 | 2090 | 2691 | SEQ ID NO:29 | L12E | 125 |
| 2006 | 2607 | SEQ ID NO:28 | L12Q | 41 | 2091 | 2692 | SEQ ID NO:29 | L12Q | 126 |
| 2007 | 2608 | SEQ ID NO:28 | E15Q | 42 | 2092 | 2693 | SEQ ID NO:29 | E15Q | 127 |
| 2008 | 2609 | SEQ ID NO:28 | E15R | 43 | 2093 | 2694 | SEQ ID NO:29 | E15R | 128 |
| 2009 | 2610 | SEQ ID NO:28 | E15A | 44 | 2094 | 2695 | SEQ ID NO:29 | E15A | 129 |
| 2010 | 2611 | SEQ ID NO:28 | E15S | 45 | 2095 | 2696 | SEQ ID NO:29 | E15S | 130 |
| 2011 | 2612 | SEQ ID NO:28 | H16N | 46 | 2096 | 2697 | SEQ ID NO:29 | H16N | 131 |
| 2012 | 2613 | SEQ ID NO:28 | H16T | 47 | 2097 | 2698 | SEQ ID NO:29 | H16T | 132 |
| 2013 | 2614 | SEQ ID NO:28 | H16Y | 48 | 2098 | 2699 | SEQ ID NO:29 | H16Y | 133 |
| 2014 | 2615 | SEQ ID NO:28 | H16A | 49 | 2099 | 2700 | SEQ ID NO:29 | H16A | 134 |
| 2015 | 2616 | SEQ ID NO:28 | H16E | 50 | 2100 | 2701 | SEQ ID NO:29 | H16E | 135 |
| 2016 | 2617 | SEQ ID NO:28 | H16D | 51 | 2101 | 2702 | SEQ ID NO:29 | H16D | 136 |
| 2017 | 2618 | SEQ ID NO:28 | H16R | 52 | 2102 | 2703 | SEQ ID NO:29 | H16R | 137 |
| 2018 | 2619 | SEQ ID NO:28 | L19D | 53 | 2103 | 2704 | SEQ ID NO:29 | L19D | 138 |
| 2019 | 2620 | SEQ ID NO:28 | L19E | 54 | 2104 | 2705 | SEQ ID NO:29 | L19E | 139 |
| 2020 | 2621 | SEQ ID NO:28 | L19R | 55 | 2105 | 2706 | SEQ ID NO:29 | L19R | 140 |
| 2021 | 2622 | SEQ ID NO:28 | L19S | 56 | 2106 | 2707 | SEQ ID NO:29 | L19S | 141 |
| 2022 | 2623 | SEQ ID NO:28 | D20N | 57 | 2107 | 2708 | SEQ ID NO:29 | D20N | 142 |
| 2023 | 2624 | SEQ ID NO:28 | D20Q | 58 | 2108 | 2709 | SEQ ID NO:29 | D20Q | 143 |
| 2024 | 2625 | SEQ ID NO:28 | D20E | 59 | 2109 | 2710 | SEQ ID NO:29 | D20E | 144 |
| 2025 | 2626 | SEQ ID NO:28 | D20A | 60 | 2110 | 2711 | SEQ ID NO:29 | D20A | 145 |
| 2026 | 2627 | SEQ ID NO:28 | D20R | 61 | 2111 | 2712 | SEQ ID NO:29 | D20R | 146 |
| 2027 | 2628 | SEQ ID NO:28 | D20S | 62 | 2112 | 2713 | SEQ ID NO:29 | D20S | 147 |
| 2028 | 2629 | SEQ ID NO:28 | D84N | 63 | 2113 | 2714 | SEQ ID NO:29 | D84N | 148 |
| 2029 | 2630 | SEQ ID NO:28 | D84E | 64 | 2114 | 2715 | SEQ ID NO:29 | D84E | 149 |
| 2030 | 2631 | SEQ ID NO:28 | D84Q | 65 | 2115 | 2716 | SEQ ID NO:29 | D84Q | 150 |
| 2031 | 2632 | SEQ ID NO:28 | D84T | 66 | 2116 | 2717 | SEQ ID NO:29 | D84T | 151 |
| 2032 | 2633 | SEQ ID NO:28 | D84S | 67 | 2117 | 2718 | SEQ ID NO:29 | D84S | 152 |
| 2033 | 2634 | SEQ ID NO:28 | D84R | 68 | 2118 | 2719 | SEQ ID NO:29 | D84R | 153 |
| 2034 | 2635 | SEQ ID NO:28 | D84G | 69 | 2119 | 2720 | SEQ ID NO:29 | D84G | 154 |
| 2035 | 2636 | SEQ ID NO:28 | D84M | 70 | 2120 | 2721 | SEQ ID NO:29 | D84M | 155 |
| 2036 | 2637 | SEQ ID NO:28 | D84F | 71 | 2121 | 2722 | SEQ ID NO:29 | D84F | 156 |
| 2037 | 2638 | SEQ ID NO:28 | D84L | 72 | 2122 | 2723 | SEQ ID NO:29 | D84L | 157 |
| 2038 | 2639 | SEQ ID NO:28 | D84K | 73 | 2123 | 2724 | SEQ ID NO:29 | D84K | 158 |
| 2039 | 2640 | SEQ ID NO:28 | D84H | 74 | 2124 | 2725 | SEQ ID NO:29 | D84H | 159 |
| 2040 | 2641 | SEQ ID NO:28 | S87T | 75 | 2125 | 2726 | SEQ ID NO:29 | S87T | 160 |
| 2041 | 2642 | SEQ ID NO:28 | S87R | 76 | 2126 | 2727 | SEQ ID NO:29 | S87R | 161 |
| 2042 | 2643 | SEQ ID NO:28 | S87K | 77 | 2127 | 2728 | SEQ ID NO:29 | S87K | 162 |
| 2043 | 2644 | SEQ ID NO:28 | S87L | 78 | 2128 | 2729 | SEQ ID NO:29 | S87L | 163 |
| 2044 | 2645 | SEQ ID NO:28 | S87M | 79 | 2129 | 2730 | SEQ ID NO:29 | S87M | 164 |
| 2045 | 2646 | SEQ ID NO:28 | S87H | 80 | 2130 | 2731 | SEQ ID NO:29 | S87H | 165 |
| 2046 | 2647 | SEQ ID NO:28 | N88D | 81 | 2131 | 2732 | SEQ ID NO:29 | N88D | 166 |
| 2047 | 2648 | SEQ ID NO:28 | N88T | 82 | 2132 | 2733 | SEQ ID NO:29 | N88T | 167 |
| 2048 | 2649 | SEQ ID NO:28 | N88Q | 83 | 2133 | 2734 | SEQ ID NO:29 | N88Q | 168 |
| 2049 | 2650 | SEQ ID NO:28 | N88R | 84 | 2134 | 2735 | SEQ ID NO:29 | N88R | 169 |
| 2050 | 2651 | SEQ ID NO:28 | N88E | 85 | 2135 | 2736 | SEQ ID NO:29 | N88E | 170 |
| 2051 | 2652 | SEQ ID NO:28 | N88K | 86 | 2136 | 2737 | SEQ ID NO:29 | N88K | 171 |
| 2052 | 2653 | SEQ ID NO:28 | N88H | 87 | 2137 | 2738 | SEQ ID NO:29 | N88H | 172 |
| 2053 | 2654 | SEQ ID NO:28 | N88M | 88 | 2138 | 2739 | SEQ ID NO:29 | N88M | 173 |
| 2054 | 2655 | SEQ ID NO:28 | N88S | 89 | 2139 | 2740 | SEQ ID NO:29 | N88S | 174 |
| 2055 | 2656 | SEQ ID NO:28 | N88L | 90 | 2140 | 2741 | SEQ ID NO:29 | N88L | 175 |
| 2056 | 2657 | SEQ ID NO:28 | V91I | 91 | 2141 | 2742 | SEQ ID NO:29 | V91I | 176 |
| 2057 | 2658 | SEQ ID NO:28 | V91L | 92 | 2142 | 2743 | SEQ ID NO:29 | V91L | 177 |
| 2058 | 2659 | SEQ ID NO:28 | V91D | 93 | 2143 | 2744 | SEQ ID NO:29 | V91D | 178 |
| 2059 | 2660 | SEQ ID NO:28 | V91E | 94 | 2144 | 2745 | SEQ ID NO:29 | V91E | 179 |
| 2060 | 2661 | SEQ ID NO:28 | V91N | 95 | 2145 | 2746 | SEQ ID NO:29 | V91N | 180 |
| 2061 | 2662 | SEQ ID NO:28 | V91Q | 96 | 2146 | 2747 | SEQ ID NO:29 | V91Q | 181 |
| 2062 | 2663 | SEQ ID NO:28 | V91S | 97 | 2147 | 2748 | SEQ ID NO:29 | V91S | 182 |
| 2063 | 2664 | SEQ ID NO:28 | V91H | 98 | 2148 | 2749 | SEQ ID NO:29 | V91H | 183 |
| 2064 | 2665 | SEQ ID NO:28 | I92E | 99 | 2149 | 2750 | SEQ ID NO:29 | I92E | 184 |
| 2065 | 2666 | SEQ ID NO:28 | I92T | 100 | 2150 | 2751 | SEQ ID NO:29 | I92T | 185 |
| 2066 | 2667 | SEQ ID NO:28 | I92K | 101 | 2151 | 2752 | SEQ ID NO:29 | I92K | 186 |
| 2067 | 2668 | SEQ ID NO:28 | I92R | 102 | 2152 | 2753 | SEQ ID NO:29 | I92R | 187 |
| 2068 | 2669 | SEQ ID NO:28 | I92L | 103 | 2153 | 2754 | SEQ ID NO:29 | I92L | 188 |
| 2069 | 2670 | SEQ ID NO:28 | E95Q | 104 | 2154 | 2755 | SEQ ID NO:29 | E95Q | 189 |
| 2070 | 2671 | SEQ ID NO:28 | E95G | 105 | 2155 | 2756 | SEQ ID NO:29 | E95G | 190 |
| 2071 | 2672 | SEQ ID NO:28 | E95D | 106 | 2156 | 2757 | SEQ ID NO:29 | E95D | 191 |
| 2072 | 2673 | SEQ ID NO:28 | E95N | 107 | 2157 | 2758 | SEQ ID NO:29 | E95N | 192 |
| 2073 | 2674 | SEQ ID NO:28 | Q126E | 108 | 2158 | 2759 | SEQ ID NO:29 | Q126E | 193 |
| 2074 | 2675 | SEQ ID NO:28 | Q126D | 109 | 2159 | 2760 | SEQ ID NO:29 | Q126D | 194 |
| 2075 | 2676 | SEQ ID NO:28 | Q126A | 110 | 2160 | 2761 | SEQ ID NO:29 | Q126A | 195 |
| 2076 | 2677 | SEQ ID NO:28 | Q126S | 111 | 2161 | 2762 | SEQ ID NO:29 | Q126S | 196 |
| 2077 | 2678 | SEQ ID NO:28 | D84N+E95Q | 112 | 2162 | 2763 | SEQ ID NO:29 | D84N+ E95Q | 197 |
| 2078 | 2679 | SEQ ID NO:28 | D84E+E95Q | 113 | 2163 | 2764 | SEQ ID NO:29 | D84E+ E95Q | 198 |
| 2079 | 2680 | SEQ ID NO:28 | D84T+E95Q | 114 | 2164 | 2765 | SEQ ID NO:29 | D84T+ E95Q | 199 |
| 2080 | 2681 | SEQ ID NO:28 | D84Q+E95Q | 115 | 2165 | 2766 | SEQ ID NO:29 | D84Q+ E95Q | 200 |
| 2081 | 2682 | SEQ ID NO:28 | D84T+H16T | 116 | 2166 | 2767 | SEQ ID NO:29 | D84T+ H16T | 201 |
| 2082 | 2683 | SEQ ID NO:28 | D84N+ V91I | 117 | 2167 | 2768 | SEQ ID NO:29 | D84N + V91I | 202 |
| 2083 | 2684 | SEQ ID NO:28 | D84T+Q126E | 118 | 2168 | 2769 | SEQ ID NO:29 | D84T+ Q126E | 203 |
| 2084 | 2685 | SEQ ID NO:28 | D84N+Q126 E | 119 | 2169 | 2770 | SEQ ID NO:29 | D84N + Q126E | 204 |
| 2085 | 2686 | SEQ ID NO:28 | H16T+D84Q | 120 | 2170 | 2771 | SEQ ID NO:29 | H16T + D84Q | 205 |
| 2086 | 2687 | SEQ ID NO:28 | H16T+V91I | 121 | 2171 | 2772 | SEQ ID NO:29 | H16T + V91I | 206 |
| | | | | | | | | | |

| Molecule ID | | Annotations for IL-2 portions | | IL-2 sequence SEQ ID NO | Molecule ID | | Annotations for IL-2 portions | | IL-2 sequence SEQ ID NO |
|---|---|---|---|---|---|---|---|---|---|
| Format 1 | Format 2 | SEQ ID NO: | Mutations | | Format 1 | Format 2 | SEQ ID NO: | Mutations | |
| 2172 | 2773 | SEQ ID NO: 30 | | 207 | 2264 | 2865 | SEQ ID NO: 32 | | 299 |
| 2173 | 2774 | SEQ ID NO: 30 | L12R | 208 | 2265 | 2866 | SEQ ID NO: 32 | L12R | 300 |
| 2174 | 2775 | SEQ ID NO: 30 | L12K | 209 | 2266 | 2867 | SEQ ID NO: 32 | L12K | 301 |
| 2175 | 2776 | SEQ ID NO: 30 | L12E | 210 | 2267 | 2868 | SEQ ID NO: 32 | L12E | 302 |
| 2176 | 2777 | SEQ ID NO: 30 | L12Q | 211 | 2268 | 2869 | SEQ ID NO: 32 | L12Q | 303 |
| 2177 | 2778 | SEQ ID NO: 30 | E15Q | 212 | 2269 | 2870 | SEQ ID NO: 32 | E15Q | 304 |
| 2178 | 2779 | SEQ ID NO: 30 | E15R | 213 | 2270 | 2871 | SEQ ID NO: 32 | E15R | 305 |
| 2179 | 2780 | SEQ ID NO: 30 | E15A | 214 | 2271 | 2872 | SEQ ID NO: 32 | E15A | 306 |
| 2180 | 2781 | SEQ ID NO: 30 | E15S | 215 | 2272 | 2873 | SEQ ID NO: 32 | E15S | 307 |
| 2181 | 2782 | SEQ ID NO: 30 | H16N | 216 | 2273 | 2874 | SEQ ID NO: 32 | H16N | 308 |
| 2182 | 2783 | SEQ ID NO: 30 | H16T | 217 | 2274 | 2875 | SEQ ID NO: 32 | H16T | 309 |
| 2183 | 2784 | SEQ ID NO: 30 | H16Y | 218 | 2275 | 2876 | SEQ ID NO: 32 | H16Y | 310 |
| 2184 | 2785 | SEQ ID NO: 30 | H16A | 219 | 2276 | 2877 | SEQ ID NO: 32 | H16A | 311 |
| 2185 | 2786 | SEQ ID NO: 30 | H16E | 220 | 2277 | 2878 | SEQ ID NO: 32 | H16E | 312 |
| 2186 | 2787 | SEQ ID NO: 30 | H16D | 221 | 2278 | 2879 | SEQ ID NO: 32 | H16D | 313 |
| 2187 | 2788 | SEQ ID NO: 30 | H16R | 222 | 2279 | 2880 | SEQ ID NO: 32 | H16R | 314 |
| 2188 | 2789 | SEQ ID NO: 30 | L19D | 223 | 2280 | 2881 | SEQ ID NO: 32 | L19D | 315 |
| 2189 | 2790 | SEQ ID NO: 30 | L19E | 224 | 2281 | 2882 | SEQ ID NO: 32 | L19E | 316 |
| 2190 | 2791 | SEQ ID NO: 30 | L19R | 225 | 2282 | 2883 | SEQ ID NO: 32 | L19R | 317 |
| 2191 | 2792 | SEQ ID NO: 30 | L19S | 226 | 2283 | 2884 | SEQ ID NO: 32 | L19S | 318 |
| 2192 | 2793 | SEQ ID NO: 30 | D20N | 227 | 2284 | 2885 | SEQ ID NO: 32 | D20N | 319 |
| 2193 | 2794 | SEQ ID NO: 30 | D20Q | 228 | 2285 | 2886 | SEQ ID NO: 32 | D20Q | 320 |
| 2194 | 2795 | SEQ ID NO: 30 | D20E | 229 | 2286 | 2887 | SEQ ID NO: 32 | D20E | 321 |
| 2195 | 2796 | SEQ ID NO: 30 | D20A | 230 | 2287 | 2888 | SEQ ID NO: 32 | D20A | 322 |
| 2196 | 2797 | SEQ ID NO: 30 | D20R | 231 | 2288 | 2889 | SEQ ID NO: 32 | D20R | 323 |
| 2197 | 2798 | SEQ ID NO: 30 | D20S | 232 | 2289 | 2890 | SEQ ID NO: 32 | D20S | 324 |
| 2198 | 2799 | SEQ ID NO: 30 | D84N | 233 | 2290 | 2891 | SEQ ID NO: 32 | D84N | 325 |
| 2199 | 2800 | SEQ ID NO: 30 | D84E | 234 | 2291 | 2892 | SEQ ID NO: 32 | D84E | 326 |
| 2200 | 2801 | SEQ ID NO: 30 | D84Q | 235 | 2292 | 2893 | SEQ ID NO: 32 | D84Q | 327 |
| 2201 | 2802 | SEQ ID NO: 30 | D84T | 236 | 2293 | 2894 | SEQ ID NO: 32 | D84T | 328 |
| 2202 | 2803 | SEQ ID NO: 30 | D84S | 237 | 2294 | 2895 | SEQ ID NO: 32 | D84S | 329 |
| 2203 | 2804 | SEQ ID NO: 30 | D84R | 238 | 2295 | 2896 | SEQ ID NO: 32 | D84R | 330 |
| 2204 | 2805 | SEQ ID NO: 30 | D84G | 239 | 2296 | 2897 | SEQ ID NO: 32 | D84G | 331 |
| 2205 | 2806 | SEQ ID NO: 30 | D84M | 240 | 2297 | 2898 | SEQ ID NO: 32 | D84M | 332 |
| 2206 | 2807 | SEQ ID NO: 30 | D84F | 241 | 2298 | 2899 | SEQ ID NO: 32 | D84F | 333 |
| 2207 | 2808 | SEQ ID NO: 30 | D84L | 242 | 2299 | 2900 | SEQ ID NO: 32 | D84L | 334 |
| 2208 | 2809 | SEQ ID NO: 30 | D84K | 243 | 2300 | 2901 | SEQ ID NO: 32 | D84K | 335 |
| 2209 | 2810 | SEQ ID NO: 30 | D84H | 244 | 2301 | 2902 | SEQ ID NO: 32 | D84H | 336 |
| 2210 | 2811 | SEQ ID NO: 30 | S87T | 245 | 2302 | 2903 | SEQ ID NO: 32 | S87T | 337 |
| 2211 | 2812 | SEQ ID NO: 30 | S87R | 246 | 2303 | 2904 | SEQ ID NO: 32 | S87R | 338 |
| 2212 | 2813 | SEQ ID NO: 30 | S87K | 247 | 2304 | 2905 | SEQ ID NO: 32 | S87K | 339 |
| 2213 | 2814 | SEQ ID NO: 30 | S87L | 248 | 2305 | 2906 | SEQ ID NO: 32 | S87L | 340 |
| 2214 | 2815 | SEQ ID NO: 30 | S87M | 249 | 2306 | 2907 | SEQ ID NO: 32 | S87M | 341 |
| 2215 | 2816 | SEQ ID NO: 30 | S87H | 250 | 2307 | 2908 | SEQ ID NO: 32 | S87H | 342 |
| 2216 | 2817 | SEQ ID NO: 30 | N88D | 251 | 2308 | 2909 | SEQ ID NO: 32 | N88D | 343 |
| 2217 | 2818 | SEQ ID NO: 30 | N88T | 252 | 2309 | 2910 | SEQ ID NO: 32 | N88T | 344 |
| 2218 | 2819 | SEQ ID NO: 30 | N88Q | 253 | 2310 | 2911 | SEQ ID NO: 32 | N88Q | 345 |
| 2219 | 2820 | SEQ ID NO: 30 | N88R | 254 | 2311 | 2912 | SEQ ID NO: 32 | N88R | 346 |
| 2220 | 2821 | SEQ ID NO: 30 | N88E | 255 | 2312 | 2913 | SEQ ID NO: 32 | N88E | 347 |
| 2221 | 2822 | SEQ ID NO: 30 | N88K | 256 | 2313 | 2914 | SEQ ID NO: 32 | N88K | 348 |
| 2222 | 2823 | SEQ ID NO: 30 | N88H | 257 | 2314 | 2915 | SEQ ID NO: 32 | N88H | 349 |
| 2223 | 2824 | SEQ ID NO: 30 | N88M | 258 | 2315 | 2916 | SEQ ID NO: 32 | N88M | 350 |
| 2224 | 2825 | SEQ ID NO: 30 | N88S | 259 | 2316 | 2917 | SEQ ID NO: 32 | N88S | 351 |
| 2225 | 2826 | SEQ ID NO: 30 | N88L | 260 | 2317 | 2918 | SEQ ID NO: 32 | N88L | 352 |
| 2226 | 2827 | SEQ ID NO: 30 | V91I | 261 | 2318 | 2919 | SEQ ID NO: 32 | V91I | 353 |
| 2227 | 2828 | SEQ ID NO: 30 | V91L | 262 | 2319 | 2920 | SEQ ID NO: 32 | V91L | 354 |
| 2228 | 2829 | SEQ ID NO: 30 | V91D | 263 | 2320 | 2921 | SEQ ID NO: 32 | V91D | 355 |
| 2229 | 2830 | SEQ ID NO: 30 | V91E | 264 | 2321 | 2922 | SEQ ID NO: 32 | V91E | 356 |
| 2230 | 2831 | SEQ ID NO: 30 | V91N | 265 | 2322 | 2923 | SEQ ID NO: 32 | V91N | 357 |
| 2231 | 2832 | SEQ ID NO: 30 | V91Q | 266 | 2323 | 2924 | SEQ ID NO: 32 | V91Q | 358 |
| 2232 | 2833 | SEQ ID NO: 30 | V91S | 267 | 2324 | 2925 | SEQ ID NO: 32 | V91S | 359 |
| 2233 | 2834 | SEQ ID NO: 30 | V91H | 268 | 2325 | 2926 | SEQ ID NO: 32 | V91H | 360 |
| 2234 | 2835 | SEQ ID NO: 30 | I92E | 269 | 2326 | 2927 | SEQ ID NO: 32 | I92E | 361 |
| 2235 | 2836 | SEQ ID NO: 30 | I92T | 270 | 2327 | 2928 | SEQ ID NO: 32 | I92T | 362 |
| 2236 | 2837 | SEQ ID NO: 30 | I92K | 271 | 2328 | 2929 | SEQ ID NO: 32 | I92K | 363 |
| 2237 | 2838 | SEQ ID NO: 30 | I92R | 272 | 2329 | 2930 | SEQ ID NO: 32 | I92R | 364 |
| 2238 | 2839 | SEQ ID NO: 30 | I92L | 273 | 2330 | 2931 | SEQ ID NO: 32 | I92L | 365 |
| 2239 | 2840 | SEQ ID NO: 30 | E95Q | 274 | 2331 | 2932 | SEQ ID NO: 32 | E95Q | 366 |
| 2240 | 2841 | SEQ ID NO: 30 | E95G | 275 | 2332 | 2933 | SEQ ID NO: 32 | E95G | 367 |
| 2241 | 2842 | SEQ ID NO: 30 | E95D | 276 | 2333 | 2934 | SEQ ID NO: 32 | E95D | 368 |
| 2242 | 2843 | SEQ ID NO: 30 | E95N | 277 | 2334 | 2935 | SEQ ID NO: 32 | E95N | 369 |
| 2243 | 2844 | SEQ ID NO: 30 | Q126E | 278 | 2335 | 2936 | SEQ ID NO: 32 | Q126E | 370 |
| 2244 | 2845 | SEQ ID NO: 30 | Q126D | 279 | 2336 | 2937 | SEQ ID NO: 32 | Q126D | 371 |
| 2245 | 2846 | SEQ ID NO: 30 | Q126A | 280 | 2337 | 2938 | SEQ ID NO: 32 | Q126A | 372 |
| 2246 | 2847 | SEQ ID NO: 30 | Q126S | 281 | 2338 | 2939 | SEQ ID NO: 32 | Q126S | 373 |
| 2247 | 2848 | SEQ ID NO: 30 | D84N+E95Q | 282 | 2339 | 2940 | SEQ ID NO: 32 | D84N+E95Q | 374 |
| 2248 | 2849 | SEQ ID NO: 30 | D84E+E95Q | 283 | 2340 | 2941 | SEQ ID NO: 32 | D84E+E95Q | 375 |
| 2249 | 2850 | SEQ ID NO: 30 | D84T+E95Q | 284 | 2341 | 2942 | SEQ ID NO: 32 | D84T+E95Q | 376 |
| 2250 | 2851 | SEQ ID NO: 30 | D84Q+E95Q | 285 | 2342 | 2943 | SEQ ID NO: 32 | D84Q+E95Q | 377 |
| 2251 | 2852 | SEQ ID NO: 30 | D84T+H16T | 286 | 2343 | 2944 | SEQ ID NO: 32 | D84T+H16T | 378 |
| 2252 | 2853 | SEQ ID NO: 30 | D84N +V91I | 287 | 2344 | 2945 | SEQ ID NO: 32 | D84N +V91I | 379 |
| 2253 | 2854 | SEQ ID NO: 30 | D84T +Q126E | 288 | 2345 | 2946 | SEQ ID NO: 32 | D84T +Q126E | 380 |
| 2254 | 2855 | SEQ ID NO: 30 | D84N +Q126E | 289 | 2346 | 2947 | SEQ ID NO: 32 | D84N +Q126E | 381 |
| 2255 | 2856 | SEQ ID NO: 30 | H16T +D84Q | 290 | 2347 | 2948 | SEQ ID NO: 32 | H16T +D84Q | 382 |
| 2256 | 2857 | SEQ ID NO: 30 | H16T +V91I | 291 | 2348 | 2949 | SEQ ID NO: 32 | H16T +V91I | 383 |
| 2257 | 2858 | SEQ ID NO: 31 | | 292 | | | | | |
| 2258 | 2859 | SEQ ID NO: 31 | D84N | 293 | | | | | |
| 2259 | 2860 | SEQ ID NO: 31 | D84Q | 294 | | | | | |
| 2260 | 2861 | SEQ ID NO: 31 | E95N | 295 | | | | | |
| 2261 | 2862 | SEQ ID NO: 31 | D84N+E95N | 296 | | | | | |
| 2262 | 2863 | SEQ ID NO: 31 | H16N | 297 | | | | | |
| 2263 | 2864 | SEQ ID NO: 31 | H16N+D84N | 298 | | | | | |

| Molecule_ID | | Annotations for IL-2 portions | | IL-2 sequence SEQ ID NO | Molecule_ID | | Annotations for IL-2 portions | | IL-2 sequence SEQ ID NO |
|---|---|---|---|---|---|---|---|---|---|
| Format 1 | Format 2 | SEQ ID NO: | Mutations | | Format 1 | Format 2 | SEQ ID NO: | Mutations | |
| 2349 | 2950 | SEQ ID NO: 33 | | 384 | 2434 | 3035 | SEQ ID NO:34 | | 469 |
| 2350 | 2951 | SEQ ID NO: 33 | L12R | 385 | 2435 | 3036 | SEQ ID NO:34 | L12R | 470 |
| 2351 | 2952 | SEQ ID NO: 33 | L12K | 386 | 2436 | 3037 | SEQ ID NO:34 | L12K | 471 |
| 2352 | 2953 | SEQ ID NO: 33 | L12E | 387 | 2437 | 3038 | SEQ ID NO:34 | L12E | 472 |
| 2353 | 2954 | SEQ ID NO: 33 | L12Q | 388 | 2438 | 3039 | SEQ ID NO:34 | L12Q | 473 |
| 2354 | 2955 | SEQ ID NO: 33 | E15Q | 389 | 2439 | 3040 | SEQ ID NO:34 | E15Q | 474 |
| 2355 | 2956 | SEQ ID NO: 33 | E15R | 390 | 2440 | 3041 | SEQ ID NO:34 | E15R | 475 |
| 2356 | 2957 | SEQ ID NO: 33 | E15A | 391 | 2441 | 3042 | SEQ ID NO:34 | E15A | 476 |
| 2357 | 2958 | SEQ ID NO: 33 | E15S | 392 | 2442 | 3043 | SEQ ID NO:34 | E15S | 477 |
| 2358 | 2959 | SEQ ID NO: 33 | H16N | 393 | 2443 | 3044 | SEQ ID NO:34 | H16N | 478 |
| 2359 | 2960 | SEQ ID NO: 33 | H16T | 394 | 2444 | 3045 | SEQ ID NO:34 | H16T | 479 |
| 2360 | 2961 | SEQ ID NO: 33 | H16Y | 395 | 2445 | 3046 | SEQ ID NO:34 | H16Y | 480 |
| 2361 | 2962 | SEQ ID NO: 33 | H16A | 396 | 2446 | 3047 | SEQ ID NO:34 | H16A | 481 |
| 2362 | 2963 | SEQ ID NO: 33 | H16E | 397 | 2447 | 3048 | SEQ ID NO:34 | H16E | 482 |
| 2363 | 2964 | SEQ ID NO: 33 | H16D | 398 | 2448 | 3049 | SEQ ID NO:34 | H16D | 483 |
| 2364 | 2965 | SEQ ID NO: 33 | H16R | 399 | 2449 | 3050 | SEQ ID NO:34 | H16R | 484 |
| 2365 | 2966 | SEQ ID NO: 33 | L19D | 400 | 2450 | 3051 | SEQ ID NO:34 | L19D | 485 |
| 2366 | 2967 | SEQ ID NO: 33 | L19E | 401 | 2451 | 3052 | SEQ ID NO:34 | L19E | 486 |
| 2367 | 2968 | SEQ ID NO: 33 | L19R | 402 | 2452 | 3053 | SEQ ID NO:34 | L19R | 487 |
| 2368 | 2969 | SEQ ID NO: 33 | L19S | 403 | 2453 | 3054 | SEQ ID NO:34 | L19S | 488 |
| 2369 | 2970 | SEQ ID NO: 33 | D20N | 404 | 2454 | 3055 | SEQ ID NO:34 | D20N | 489 |
| 2370 | 2971 | SEQ ID NO: 33 | D20Q | 405 | 2455 | 3056 | SEQ ID NO:34 | D20Q | 490 |
| 2371 | 2972 | SEQ ID NO: 33 | D20E | 406 | 2456 | 3057 | SEQ ID NO:34 | D20E | 491 |
| 2372 | 2973 | SEQ ID NO: 33 | D20A | 407 | 2457 | 3058 | SEQ ID NO:34 | D20A | 492 |
| 2373 | 2974 | SEQ ID NO: 33 | D20R | 408 | 2458 | 3059 | SEQ ID NO:34 | D20R | 493 |
| 2374 | 2975 | SEQ ID NO: 33 | D20S | 409 | 2459 | 3060 | SEQ ID NO:34 | D20S | 494 |
| 2375 | 2976 | SEQ ID NO: 33 | D84N | 410 | 2460 | 3061 | SEQ ID NO:34 | D84N | 495 |
| 2376 | 2977 | SEQ ID NO: 33 | D84E | 411 | 2461 | 3062 | SEQ ID NO:34 | D84E | 496 |
| 2377 | 2978 | SEQ ID NO: 33 | D84Q | 412 | 2462 | 3063 | SEQ ID NO:34 | D84Q | 497 |
| 2378 | 2979 | SEQ ID NO: 33 | D84T | 413 | 2463 | 3064 | SEQ ID NO:34 | D84T | 498 |
| 2379 | 2980 | SEQ ID NO: 33 | D84S | 414 | 2464 | 3065 | SEQ ID NO:34 | D84S | 499 |
| 2380 | 2981 | SEQ ID NO: 33 | D84R | 415 | 2465 | 3066 | SEQ ID NO:34 | D84R | 500 |
| 2381 | 2982 | SEQ ID NO: 33 | D84G | 416 | 2466 | 3067 | SEQ ID NO:34 | D84G | 501 |
| 2382 | 2983 | SEQ ID NO: 33 | D84M | 417 | 2467 | 3068 | SEQ ID NO:34 | D84M | 502 |
| 2383 | 2984 | SEQ ID NO: 33 | D84F | 418 | 2468 | 3069 | SEQ ID NO:34 | D84F | 503 |
| 2384 | 2985 | SEQ ID NO: 33 | D84L | 419 | 2469 | 3070 | SEQ ID NO:34 | D84L | 504 |
| 2385 | 2986 | SEQ ID NO: 33 | D84K | 420 | 2470 | 3071 | SEQ ID NO:34 | D84K | 505 |
| 2386 | 2987 | SEQ ID NO: 33 | D84H | 421 | 2471 | 3072 | SEQ ID NO:34 | D84H | 506 |
| 2387 | 2988 | SEQ ID NO: 33 | S87T | 422 | 2472 | 3073 | SEQ ID NO:34 | S87T | 507 |
| 2388 | 2989 | SEQ ID NO: 33 | S87R | 423 | 2473 | 3074 | SEQ ID NO:34 | S87R | 508 |
| 2389 | 2990 | SEQ ID NO: 33 | S87K | 424 | 2474 | 3075 | SEQ ID NO:34 | S87K | 509 |
| 2390 | 2991 | SEQ ID NO: 33 | S87L | 425 | 2475 | 3076 | SEQ ID NO:34 | S87L | 510 |
| 2391 | 2992 | SEQ ID NO: 33 | S87M | 426 | 2476 | 3077 | SEQ ID NO:34 | S87M | 511 |
| 2392 | 2993 | SEQ ID NO: 33 | S87H | 427 | 2477 | 3078 | SEQ ID NO:34 | S87H | 512 |
| 2393 | 2994 | SEQ ID NO: 33 | N88D | 428 | 2478 | 3079 | SEQ ID NO:34 | N88D | 513 |
| 2394 | 2995 | SEQ ID NO: 33 | N88T | 429 | 2479 | 3080 | SEQ ID NO:34 | N88T | 514 |
| 2395 | 2996 | SEQ ID NO: 33 | N88Q | 430 | 2480 | 3081 | SEQ ID NO:34 | N88Q | 515 |
| 2396 | 2997 | SEQ ID NO: 33 | N88R | 431 | 2481 | 3082 | SEQ ID NO:34 | N88R | 516 |
| 2397 | 2998 | SEQ ID NO: 33 | N88E | 432 | 2482 | 3083 | SEQ ID NO:34 | N88E | 517 |
| 2398 | 2999 | SEQ ID NO: 33 | N88K | 433 | 2483 | 3084 | SEQ ID NO:34 | N88K | 518 |
| 2399 | 3000 | SEQ ID NO: 33 | N88H | 434 | 2484 | 3085 | SEQ ID NO:34 | N88H | 519 |
| 2400 | 3001 | SEQ ID NO: 33 | N88M | 435 | 2485 | 3086 | SEQ ID NO:34 | N88M | 520 |
| 2401 | 3002 | SEQ ID NO: 33 | N88S | 436 | 2486 | 3087 | SEQ ID NO:34 | N88S | 521 |
| 2402 | 3003 | SEQ ID NO: 33 | N88L | 437 | 2487 | 3088 | SEQ ID NO:34 | N88L | 522 |
| 2403 | 3004 | SEQ ID NO: 33 | V91I | 438 | 2488 | 3089 | SEQ ID NO:34 | V91I | 523 |
| 2404 | 3005 | SEQ ID NO: 33 | V91L | 439 | 2489 | 3090 | SEQ ID NO:34 | V91L | 524 |
| 2405 | 3006 | SEQ ID NO: 33 | V91D | 440 | 2490 | 3091 | SEQ ID NO:34 | V91D | 525 |
| 2406 | 3007 | SEQ ID NO: 33 | V91E | 441 | 2491 | 3092 | SEQ ID NO:34 | V91E | 526 |
| 2407 | 3008 | SEQ ID NO: 33 | V91N | 442 | 2492 | 3093 | SEQ ID NO:34 | V91N | 527 |
| 2408 | 3009 | SEQ ID NO: 33 | V91Q | 443 | 2493 | 3094 | SEQ ID NO:34 | V91Q | 528 |
| 2409 | 3010 | SEQ ID NO: 33 | V91S | 444 | 2494 | 3095 | SEQ ID NO:34 | V91S | 529 |
| 2410 | 3011 | SEQ ID NO: 33 | V91H | 445 | 2495 | 3096 | SEQ ID NO:34 | V91H | 530 |
| 2411 | 3012 | SEQ ID NO: 33 | I92E | 446 | 2496 | 3097 | SEQ ID NO:34 | I92E | 531 |
| 2412 | 3013 | SEQ ID NO: 33 | I92T | 447 | 2497 | 3098 | SEQ ID NO:34 | I92T | 532 |
| 2413 | 3014 | SEQ ID NO: 33 | I92K | 448 | 2498 | 3099 | SEQ ID NO:34 | I92K | 533 |
| 2414 | 3015 | SEQ ID NO: 33 | I92R | 449 | 2499 | 3100 | SEQ ID NO:34 | I92R | 534 |
| 2415 | 3016 | SEQ ID NO: 33 | I92L | 450 | 2500 | 3101 | SEQ ID NO:34 | I92L | 535 |
| 2416 | 3017 | SEQ ID NO: 33 | E95Q | 451 | 2501 | 3102 | SEQ ID NO:34 | E95Q | 536 |
| 2417 | 3018 | SEQ ID NO: 33 | E95G | 452 | 2502 | 3103 | SEQ ID NO:34 | E95G | 537 |
| 2418 | 3019 | SEQ ID NO: 33 | E95D | 453 | 2503 | 3104 | SEQ ID NO:34 | E95D | 538 |
| 2419 | 3020 | SEQ ID NO: 33 | E95N | 454 | 2504 | 3105 | SEQ ID NO:34 | E95N | 539 |
| 2420 | 3021 | SEQ ID NO: 33 | Q126E | 455 | 2505 | 3106 | SEQ ID NO:34 | Q126E | 540 |
| 2421 | 3022 | SEQ ID NO: 33 | Q126D | 456 | 2506 | 3107 | SEQ ID NO:34 | Q126D | 541 |
| 2422 | 3023 | SEQ ID NO: 33 | Q126A | 457 | 2507 | 3108 | SEQ ID NO:34 | Q126A | 542 |
| 2423 | 3024 | SEQ ID NO: 33 | Q126S | 458 | 2508 | 3109 | SEQ ID NO:34 | Q126S | 543 |
| 2424 | 3025 | SEQ ID NO: 33 | D84N+E95Q | 459 | 2509 | 3110 | SEQ ID NO:34 | D84N+E95Q | 544 |
| 2425 | 3026 | SEQ ID NO: 33 | D84E+E95Q | 460 | 2510 | 3111 | SEQ ID NO:34 | D84E+E95Q | 545 |
| 2426 | 3027 | SEQ ID NO: 33 | D84T+E95Q | 461 | 2511 | 3112 | SEQ ID NO:34 | D84T+E95Q | 546 |
| 2427 | 3028 | SEQ ID NO: 33 | D84Q+E95Q | 462 | 2512 | 3113 | SEQ ID NO:34 | D84Q+E95Q | 547 |
| 2428 | 3029 | SEQ ID NO: 33 | D84T+H16T | 463 | 2513 | 3114 | SEQ ID NO:34 | D84T+H16T | 548 |
| 2429 | 3030 | SEQ ID NO: 33 | D84N +V91I | 464 | 2514 | 3115 | SEQ ID NO:34 | D84N +V91I | 549 |
| 2430 | 3031 | SEQ ID NO: 33 | D84T+Q126E | 465 | 2515 | 3116 | SEQ ID NO:34 | D84T+Q126E | 550 |
| 2431 | 3032 | SEQ ID NO: 33 | D84N+Q126E | 466 | 2516 | 3117 | SEQ ID NO:34 | D84N+Q126E | 551 |
| 2432 | 3033 | SEQ ID NO: 33 | H16T+D84Q | 467 | 2517 | 3118 | SEQ ID NO:34 | H16T +D84Q | 552 |
| 2433 | 3034 | SEQ ID NO: 33 | H16T +V91I | 468 | 2518 | 3119 | SEQ ID NO:34 | H16T +V91I | 553 |

| Molecule ID | | Annotations for IL-2 portions | | IL-2 sequence SEQ ID NO | Molecule_ID | | Annotations for IL-2 portions | | IL-2 sequence SEQ ID NO |
|---|---|---|---|---|---|---|---|---|---|
| Format 1 | Format 2 | SEQ ID NO: | Mutations | | Format 1 | Format 2 | SEQ ID NO: | Mutations | |
| 2519 | 3120 | SEQ ID NO:35 | | 554 | 2561 | 3164 | SEQ ID NO:35 | N88D | 598 |
| 2518 | 3121 | SEQ ID NO:35 | L12R | 555 | 2562 | 3165 | SEQ ID NO:35 | N88T | 599 |
| 2519 | 3122 | SEQ ID NO:35 | L12K | 556 | 2563 | 3166 | SEQ ID NO:35 | N88Q | 600 |
| 2520 | 3123 | SEQ ID NO:35 | L12E | 557 | 2564 | 3167 | SEQ ID NO:35 | N88R | 601 |
| 2521 | 3124 | SEQ ID NO:35 | L12Q | 558 | 2565 | 3168 | SEQ ID NO:35 | N88E | 602 |
| 2522 | 3125 | SEQ ID NO:35 | E15Q | 559 | 2566 | 3169 | SEQ ID NO:35 | N88K | 603 |
| 2523 | 3126 | SEQ ID NO:35 | E15R | 560 | 2567 | 3170 | SEQ ID NO:35 | N88H | 604 |
| 2524 | 3127 | SEQ ID NO:35 | E15A | 561 | 2568 | 3171 | SEQ ID NO:35 | N88M | 605 |
| 2525 | 3128 | SEQ ID NO:35 | E15S | 562 | 2569 | 3172 | SEQ ID NO:35 | N88S | 606 |
| 2526 | 3129 | SEQ ID NO:35 | H16N | 563 | 2570 | 3173 | SEQ ID NO:35 | N88L | 607 |
| 2527 | 3130 | SEQ ID NO:35 | H16T | 564 | 2571 | 3174 | SEQ ID NO:35 | V91I | 608 |
| 2528 | 3131 | SEQ ID NO:35 | H16Y | 565 | 2572 | 3175 | SEQ ID NO:35 | V91L | 609 |
| 2529 | 3132 | SEQ ID NO:35 | H16A | 566 | 2573 | 3176 | SEQ ID NO:35 | V91D | 610 |
| 2530 | 3133 | SEQ ID NO:35 | H16E | 567 | 2574 | 3177 | SEQ ID NO:35 | V91E | 611 |
| 2531 | 3134 | SEQ ID NO:35 | H16D | 568 | 2575 | 3178 | SEQ ID NO:35 | V91N | 612 |
| 2532 | 3135 | SEQ ID NO:35 | H16R | 569 | 2576 | 3179 | SEQ ID NO:35 | V91Q | 613 |
| 2533 | 3136 | SEQ ID NO:35 | L19D | 570 | 2577 | 3180 | SEQ ID NO:35 | V91S | 614 |
| 2534 | 3137 | SEQ ID NO:35 | L19E | 571 | 2578 | 3181 | SEQ ID NO:35 | V91H | 615 |
| 2535 | 3138 | SEQ ID NO:35 | L19R | 572 | 2579 | 3182 | SEQ ID NO:35 | I92E | 616 |
| 2536 | 3139 | SEQ ID NO:35 | L19S | 573 | 2580 | 3183 | SEQ ID NO:35 | I92T | 617 |
| 2537 | 3140 | SEQ ID NO:35 | D20N | 574 | 2581 | 3184 | SEQ ID NO:35 | I92K | 618 |
| 2538 | 3141 | SEQ ID NO:35 | D20Q | 575 | 2582 | 3185 | SEQ ID NO:35 | I92R | 619 |
| 2539 | 3142 | SEQ ID NO:35 | D20E | 576 | 2583 | 3186 | SEQ ID NO:35 | I92L | 620 |
| 2540 | 3143 | SEQ ID NO:35 | D20A | 577 | 2584 | 3187 | SEQ ID NO:35 | E95Q | 621 |
| 2541 | 3144 | SEQ ID NO:35 | D20R | 578 | 2585 | 3188 | SEQ ID NO:35 | E95G | 622 |
| 2542 | 3145 | SEQ ID NO:35 | D20S | 579 | 2586 | 3189 | SEQ ID NO:35 | E95D | 623 |
| 2543 | 3146 | SEQ ID NO:35 | D84N | 580 | 2587 | 3190 | SEQ ID NO:35 | E95N | 624 |
| 2544 | 3147 | SEQ ID NO:35 | D84E | 581 | 2588 | 3191 | SEQ ID NO:35 | Q126E | 625 |
| 2545 | 3148 | SEQ ID NO:35 | D84Q | 582 | 2589 | 3192 | SEQ ID NO:35 | Q126D | 626 |
| 2546 | 3149 | SEQ ID NO:35 | D84T | 583 | 2590 | 3193 | SEQ ID NO:35 | Q126A | 627 |
| 2547 | 3150 | SEQ ID NO:35 | D84S | 584 | 2591 | 3194 | SEQ ID NO:35 | Q126S | 628 |
| 2548 | 3151 | SEQ ID NO:35 | D84R | 585 | 2592 | 3195 | SEQ ID NO:35 | D84N+E95Q | 629 |
| 2549 | 3152 | SEQ ID NO:35 | D84G | 586 | 2593 | 3196 | SEQ ID NO:35 | D84E+E95Q | 630 |
| 2550 | 3153 | SEQ ID NO:35 | D84M | 587 | 2594 | 3197 | SEQ ID NO:35 | D84T+E95Q | 631 |
| 2551 | 3154 | SEQ ID NO:35 | D84F | 588 | 2595 | 3198 | SEQ ID NO:35 | D84Q+E95Q | 632 |
| 2552 | 3155 | SEQ ID NO:35 | D84L | 589 | 2596 | 3199 | SEQ ID NO:35 | D84T+H16T | 633 |
| 2553 | 3156 | SEQ ID NO:35 | D84K | 590 | 2597 | 3200 | SEQ ID NO:35 | D84N +V91I | 634 |
| 2554 | 3157 | SEQ ID NO:35 | D84H | 591 | 2598 | 3201 | SEQ ID NO:35 | D84T+Q126E | 635 |
| 2555 | 3158 | SEQ ID NO:35 | S87T | 592 | 2599 | 3202 | SEQ ID NO:35 | D84N+Q126E | 636 |
| 2556 | 3159 | SEQ ID NO:35 | S87R | 593 | 2600 | 3203 | SEQ ID NO:35 | H16T +D84Q | 637 |
| 2557 | 3160 | SEQ ID NO:35 | S87K | 594 | 2601 | 3204 | SEQ ID NO:35 | H16T +V91I | 638 |
| 2558 | 3161 | SEQ ID NO:35 | S87L | 595 | | | | | |
| 2559 | 3162 | SEQ ID NO:35 | S87M | 596 | | | | | |
| 2560 | 3163 | SEQ ID NO:35 | S87H | 597 | | | | | |

## Claims

1. An IL-2 mutant protein, compared to a wild-type IL-2 (preferably a human IL-2, and more preferably an IL-2 comprising a sequence of SEQ ID NO: 1), comprising mutations:
(i) a mutation that changes (e.g., eliminates or reduces) binding affinity for an IL-2Rα receptor, at a binding interface of IL-2 to IL-2Rα, particularly at at least one position selected from positions 35, 37, 38, 41, 42, 43, 45, 61, 68 and 72;
and/or
(ii) a shortened B'C' loop region (i.e., a sequence linking amino acid residues aa72 and aa84), wherein preferably, the shortened loop region has less than 10, 9, 8, 7, 6 or 5 amino acids in length, and more preferably has 7 amino acids in length; preferably, the shortened B'C' loop region leads to an improved protein expression yield and/or purity;
and comprising a mutation:
(iii) a mutation that weakens binding to an IL-2Rβγ receptor, at a binding interface of IL-2 to IL-2Rβγ, particularly at at least one position selected from positions 12, 15, 16, 19, 20, 84, 87, 88, 91, 92, 95 and 126; wherein, the amino acid positions are numbered according to SEQ ID NO: 1;
preferably, the mutant protein comprises the mutations (i) and (iii) or comprises the mutations (ii) and (iii), or comprises the mutations (i), (ii) and (iii);
more preferably, the mutant protein comprises the mutations (ii) and (iii) but does not comprise the mutation (i).

2. The mutant protein according to claim 1, wherein the IL-2Rβγ binding interface mutation (iii) includes a mutation selected from the following:
L12R, L12K, L12E, L12Q, E15Q, E15R, E15A, E15S, H16N, H16T, H16Y, H16A, H16E, H16D, H16R, L19D, L19E, L19R, L19S, D20N, D20Q, D20E, D20A, D20R, D20S, D84N, D84E, D84Q, D84T, D84S, D84R, D84G, D84M, D84F, D84L, D84K, D84H, S87T, S87R, S87K, S87L, S87M, S87H, N88D, N88T, N88Q, N88R, N88E, N88K, N88H, N88M, N88S, N88L, V91I, V91L, V91D, V91E, V91N, V91Q, V91S, V91H, I92E, I92T, I92K, I92R, I92L, E95Q, E95G, E95D, E95N, Q126E, Q126D, Q126A, Q126S, D84N+E95Q, D84E+E95Q, D84T+E95Q, D84Q+E95Q, D84T+H16T, D84N+V91I, D84T+Q126E, D84N+Q126E, H16T+D84Q and H16T+V91I;
preferably, the IL-2Rβγ binding interface mutation (iii) includes a mutation selected from the following:
D20N, D84E, D84N, D84N+E95Q, D84N+Q126E, D84N+V91I, D84Q, D84T, D84T+H16T, D84T+Q126E, E15Q, E95N, E95Q, H16N, H16N+D84N, H16T, H16T+D84Q, H16T+V91I, N88D, N88R, N88Q, N88T, Q126E and V91I;
more preferably, the IL-2Rβγ binding interface mutation (iii) includes
- a mutation selected from the following: D84N, E95Q and H16N; or
- a mutation selected from the following: D84Q and H16T+V911; or
- a mutation selected from the following: D84T, Q126E, D84N+V91I and D84N+E95Q; or
- a mutation selected from the following: N88D, N88R, D20N, D84N, D84N+E95Q, D84T+H16T, D84T+Q126E, D84N+Q126E and H16T+D84Q; or
- a mutation selected from the following: D84N and D84N+E95Q;
and still more preferably, the IL-2Rβγ binding interface mutation (iii) includes N88D or D20N or N88R.

3. The mutant protein according to any one of the preceding claims, wherein the mutation (i) includes a combination of mutations K35E+T37E+R38E+F42A, or a combination of mutations K35E+T37E+R38E, or a mutation F42A;
preferably, the mutation (i) includes a combination of mutations K35E+T37E+R38E+F42A.

4. The mutant protein according to any one of the preceding claims, wherein the mutation (ii) includes:
(a) a substitution of aa74 to aa83 in a B'C' loop region, for example, with a short B'C' loop sequence from four-helical short-chain cytokine IL family members, such as a B'C' loop sequence from IL-15, preferably, with a sequence GDASIH; or
(b) a truncation of aa74 to aa83 in a B'C' loop region, for example, by 1, 2, 3 or 4 amino acids at the C-terminus; preferably a truncation to form a sequence (Q/G)SKN(F/I)H, and more preferably a truncation to form a sequence selected from the following:
| B'C' loop sequence |
|---|
| QSKNFH |
| GSKNFH |
| QSANFH |
| Q SANIH |

5. The mutant protein according to any one of the preceding claims, relative to the wild-type IL-2, comprising:
(i) a combination of mutations K35**E**+T37**E**+R38**E**+F42**A**;
(ii) a B'C' loop region sequence: AQSKNFH; or AGDASIH or SGDASIH;
(iii) an IL-2Rβγ binding interface mutation, selected from the following:
D20N, D84E, D84N, D84N+E95Q, D84N+Q126E, D84N+V91I, D84Q, D84T, D84T+H16T, D84T+Q126E, E15Q, E95N, E95Q, H16N, H16N+D84N, H16T, H16T+D84Q, H16T+V91I, N88D, N88R, N88Q, N88T, Q126E and V91I;
wherein more preferably, the IL-2Rβγ binding interface mutation:
- is selected from D84N, E95Q and H16N; or
- is selected from D84T, Q126E, D84N+V911 and D84N+E95Q; or
- is selected from N88D, N88R, D20N, D84N+E95Q, D84T+H16T, D84T+Q126E, D84N+Q126E and H16T+D84Q;
and optionally, (iv) a mutation T3A.

6. The mutant protein according to any one of the preceding claims, relative to the wild-type IL-2, maintaining binding to the IL-2Rα receptor and comprising:
(ii) a B'C' loop region sequence: AQSKNFH; or AGDASIH or SGDASIH, particularly AGDASIH;
(iii) an IL-2Rβγ binding interface mutation, selected from the following:
D20N, D84E, D84N, D84N+E95Q, D84N+Q126E, D84N+V91I, D84Q, D84T, D84T+H16T, D84T+Q126E, E15Q, E95N, E95Q, H16N, H16N+D84N, H16T, H16T+D84Q, H16T+V91I, N88D, N88R, N88Q, N88T, Q126E and V91I;
more preferably, the IL-2Rβγ binding interface mutation is selected from: D20N, N88D and N88R;
and optionally, (iv) a mutation T3A.

7. The mutant protein according to any one of claims 1-6, relative to the wild-type IL-2, further comprising no more than 0-5 amino acid mutations, preferably comprising a mutation C125S or C125A.

8. The mutant protein according to any one of claims 1-7, comprising:
- an amino acid sequence having at least 90%, 92%, 93%, 94%, 95%, 96%, 97% or 98% identity to an amino acid sequence selected from SEQ ID NOs: 37-638 (particularly SEQ ID NO: 148 or 197 or SEQ ID NO: 489, 513 or 516); and
- an amino acid sequence selected from SEQ ID NOs: 37-638 (particularly SEQ ID NO: 148 or 197 or SEQ ID NO: 489, 513 or 516).

9. The mutant protein according to any one of claims 1-8, compared to the protein before weakening by introducing the mutation (iii), having reduced binding affinity for the IL-2Rβγ receptor,
and having at least one or two properties selected from the following:
- maintained binding to IL-2Rα compared to the wild-type IL-2, or
- improved expression yield and/or ease of purification to higher purity (e.g. higher purity as measured by SEC-HPLC after one-step affinity chromatography) when expressed in mammalian cells (e.g. 293 cells or CHO cells), e.g. as an Fc fusion protein, compared to the wild-type IL-2.

10. An IL-2 mutant protein fusion protein, comprising the IL2 mutant protein according to any one of claims 1-9.

11. The fusion protein according to claim 10, wherein the IL-2 mutant protein is fused to an Fc antibody fragment (e.g., a human IgG1 Fc),
preferably, the IL-2 mutant protein is fused to an Fc via a linker, wherein the linker is preferably GSGS, and more preferably (G4S)₂;
preferably the Fc fragment comprises mutations that reduce or eliminate binding of Fc to FcyR, e.g., L234A+L235A;
preferably the Fc fragment has an amino acid sequence having at least 85%, at least 95%, at least 96% or 100% identity to SEQ ID NO: 12.

12. The fusion protein according to claim 11, wherein the Fc fragment comprises a Knob mutation, e.g., mutations T366W and S354C; or the Fc fragment comprises a Hole mutation, e.g., mutations Y349C, T366S, L368A and Y407V.

13. An IL-2-Fc dimer protein, comprising the IL-2 mutant protein fusion protein according to claim 11 or 12, wherein
preferably, the dimer protein, compared to a corresponding dimer protein comprising a wild-type IL-2-Fc fusion protein, has one or more of the following properties:
- an increased expression yield and/or purity, when expressed in mammalian cells (e.g., CHO or HEK293 cells);
- low toxicity when administered *in vivo,* for example, as determined by measuring the body weight or change in the body weight of an animal after administration; and
- better anti-tumor efficacy.

14. The IL-2-Fc dimer protein according to claim 13, being a homodimer, wherein a first monomer and a second monomer comprise, from N-terminus to C-terminus, i) the IL-2 mutant protein; ii) the linker; and iii) the Fc fragment;
preferably each of the monomers comprises an IL-2 mutant protein selected from SEQ ID NOs: 37-638 linked at the C-terminus to an amino acid sequence of SEQ ID NO: 12 by a linker (G4S)2.

15. The IL-2-Fc dimer protein according to claim 13, being a heterodimer and comprising:
a) a first monomer, comprising, from N-terminus to C-terminus, i) the IL-2 mutant protein; ii) the linker; and iii) a first Fc fragment; and
b) a second monomer, comprising a second Fc fragment and optionally an IgG1 hinge region;
wherein preferably, the first Fc fragment and the second Fc fragment comprise a first heterodimerization mutation and a second heterodimerization mutation that promote the formation of the heterodimer from the first monomer and the second monomer, respectively;
preferably, the first and second heterodimerization mutations comprise a combination of Knob:Hole mutations T366W/S354C:Y349C/T366S/L368A/Y407V;
preferably, the first heterodimerization mutation in the first Fc fragment includes a Knob mutation and the second heterodimerization mutation in the second Fc fragment includes a Hole mutation; alternatively, the first heterodimerization mutation in the first Fc fragment includes a Hole mutation and the second heterodimerization mutation in the second Fc fragment includes a Knob mutation;
preferably, the first monomer comprises an IL-2 mutant protein selected from SEQ ID NOs: 37-638 linked at the C-terminus to an amino acid sequence of SEQ ID NO: 9 by a linker (G4S)2; and the second monomer comprises an amino acid sequence of SEQ ID NO: 10.

16. An immunoconjugate, comprising the IL-2 mutant protein according to any one of claims 1-9 and an antigen-binding molecule, wherein preferably, the antigen-binding molecule is an immunoglobulin molecule, particularly an IgG molecule, or an antibody or an antibody fragment, and more particularly an Fab molecule or an scFv molecule.

17. An isolated polynucleotide, encoding the IL2 mutant protein according to any one of claims 1-9, or the fusion protein according to any one of claims 10-12, or the IL-2-Fc dimer protein according to any one of claims 13-15, or the immunoconjugate according to claim 16.

18. An expression vector, comprising the polynucleotide according to claim 17.

19. A host cell, comprising the polynucleotide according to claim 17 or the vector according to claim 18, wherein preferably, the host cell is a mammalian cell, particularly an HEK293 cell or a CHO cell, or a yeast.

20. A method for producing the IL-2 mutant protein according to any one of claims 1-9, or the fusion protein according to any one of claims 10-12, or the IL-2-Fc dimer protein according to any one of claims 13-15, or the immunoconjugate according to claim 16, comprising culturing the host cell according to claim 19 under conditions suitable for expression of the IL-2 mutant protein or the fusion protein or the IL-2-Fc dimer protein or the immunoconjugate.

21. A pharmaceutical composition, comprising the IL-2 mutant protein according to any one of claims 1-9, or the fusion protein according to any one of claims 10-12, or the IL-2-Fc dimer protein according to any one of claims 13-15, or the immunoconjugate according to claim 16, and a pharmaceutically acceptable carrier.

22. A method for treating a disease in a subject, comprising administering to the subject the IL-2 mutant protein according to any one of claims 1-9, or the fusion protein according to any one of claims 10-12, or the IL-2-Fc dimer protein according to any one of claims 13-15, or the immunoconjugate according to claim 16, or the pharmaceutical composition according to claim 21, wherein preferably the disease is cancer or an autoimmune disease.

23. A method for stimulating the immune system of a subject, comprising administering to the subject an effective amount of the IL-2 mutant protein according to any one of claims 1-9, or the fusion protein according to any one of claims 10-12, or the IL-2-Fc dimer protein according to any one of claims 13-15, or the immunoconjugate according to claim 16, or the pharmaceutical composition according to claim 21.

24. A method for obtaining an IL-2 mutant protein, comprising the following steps:
- shortening a sequence of a B'C' loop region of IL-2 by mutation, and optionally introducing one or more mutations into a binding interface of IL-2 to IL-2Rβγ and/or introducing one or more mutations into a binding interface of IL-2 to IL-2Rα; and
- expressing the IL-2 mutant protein in a mammalian cell (e.g., an HEK293 or CHO cell), for example, in the form of an Fc fusion (e.g., an FcLALA fusion);
or comprising the following steps:
- introducing one or more mutations into a binding interface of IL-2 to IL-2Rβγ, and optionally shortening a sequence of a B'C' loop region of IL-2 by mutation and/or introducing one or more mutations into a binding interface of IL-2 to IL-2Rα, preferably not introducing a mutation into the binding interface of IL-2 to IL-2Rα; and
- expressing the IL-2 mutant protein in a mammalian cell (e.g., an HEK293 or CHO cell), for example, in the form of an Fc fusion (e.g., an FcLALA fusion);
wherein preferably, the shortened loop region has less than 10, 9, 8, 7, 6 or 5 amino acids in length, and more preferably has 7 amino acids in length;
more preferably, the B'C' loop region mutation includes:
(a) a substitution of aa74 to aa83 in the B'C' loop region, for example, with a short B'C' loop sequence from four-helical short-chain cytokine IL family members, such as a B'C' loop sequence from IL-15, preferably, with a sequence including GDASIH; or
(b) a truncation of aa74 to aa83 in the B'C' loop region, for example, by 1, 2, 3 or 4 amino acids at the C-terminus; preferably a truncation to form a sequence (Q/G)SKN(F/I)H, and more preferably a truncation to form a sequence selected from the following:
| B'C' loop sequence |
|---|
| QSKNFH |
| GSKNFH |
| QSANFH |
| QSANIH |
preferably, the IL-2Rβγ binding interface mutation includes the IL-2Rβγ binding interface mutation described in claim 1 or 2;
preferably, the IL-2Rα binding interface mutation includes the IL-2Rα binding interface mutation described in claim 1 or 3;
preferably, the mutant protein has one of the following improved properties or any combination thereof: (i) improved expression yield and/or protein purity (e.g., purity after one-step affinity chromatography as determined by SEC-HPLC); (ii) weakened binding to IL2RP and/or (iii) altered binding to IL-2Rα; preferably has the properties (i) and (ii).

25. A method for engineering a B'C' loop region of an IL-2 protein, comprising:
(a) substituting aa74 to aa83 in the B'C' loop region, for example, with a short B'C' loop sequence from four-helical short-chain cytokine IL family members, such as a B'C' loop sequence from IL-15, preferably, with a sequence including GDASIH; or
(b) substituting aa73 to aa83 in the B'C' loop region, for example, with a short B'C' loop sequence from four-helical short-chain cytokine IL family members, such as a B'C' loop sequence from IL-15, preferably, with a sequence including AGDASIH; or
(c) truncating aa74 to aa83 in the B'C' loop region, for example, by 1, 2, 3 or 4 amino acids at the C-terminus; preferably to form a sequence (Q/G)S(K/A/D)N(F/I)H, and more preferably to form a sequence selected from the following:
| B'C' loop sequence |
|---|
| QSKNFH |
| GSKNFH |
| QSANFH |
| QSANIH |
| QSANFH |
| QSDNFH |

26. An IL-2 protein, engineered by using the method according to claim 25.
